(19) Europäisches Patentamt
European Patent Office
Office européen des brevets

(11) EP 2 814 982 B1

# (12) EUROPEAN PATENT SPECIFICATION

(45) Date of publication and mention of the grant of the patent:
**05.10.2016 Bulletin 2016/40**

(21) Application number: **13707823.4**

(22) Date of filing: **18.02.2013**

(51) Int Cl.:
***C12Q 1/68*** *(2006.01)*

(86) International application number:
**PCT/EP2013/053204**

(87) International publication number:
**WO 2013/121048 (22.08.2013 Gazette 2013/34)**

## (54) NEW METHOD FOR PROGNOSING THE SURVIVAL OF PATIENTS SUFFERING FROM CHRONIC MYELOMONOCYTIC LEUKAEMIA

NEUES VERFAHREN ZUR PROGNOSE DER ÜBERLEBENSCHANCE VON PATIENTEN MIT CHRONISCHER MYELOMONOZYTÄRER LEUKÄMIE

NOUVEAU PROCÉDÉ DESTINÉ À PRONOSTIQUER LES CHANCES DE SURVIE DE PATIENTS SOUFFRANT D'UNE LEUCÉMIE MYÉLOMONOCYTAIRE CHRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2012 EP 12305187**

(43) Date of publication of application:
**24.12.2014 Bulletin 2014/52**

(73) Proprietors:
- **Centre Hospitalier Universitaire Nimes 30000 Nimes (FR)**
- **ACOBIOM 34790 Grabels (FR)**

(72) Inventors:
- **PIQUEMAL, David 30380 Saint-Christol-lès-Alès (FR)**
- **JOURDAN, Eric 30900 Nîmes (FR)**
- **COMMES, Thérèse 34570 Vailhauques (FR)**
- **BOU SAMRA, Elias Broumana (LB)**

(74) Representative: **Regimbeau 20, rue de Chazelles 75847 Paris Cedex 17 (FR)**

(56) References cited:
- **AFFYMETRIX: "GeneChip TM Human Genome U133 Set", INTERNET CITATION, [Online] pages 1-2, XP002613737, Retrieved from the Internet: URL:http://www.helmholtz-hzi.de/fileadmin/user_upload/research/Research_Programme/Technological_Platforms/Gene_Expression_Analysis/Service/humangenomeu133.pdf> [retrieved on 2010-12-10]**
- **ULRIKE BACHER ET AL: "Recent advances in diagnosis, molecular pathology and therapy of chronic myelomonocytic leukaemia", BRITISH JOURNAL OF HAEMATOLOGY, vol. 153, no. 2, 1 April 2011 (2011-04-01) , pages 149-167, XP055059784, ISSN: 0007-1048, DOI: 10.1111/j.1365-2141.2011.08631.x**
- **V GROSSMANN ET AL: "Molecular profiling of chronic myelomonocytic leukemia reveals diverse mutations in >80% of patients with TET2 and EZH2 being of high prognostic relevance", LEUKEMIA, vol. 25, no. 5, 1 May 2011 (2011-05-01), pages 877-879, XP055059749, ISSN: 0887-6924, DOI: 10.1038/leu.2011.10**
- **CHRISTINA ECONOMOPOULOU ET AL: "Cell cycle and apoptosis regulatory gene expression in the bone marrow of patients with de novo myelodysplastic syndromes (MDS)", ANNALS OF HEMATOLOGY, SPRINGER, BERLIN, DE, vol. 89, no. 4, 8 October 2009 (2009-10-08), pages 349-358, XP019800690, ISSN: 1432-0584**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KONTOS CHRISTOS K ET AL: "Let-7a, Mir-17 and Mir-20a Expression Levels in CD34+Bone Marrow Cells of Patients with Myelodysplastic Syndromes (MDS) Are Associated with Established Prognostic Factors, Supporting Their Implication in the Pathogenesis of the Disease", BLOOD, vol. 118, no. 21, November 2011 (2011-11), page 1620, XP009168820, & 53RD ANNUAL MEETING AND EXPOSITION OF THE AMERICAN-SOCIETY-OF-HEMATOLOGY (ASH); SAN DIEGO, CA, USA; DECEMBER 10 -13, 2011
- NASSIRI MEHDI ET AL: "MicroRNA Expression Profiles in Chronic Myelomonocytic Leukemia.", BLOOD, vol. 112, no. 11, November 2008 (2008-11), pages 933-934, XP009168819, & 50TH ANNUAL MEETING OF THE AMERICAN-SOCIETY-OF-HEMATOLOGY; SAN FRANCISCO, CA, USA; DECEMBER 06 -09, 2008 ISSN: 0006-4971
- SCHNITTGER SUSANNE ET AL: "SRSF2 is Mutated in 47.2% (77/163) of Chronic Myelomonocytic Leukemia (CMML) and Prognostically Favorable in Cases with Concomitant RUNX1 mutations", BLOOD, vol. 118, no. 21, November 2011 (2011-11), page 126, XP009168809, & 53RD ANNUAL MEETING AND EXPOSITION OF THE AMERICAN-SOCIETY-OF-HEMATOLOGY (ASH); SAN DIEGO, CA, USA; DECEMBER 10 -13, 2011
- K THEILGAARD-MÖNCH ET AL: "Gene expression profiling in MDS and AML: potential and future avenues", LEUKEMIA, vol. 25, no. 6, 29 March 2011 (2011-03-29) , pages 909-920, XP55021056, ISSN: 0887-6924, DOI: 10.1038/leu.2011.48
- KEN I MILLS ET AL: "Microarray-based classifiers and prognosis models identify subgroups with distinct clinical outcomes and high risk of AML transformation of myelodysplastic syndrome", BLOOD, AMERICAN SOCIETY OF HEMATOLOGY, UNITED STATES, vol. 114, no. 5, 30 July 2009 (2009-07-30) , pages 1063-1072, XP002658996, ISSN: 1528-0020, DOI: 10.1182/BLOOD-2008-10-187203 [retrieved on 2009-05-14]
- ELIAS BOU SAMRA ET AL: "New prognostic markers, determined using gene expression analyses, reveal two distinct subtypes of chronic myelomonocytic leukaemia patients", BRITISH JOURNAL OF HAEMATOLOGY, vol. 157, no. 3, 1 May 2012 (2012-05-01), pages 347-356, XP055050732, ISSN: 0007-1048, DOI: 10.1111/j.1365-2141.2012.09069.x
- T. Haferlach ET AL: "Clinical Utility of Microarray-Based Gene Expression Profiling in the Diagnosis and Subclassification of Leukemia: Report From the International Microarray Innovations in Leukemia Study Group", Journal of Clinical Oncology, vol. 28, no. 15, 20 April 2010 (2010-04-20), pages 2529-2537, XP055194995, ISSN: 0732-183X, DOI: 10.1200/JCO.2009.23.4732

## Description

## TECHNICAL BACKGROUND OF THE INVENTION

Chronic myelomonocytic leukemia (CMML) is a clonal hematopoietic stem cell disorder frequently seen in the ederly people. First considered as a myelodysplasic disorder in the French American british (FAB), CMML was reclassified by the World Health Organization (WHO) as myelodysplasic/myeloproliferative entity. This reclassification allows considering the heterogeneity of the CMML syndrome in diagnosis and prognosis. Despite this heterogeneity, the diagnosis of CMML is definitely straightforward in the presence of a combination of persistent blood monocytosis and fewer than 20% blasts in peripheral blood and bone marrow. According to WHO criteria, blasts include myeloblasts, monoblasts and promonocytes. The myeloid compartment is frequently associated with cytogenetic abnormality that helps to confirm the CMML diagnosis. Thus, CMML is mainly characterized by a persistent peripheral monocytosis (>1 x $10^9$/l), less than 20% blasts in blood and bone marrow, and a variable degree of dysplasia in one or more myeloid lineages. However CMML patients often showed heterogeneity in cytogenetics, and these cytogenetic markers have therefore a poor prognostic value. Major difficulties are faced in the clinical classification of this disease and the variable risk of its progression.

Molecular studies based on mutation identification may provide promising insights in the diagnosis and prognosis process. Twenty two percent of patients exhibit point mutations of *RAS* genes (*RAS*, *KRAS*) at diagnosis or during the disease course and as many as 50% present *TET2* mutations (Ricci, C. et al., Clinical Cancer Research ,2010). More recently, by applying next-generation sequencing (NGS) technology to characterize molecular mutations, Kohlmann *et al.* detected at least one aberration in 72.8% of CMML cases, including in the *ten-eleven translocation 2 (TET2)* gene. According to them, patients carrying these mutations present a better outcome contrary to Kosmider *et al.* where *TET2* mutations are linked to a poor prognosis (Kohlmann, A. et al., Journal of Clinical Oncology ,2010; Tefferi A et al., Leukemia, 2009; Kosmider O et al., Haematologica, 2009). In absence of major prognostic markers, the physicians face major problems in evaluating the variable risk of progression of this disease to acute myeloid leukemia (AML): the disease is greatly heterogeneous in term of clinical course, a part of the patients displaying an indolent and stable disease, other displaying a more aggressive disease. Criteria for initiating a therapy in CMML are not well established, and depend on the physician's experience.

There is therefore a need of a rapid and reliable prognostic method enabling to predict the survival chances of a patient suffering from CMML and/or the suitability of said patient for a drug trial.

Analysis of gene expression profiles (GEP) is very promising in the medical field. It helps the discovery of new tools for applied therapy, notably new prognostic and diagnostic markers, and highlights evaluation criteria for treatments and disease follow-up. However, despite the highly documented data concerning acute leukemia, slight information till now is known about myelodysplastic syndromes, and particularly about CMML.

As shown in the herein presented results, the present Inventors have found 5 new strong molecular prognostic markers including the *G6PD, 6PGD, TKT, CEACAM4* and *ELANE* genes. All are predominantly linked to a promyelocytic phenotype according to Amazonia, the public DNA microarray database. Likewise, a clear distinction between two sets of patients has been observed for the first time, depending reliably on the expression levels of each of these markers: patients having a "bad" prognosis of survival, with median time survival (MTS) of 21 months (less than two years), and patients having a "good" prognosis of survival, with MTS of 83 months (almost 4 years).

This represents an important and medically useful discovery as it will enable to determine prior to the treatment which patients will fail therapeutic treatment, thus saving them from up to a year of an expensive treatment with significant side effects.

## FIGURE LEGENDS

**Fig. 1. Supervised clustering of CMML samples using 28 significantly expressed genes (FDR<5%).** Red and green indicate over and under-expressed genes, respectively. Each row represents a single gene probe (28 genes) and each column represents a distinct CMML sample (32 samples). Samples were clustered into 2 subtypes, A and B. Subtypes A and B group 13 and 19 CMML patients, respectively.

**Fig. 2. Kaplan-Meïer estimates of overall survival (OS).** The index computation based on the expression data of the 5 selected genes (*TKT*, *G6PD, ELANE*, *PGD* and *CEACAM4*) allowed the discrimination between two distinct groups of patients. Patients were equally distributed (N=16 in each group). We characterize a good survival group (dotted grey) with a low index score and 94% probability of survival (15/16), and a poor survival group (black) with a high index score and 19% probability of survival (3/16). A P-value of 0.007 was obtained.

**Fig. 3. Microarray expression of selected genes.** Expression histograms of five specific genes *TKT, G6PD, PGD, ELANE* and *CEACAM4* in various normal haematological tissues. Histograms were obtained from the *Amazonia* website from the HG-U133 Plus 2.0; Affymetrix (Santa Clara, CA) oligonucleotide microarray datasets.

**Fig. 4. Kaplan-Meïer estimates of overall survival (OS). A)** The index computation based on the expression data of the 5 selected genes *(TKT, G6PD, ELANE*, *PGD* and *CEACAM4*) in the new cohort of 21 CMML samples allowed the discrimination between two distinct groups of patients. We characterized a good survival group (green) with a low index score and 56% probability of survival (5/9), and a poor survival group (red) with a high index score and 25% probability of survival (3/12). A P-value of 0.03 was obtained. **B)** The index computation based on the expression data of the 5 selected genes (*TKT, G6PD, ELANE*, *PGD* and *CEACAM4*) in both mixed cohorts of 53 CMML samples allowed the discrimination between two distinct groups of patients. We characterized a good survival group (green) with a low index score and 80% probability of survival (20/25), and a poor survival group (red) with a high index score and 21% probability of survival (6/28). A P-value of 0.002 was obtained.

**DESCRIPTION OF THE INVENTION**

Interestingly, the present Inventors have found that the survival chances of patients suffering from chronic myelomonocytic leukaemia can be assessed on the simple analysis of the expression level in PBMC cells of a set of 5 genes or homologous thereof, and comparison with the expression level of the same genes in PBMCs of healthy patients.

**The present invention is defined in the appended claims** 1 to 10.

**Also disclosed is** a method for *in vitro* determining the prognosis of chronic myelomonocytic leukaemia (CMML) in a human patient suffering thereof, comprising at least the following steps:

a) measuring in a test sample of said patient the expression levels of at least two genes chosen in the group consisting of: *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) or homologous thereof,
b) comparing said expression levels to the expression level of said at least two genes in at least one control sample obtained from at least one known healthy human subject,
c) predicting the outcome of the chronic myelomonocytic leukaemia in said patient and/or the suitability of said patient for a drug trial.

Also disclosed is a method for *in vitro* determining the prognosis of CMML in a human patient suffering thereof, comprising at least the following steps:

a) obtaining a test sample from said human patient,
b) measuring the expression profile comprising at least two genes chosen in the group consisting of: *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) or homologous thereof in said test sample,
c) comparing said expression profile with at least one reference profile,
c) predicting the outcome of CMML in said patient.

According to the invention, a "CMML suffering patient" is a human subject showing persistent blood monocytosis and fewer than 20% blasts (myeloblasts, monoblasts and/or promonocytes) in peripheral blood and bone marrow.

The present invention enables to "prognose" (or to "determine the prognosis" of) the future life-span of a patient suffering from CMML, i.e. to predict the outcome of said disease in terms of month-survival for said patient, said patient being treated or not against this disease.

The term "test sample" designates any sample that may be taken from a CMML suffering patient, such as a serum sample, a plasma sample, a urine sample, a blood sample, a lymph sample, or a biopsy. The test sample for the determination of the gene expression levels is a peripheral blood sample comprising peripheral blood mononuclear cells (PBMC). Such PBMC samples can be obtained by a completely non-invasive harmless blood collection from the patient, followed by a classical ficoll separation as described in Cytotherapy (Janssen WE et al., 2010). More preferably, purity of the PBMC sample is up to 70%, preferably up to 80 % and more preferably up to 90% as classically obtained by Ficoll purification processes.

The term "expression profile" **may designate** the expression levels of a group of at least two genes chosen in the group consisting of *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) or homologous thereof.

Also disclosed is the expression level of at least three, preferably four, and more preferably five of the genes chosen in the group consisting of *G6PD* (SEQ ID NO:2 or *3), 6PGD* (SEQ ID NO:4), *TKT* (SEQ ID NO:9 or 10), *CEACAM4*

(SEQ ID NO:11) and *ELANE* (SEQ ID NO:23) or homologous thereof is measured in the method of the invention. The expression level of the five genes *G6PD* (SEQ ID NO:2 or 3), *6PGD* (SEQ ID NO:4), *TKT* (*SEQ* ID NO:9 or 10), *CEACAM4* (SEQ ID NO:11) and *ELANE* (SEQ ID NO:23) is measured, and the expression profile of the invention therefore consists of the expression level of these five genes.

A sixth gene can also be used in the method of the invention. This sixth gene is the *LYZ* gene of SEQ ID NO:1. Also disclosed is the expression level of at least four, preferably five, and more preferably six of the genes chosen in the group consisting of *LYZ* (SEQ ID NO:1), *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) or homologous thereof is measured in the method of the invention. In a more preferred embodiment, the expression level of the six genes *LYZ* (SEQ ID NO:1, *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) is measured, and the expression profile consists of the expression level of these genes.

The 6 genes that were determined by the Inventors to be able to discriminate between patients having a bad or a long survival are listed in the following table 1:

Table 1

| Symbole | Accession number NCBI | Encoded protein |
|---|---|---|
| LYZ | NM_000239.2 (SEQ ID NO:1) | Lysozyme C (enzyme EC 3.2.1.17)<br>Muramidase N-acetylmuramide glycanhydrolase<br>NP_000230.1 (SEQ ID NO:12) |
| G6PD | NM_000402.3 isoform A (SEQ ID NO:2)<br>NM_001042351.1 isoform B (SEQ ID NO:3) | Glucose-6-phosphate dehydrogenase (enzyme EC 1.1.1.49)<br>Isoform A: NP_000393.4 (SEQ ID NO:13)<br>Isoform B : NP_001035810.1 (SEQ ID NO:14) |
| 6PGD (or PGD) | NM_002631.2 (SEQ ID NO:4) | Phosphogluconate dehydrogenase (PGDH) 6 phosphogluconate dehydrogenase decarboxylating (6PGD) EC 1.1.1.44<br>NP_002622.2 (SEQ ID NO:15) |
| ELANE | NM 001972.2 (SEQ ID NO:23) | neutrophil-expressed Elastase<br>NP_001963.1 (SEQ ID NO:24) |
| TKT | NM_001064.3 (SEQ ID NO:9)<br>NM_001135055.2 (SEQ ID NO:10) | Transketolase humaine<br>NP_001055.1 : variant 1 (SEQ ID NO:20)<br>NP_001128527.1 : variant (SEQ ID NO:21) |
| CEACAM4 | NM_001817.2 (SEQ ID NO:11) | Homo sapiens carcinoembryonic antigen-related cell adhesion molecule 4<br>NP_001808.2 (SEQ ID NO:22) |

The term "homologous" refers to sequences that have sequence similarity. The term "sequence similarity", in all its grammatical forms, refers to the degree of identity or correspondence between nucleic acid sequences. In the context of the invention, two nucleic acid sequences are "homologous" when at least about 80%, alternatively at least about 81%, alternatively at least about 82%, alternatively at least about 83%, alternatively at least about 84%, alternatively at least about 85%, alternatively at least about 86%, alternatively at least about 87%, alternatively at least about 88%, alternatively at least about 89%, alternatively at least about 90%, alternatively at least about 91%, alternatively at least about 92%, alternatively at least about 93%, alternatively at least about 94%, alternatively at least about 95%, alternatively at least about 96%, alternatively at least about 97%, alternatively at least about 98%, alternatively at least about 99% of the nucleic acids are similar. Preferably the similar or homologous nucleic acid sequences are identified by alignment using, for example, the algorithm of Needleman-Wunsch.

The expression levels (or expression profile) may be determined by any technology known by a man skilled in the art. In particular, each gene expression level may be measured at the genomic and/or nucleic and/ or proteic level.

In a preferred embodiment, measuring the expression levels of the said genes (or the expression profile) is performed by measuring the amount of nucleic acid transcripts of each gene. The amount of nucleic acid transcripts of each gene can be measured by any technology known by a man skilled in the art. In particular, the measure can be carried out directly on extracted messenger RNA (mRNA) sample, or on retrotranscribed complementary DNA (cDNA) prepared from extracted mRNA by technologies well-known in the art. From the mRNA or cDNA sample, the amount of nucleic acid transcript may be measured using any technology known by a man skilled in the art, including microarrays, quantitative PCR, DNA chips, hybridization wit labelled probes, or flow lateral dipstick (Surasilp T et al., Mol Cell Probes. 2011). In a preferred embodiment, the expression levels are determined using quantitative PCR. Quantitative or real-time, PCR is a well-known and easily available technology for those skilled in the art and does therefore not need a precise description.

In this case, the measuring level is preferably performed by including an invariant endogenous reference gene (such as the *RPS19* gene), in the RNA / DNA detection assay to correct for sample to sample variations in PCR (or hybridization) efficiency and errors in sample quantification.

In another preferred embodiment, the expression levels of the said genes are determined by the use of nucleic microarrays.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression levels of define gene in a target nucleic sample, said sample can be labelled, contacted with the microarray in hybridization conditions, leading to the formation of complexes between target nucleic acids that are complementary to probe sequences attached to the microarray surface. The presence of labelled hybridized complexes can then be detected. Many variants of the microarray hybridization technology are available to the man skilled in the art.

The nucleic acid microarray is an oligonucleotide microarray comprising or consisting of 5 oligonucleotides specific for the 5 genes *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) (see Table 1 below). Preferably, the microarray also comprises the *LYZ* gene of SEQ ID NO:1.

Preferably, the oligonucleotides are about 50 bases in length. It is acknowledged that the nucleic acid microarray, or oligonucleotide microarray encompass the microarrays specific for the homologous genes as defined below.

Suitable oligonucleotides may be designed, based on the genomic sequence of each gene (see Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

In another preferred embodiment, measuring the expression levels of the said gene is performed by measuring the respective levels of the encoded proteins of the said genes, for example by employing antibody-based detection methods such as immunohistochemistry or western blot analysis, proteic microarray, flow cytometry or flow lateral dipstick (Surasilp T et al., Mol Cell Probes. 2011).

Said encoded proteins are namely: SEQ ID NO: 12 for the LYZ protein, SEQ ID NO:13 or 14 for the G6PD protein, SEQ ID NO:15 for the 6PGD protein, SEQ ID NO:24 for the ELANE protein, SEQ ID NO:20 or 21 for the TKT protein and SEQ ID NO:22 for the CEACAM4 protein.

For expression profiling experiments, antibodies, aptamers, or affibodies microarrays are mainly used, most of the time antibodies microarrays (Hall et al, 2007). The antibodies, aptamers, or affibodies are attached to various supports using various attachment methods, using a contact or non-contact spotter (Hall et al, 2007). Examples of suitable supports include glass and silicon microscope slides, nitrocellulose, microwells (for instance made of a silicon elastomer) (Hall et al, 2007). For glass and silicon microscope slides, a coating is generally added. Examples of coatings for random attachment (i.e. resulting in a random orientation of attached proteins to the support) include aldehyde- and epoxy-derivatized coatings for random attachment through amines, and nitrocellulose, gel pads or poly-L-lysine coatings (Hall et al, 2007). Examples of coatings for non random attachment (i.e. resulting in a uniform orientation of attached proteins to the support) include nickel coating fro use with His6-tag proteins, and streptavidin coating for use with biotinylated proteins (Hall et al, 2007). For detection, two main technologies can be used: 1) direct labelling, single capture assays and 2) dual-antibody sandwich immunoassays (Kingsmore, 2006). In direct labelling, single capture assays, proteins contains in one or more samples are labelled with distinct labels (generally fluorescent or radioisotope labels), hybridized to the microarray, and labelled hybridized proteins are directly detected (Kingsmore, 2006). In dual-antibody sandwich immunoassays, the sample is hybridized to the microarray, and a secondary tagged antibody is added. A third labelled (generally fluorescent or radioisotope label) antibody specific for the tag of the secondary antibody is then used for detection (Kingsmore, 2006). Further details concerning antibodies microarrays may be found in Haab, 2005 and Eckel-Passow et al, 2005. Examples of commercial antibody microarrays include those commercialized by Clontech Laboratories, Invitrogen, Eurogentec, Kinexus etc...

The determination of the survival prognostic according to the method of the invention is carried out thanks to the comparison of the expression profile of the above-mentioned genes with at least one reference profile.

A reference profile is, in the context of the present invention, obtained from a "control sample", i.e. from a test sample obtained from a human subject who is known to be healthy. Preferably, said reference profile has been obtained from several healthy subjects (for example from at least 5 healthy subjects) by measuring the expression level of each gene and by calculating a mean thereof. As used herein, the terms "a control sample of a known healthy human subject" therefore mean "at least one control sample of at least one known healthy human subject".

The comparison of a tested sample with a control sample (or of a tested expression profile to a reference expression profile) can be done using statistical models or machine learning methods which aim is to predict a clinical response (e.g.: 0 if bad prognosis, 1 if good prognosis) based on a combination of the explanatory variables (the genes). Statistical models such as logistic regression and fisher linear discriminant analysis are particularly relevant to predict outcome. Other discriminating algorithms include kNN (k nearest neighbour), decision trees, SVM (support vector machine), NN (neural networks) and forest. The PLS regression, MIPP, sparse linear discrimination and PAM (predictive analysis of microarrays) are particularly relevant to give prediction in the case of pangenomic analysis with small reference samples. To ensure that the predictor is robust, cross validation methods such as leave-one-out should be applied to the models.

The comparison step of the method of the invention can be for example performed by calculating the ratio between the expression level of each gene in the tested sample and in the control reference sample.

Higher expression level of at least two, preferably three, more preferably four genes and even more preferably five genes chosen in the group consisting of: *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) or homologous thereof in said test sample, as compared to said control sample, indicates a long-term survival of said human patient.

As mentioned herein, the term "long-term survival" refers to survival of at least 70 months, preferably 75 months and more preferably 80 months after the sample collection has been performed, the patient being treated or not.

As used herein, the term "higher expression level" means that the expression level of a gene in said test sample is strictly superior to the one in said control sample, because said gene is up-regulated in said test sample. In other words, the term "higher" corresponds to a ratio [expression level in said test sample / expression level in said control sample] which is superior to 1 for said gene.

More precisely, if the ratio [expression level in said test sample / expression level in said control sample] is:

- superior to 1,05, preferably to 1,1, more preferably to 1,14 for the *LIZ* gene;
- superior to 1,05, preferably to 1,1, more preferably to 1,14 for the *ELANE* gene;
- superior to 1,05, preferably to 1,1, more preferably to 1,15 for the *G6PD* gene;
- superior to 1,1, preferably to 1,5, more preferably to 1,22 for the *6PGD* gene;
- superior to 1,1, preferably to 1,5, more preferably to 1,20 for the *TKT* gene; and/or
- superior to 1,25, preferably to 1,3, more preferably to 1,34 for the *CEACAM4* gene,

then said human patient will have a long-term survival (i.e. a survival of at least 70 months, preferably 75 months and more preferably 80 months after the sample collection has been performed).

The expression levels of the five genes *G6PD* (SEQ ID NO:2 or 3), *6PGD* (SEQ ID NO:4), *LILRB1* (SEQ ID NO:5 or 6 or 7 or 8), *TKT* (SEQ ID NO:9 or 10), *CEACAM4* (SEQ ID NO:11) and *ELANE* (SEQ ID NO:23) are measured.

If the expression levels of the five genes *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) are higher in said test sample obtained from the patient, than those of the control sample, then said patient will live longer than 70 months, preferably 75 months and more preferably 80 months.

More precisely, if the ratio [expression level in said test sample / expression level in said control sample] is:

- superior to 1,05, preferably to 1,1, more preferably to 1,15 for the *G6PD* gene;
- superior to 1,05, preferably to 1,1, more preferably to 1,14 for the *ELANE* gene;
- superior to 1,1, preferably to 1,5, more preferably to 1,22 for the *6PGD* gene;
- superior to 1,1, preferably to 1,5, more preferably to 1,20 for the *TKT* gene;
- superior to 1,25, preferably to 1,3, more preferably to 1,34 for the *CEACAM4* gene; and

- inferior to 0,75, preferably to 0,7, and more preferably to 0,66 for the *LILRB1* gene,

then said human patient will have a long-term survival (i.e. a survival of at least 70 months, preferably 75 months and more preferably 80 months after the sample collection has been performed).

On the contrary, and as shown in the results below, if the expression levels of at least one of the five genes *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) are lower in said test sample obtained from the patient, than those of the control sample, then said patient will have a short-term survival, i.e., will live no more than 28 months, preferably 25 months and more preferably 21 months after the sample collection has been performed.

More precisely, if the expression levels of the five genes *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) are lower in said test sample obtained from the patient, than those of the control sample, then said patient will have a short-term survival, i.e., will live no more than 28 months, preferably 25 months and more preferably 21 months after the sample collection has been performed.

In a more particular embodiment, if the ratio [expression level in said test sample / expression level in said control sample] is

- inferior to 1,14, preferably to 1,1, more preferably to 1,05 for the *LIZ* gene; and/or
- inferior to 1,14, preferably to 1,1, more preferably to 1,05 for the *ELANE* gene; and/or
- inferior to 1,15, preferably to 1,1, more preferably to 1,05 for the *G6PD* gene; and/or
- inferior to 1,22, preferably to 1,5, more preferably to 1,1 for the *6PGD* gene; and/or
- inferior to 1,2, preferably to 1,5, more preferably to 1,1 for the *TKT* gene; and/or
- inferior to 1,34, preferably to 1,3, more preferably to 1,25 for the *CEACAM4* gene;

then said human patient will have a short-term survival (i.e. a survival of at least maximally 28 months, preferably 25 months and more preferably 21 months after the sample collection has been performed).

In an even more preferred embodiment, if the ratio [expression level in said test sample / expression level in said control sample] is

- inferior to 1,14, preferably to 1,1, more preferably to 1,05 for the *ELANE* gene; and
- inferior to 1,15, preferably to 1,1, more preferably to 1,05 for the *G6PD* gene; and
- inferior to 1,22, preferably to 1,5, more preferably to 1,1 for the *6PGD* gene; and
- inferior to 1,2, preferably to 1,5, more preferably to 1,1 for the *TKT* gene; and
- inferior to 1,34, preferably to 1,3, more preferably to 1,25 for the *CEACAM4* gene;

then said human patient will have a short-term survival (i.e. a survival of at least maximally 28 months, preferably 25 months and more preferably 21 months after the sample collection has been performed).

Kits according to the present invention are defined in the appended claim 6.

The application also describes a kit for *in vitro* determining the prognosis of chronic myelomonocytic leukaemia in a human patient suffering thereof, comprising:

a) A reagent capable of specifically detecting the expression level of at least two genes chosen in the group consisting of: *G6PD* (SEQ ID NO:2 or 3), *6PGD* (SEQ ID NO:4), *ELANE* (SEQ ID NO:23), *TKT* (SEQ ID NO:9 or 10) and *CEACAM4* (SEQ ID NO:11), and
b) Instructions for using said kit for determining the prognosis of chronic myelomonocytic leukaemia in said human patient.

The kit can also comprise a reagent capable of specifically detecting the expression level of the *LYZ* gene of SEQ ID NO:1.

By "reagent capable of specifically detecting the expression level of" is meant a reagent specifically intented for the specific determination of said expression levels, either on the transcription (RNA) or on the translation (proteic) levels. This definition excludes generic reagents useful for the determination of the expression level of any gene, such as taq polymerase or an amplification buffer, although such reagents may also be included in a kit according to the invention.

In any kit for the *in vitro* prognosis of the survival of CMML suffering patients, the reagent(s) for specifically detecting the expression level of the genes comprising, or consisting of, the 6 genes from Table 1 or homologous thereof, preferably include specific amplification primers and/or probes for the specific quantitative amplification of transcripts of genes of Table 1, and/or a nucleic microarray for the detection of genes of Table 1. The determination of the expression levels may thus be performed using quantitative PCR and/or a nucleic microarray, preferably an oligonucleotide microarray.

In addition, the instructions for the determination of the survival of CMML suffering patients preferably include at least one reference expression profile, or at least one reference sample for obtaining a reference expression profile. Preferably, the determination of the patient survival is carried out by comparison with the test sample and the reference sample as described above.

The application is also directed to a nucleic acid microarray comprising or consisting of nucleic acids specific for the 6 genes from Table 1 or homologous thereof. Said nucleic acid microarray may comprise additional nucleic acids specific for genes other genes. Advantageously, said microarray consists of nucleic acids specific for the 6 genes of Table 1 above. In a preferred embodiment, said nucleic acid microarray is therefore an oligonucleotide microarray comprising or consisting of oligonucleotides specific for the 6 genes from Table 1.

As mentioned above, the man skilled in the art perfectly knows how to design "oligonucleotides specific for a gene" in view of its gene accession number.

All the embodiments concerning nucleic acid microarrays and methods of preparing them have been developed above *de facto* apply to the nucleic acid microarray of the invention.

The present application also relates to a mRNA prognostic signature for predicting outcome of a patient suffering from chronic myelomonocytic leukaemia, independently from other factors, comprising one or more up-regulated mRNAs of the genes chosen in the group consisting of: the *LYZ* (SEQ ID NO:1), *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) genes or homologous thereof, as compared with mRNA of same genes expressed in normal cells.

The expression level of at least three, preferably four, more preferably five genes chosen in the group consisting of the *ELANE* (SEQ ID NO:23), *G6PD* (SEQ ID NO:2 or 3), *6PGD* (SEQ ID NO:4), *TKT* (SEQ ID NO:9 or 10) and *CEACAM4* (SEQ ID NO:11) genes or homologous thereof is measured in the method of the application.

All the embodiments concerning said genes and methods of assessing there expression level that have been developed above *de facto* apply to said mRNA prognostic signature.

Preferably, said expression levels of said genes are measured in PBMC cells, obtained either from the patient, or from a reference healthy human subject.

The present application also relates to a method for determining if patients suffering from chronic myelomonocytic leukaemia will have a short-term survival or a long-term survival comprising the steps of:

a) obtaining a test sample from said human patient,

b) determining the expression level of the at least two genes chosen in the group consisting of: the *LYZ* (SEQ ID NO:1), *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) genes or homologous thereof in said test sample, and

c) applying a predictive model for determining if said patient will have a short-term survival or a long-term survival.

This method enables for example to identify and select the patients belonging to each group (short- or long-term survival patient groups) which can be used in particular clinical trials. It can advantageously be used as pharmacogenomic information in companion diagnostic tests. These pharmacogenomic biomarkers can help differentiate patient into responder and non responder groups, which can help estimate drug effectiveness, avoid toxicity and adverse effects, increase drug safety and adjust drug dosage and are therefore encouraged by several health Authorities.

As a matter of fact, labelling drug has become more difficult within the last 10 years. During the 3 last months Avastin® (Roche Pharmaceutical) has been recalled for breast cancer application and Aflibercept (Sanofi-Aventis) fell in late clinical trials Phase III for lung cancer application. Therefore drug approval agencies, including FDA and EMEA, are encouraging greater use of biomarker and diagnostic in drug development and prescribing decision. This encouragement and guidance has taken several forms (1), (2), including the last *Guidance for Industry Clinical Pharmacogenomics: premarketing Evaluation in early Phase Clinical Studies* (3) issued in February 2011. Proof of concept of the use in clinical studies as well as in prescribing decision of such biomarkers has been achieved for Big Pharma like Genetech Roche with Trastuzumab (Her2), a molecule labelled and associated with Companion Diagnostic. About 10% of labels for drugs approved by the FDA now contain pharmacogenomic information. Such pharmacogenomic biomarkers can thus help to increase the chance to be approved by health Authorities. More conclusively, both FDA and EMEA now require that biomarker testing be performed prior to prescribing certain drugs.

For patient suffering from chronic myelomonocytic leukaemia, the method of the invention enables for example to select those requiring an aggressive treatment (such as bone marrow transplant) from those requiring "only" supportive care (administration of blood product support and/or hematopoietic growth factors).

More precisely, it is considered that short-term survival patient groups will be preferentially included in clinical trials involving bone marrow transplantation (stem cell transplantation), or aggressive chemotherapy, for example with hypomethylating agents such as 5-azacytidine, decitabine, or lenalidomide.

On the contrary, long-term survival patient groups will be preferentially included in clinical trials involving iron uptake, or red blood cell transfusion (optionally with a chelation therapy to avoid iron overload).

In a preferred embodiment, said predictive model is reduced to practice by calculating an index as follows:

First, the expression levels of each of the five genes are measured in a patient sample and are compared to the reference expression. A ratio is calculated, leading to the calculation of a "fold change". This fold change is compared to cut-off values, and patients are then dichotomised (+1 or -1 for gene value under or below the significant cut-off) for each significant gene and pondered by the beta-coefficient of each genes (which have been calculated from Kaplan-Meïer analysis).

In a preferred embodiment, the following cut-offs values and Beta-coefficients are used:

| Gene name | Fold change | Cut-off | Dichotomisation : D = | Beta-coefficient (β) |
|---|---|---|---|---|
| ELANE | b | 3.40 | +1 if b > 3.40 ; -1 if b ≤ 3.40 | 2.01784191521554 |
| G6PD | c | 1.15 | +1 if c > 1.15 ; -1 if c ≤ 1.15 | 1.28224877578792 |
| TKT | d | 1.2 | +1 if d > 1.2 ; -1 if d ≤ 1.2 | 1.35358578043486 |
| PGD | e | 1.22 | +1 if e > 1.22 ; -1 if e ≤ 1.22 | 1.71153730912409 |
| CEACAM4 | f | 1.34 | +1 if f > 1.34 ; -1 if f ≤ 1.34 | 2.0942792881 |

Then, for each patient, the index was calculated by the sum of the dichotomised value pondered by the beta-coefficient of each gene:

$$I = D_{LYZ} \times \beta_{LYZ} + D_{LILRB1} \times \beta_{LILRB1} + D_{G6PD} \times \beta_{G6PD} + D_{TKT} \times \beta_{TKT} + D_{PGD} \times \beta_{PGD} + D_{CEACAM4} \times \beta_{CEACAM4}$$

If the calculated index I is superior to 1, then short-term survival is to be prognosed for said patient.

If the calculated index I is inferior or equal to 1, then long-term survival is to be prognosed for said patient.

Preferably, in this aspect of the invention, the expression level of all the five genes *ELANE* (SEQ ID NO:23), *G6PD* (SEQ ID NO:2 or 3), *6PGD* (SEQ ID NO:4), *TKT* (SEQ ID NO:9 or 10) and *CEACAM4* (SEQ ID NO:11) is measured.

More preferably, said predictive model comprises:

i) calculating the ratio between the expression level of the said genes in said test sample and the expression level of the same genes in a control sample of a known healthy human subject,

ii) comparing said ratio with cut-offs values for each gene and determining the dichotomisation factors for each gene,

iii) pondering said dichotomisation factors by predetermined beta-coefficient for each genes, and

iv) calculating an index I which is the sum of said dichotomised factors pondered by said beta-coefficients of said genes for said patient:

In other words, said index I is calculated as follows:

$I = D_{LIZ} \times \beta_{LYZ} + D_{LILRB1} \times \beta_{LILRB1} + D_{G6PD} \times \beta_{G6PD} + D_{TKT} \times \beta_{TKT} + D_{PGD} \times \beta_{PGD} + D_{CEACAM4} \times \beta_{CEACAM4}$, D being the dichotomisation factor of each gene and β the β coefficient of each gene.

The calculated index I is then compared to the value 1 so as to determine if said patient will have long- or short-term survival:

If the calculated index I is superior to 1, then short-term survival is to be prognosed for said patient.

If the calculated index I is inferior or equal to 1, then long-term survival is to be prognosed for said patient.

Having generally described this invention, a further understanding of characteristics and advantages of the invention can be obtained by reference to certain specific examples and figures which are provided herein for purposes of illustration only and are not intended to be limiting unless otherwise specified.

## EXAMPLES

### Introduction

Chronic myelomonocytic leukaemia (CMML) is a clonal hematopoietic stem cell disorder frequently seen in the elderly. First considered as a myelodysplastic disease in the French American British (FAB) classification (Bennett *et al*., 1994), CMML was reclassified by the World Health Organization (WHO) as a myelodysplastic/myeloproliferative neoplasm (MDS/MPN) (Jaffe *et al*., 2001). This reclassification underlines the heterogeneity of CMML in diagnosis and prognosis. Despite of this heterogeneity, the diagnosis of CMML is definitely straightforward in the presence of a combination of persistent blood monocytosis, fewer than 20% blasts in peripheral blood and bone marrow, absence of *BCR-ABL1* fusion gene and dysplasia in one or more cell lines (Vardiman *et al*., 2002; Orazi & Germing, 2008). According to WHO criteria, blasts include myeloblasts, monoblasts and promonocytes. The myeloid compartment is frequently associated with cytogenetic abnormalities that help to confirm the CMML diagnosis, but none are specific (Reiter *et al*., 2009).

In order to characterize factors predicting the course of the disease, recent data based on mutation identification have been reported, among them *RAS* and *TET2* are the most frequently affected genes. Twenty two per cent of patients exhibit point mutations of *RAS* genes (*NRAS*, *KRAS*) at diagnosis or during the disease course and as many as 50% present *TET2* mutations (Ricci *et al*., 2010; Kosmider *et al*., 2009). With respect to clinical data, Kosmider *et al.* suggest that the prevalence of *TET2* mutations is higher in CMML than in any other myeloid disease and is associated with a trend to a lower overall survival rate. On the other hand, by applying next-generation sequencing (NGS) technology, two recent reports detected frequent aberrations in the *TET2* gene in CMML cases and related it to better outcome (Kohlmann *et al*., 2010; Grossmann *et al*., 2011).

Currently, no reliable molecular prognostic markers are available with an easy technology in CMML in spite of the recent WHO reclassification. The difficulty of the clinical classification and the variable risk of progression to acute myeloid leukemia (AML) remain the major problems for physicians.

In light of these issues, we have chosen to perform gene expression profiling (GEP) as molecular studies in CMML using this approach, have not been extensively explored (Theilgaard-Monch *et al*., 2011). The aim of our study was to identify molecular predictors, from 32 CMML peripheral blood mononuclear cells (PBMC), associated with better survival and to validate its performance in an independent test set of 21 CMML samples. The present work shows that GEP has a prognosis potential in CMML and could help improving the classification of the disease.

### Design and Methods

*Patients all control samples*

CMML diagnosis was defined according to the World Health Organization (WHO) criteria, as previously published (Reiter *et al*., 2009; Orazi & Germing, 2008; Vardiman *et al*., 2002). The patients signed informed consent to participation in the study in accordance with the Declaration of Helsinki. The study was approved by the ethic board of Nimes University. PBMCs were collected in the Centre Hospitalier Universitaire (CHU) of Nimes from 32 patients who were newly diagnosed. All samples in this study were obtained from untreated patients at the time of diagnosis. For 14 patients, paired material at presentation and at different periods of follow-up was also available for gene expression analyses. Sixteen blood samples of acute myeloid leukaemia (AML) and two samples of proliferative and differentiated U937 leukaemia cells, cultured as previously described (Piquemal *et al*., 2002), were also included in the analyses. AML samples include 4 *de novo* and 12 secondary AML (transformed CMML). Control samples of PBMC obtained from three healthy donors were used as reference.

*Molecular markers screening*

Genes were selected from transcriptomic data established by SAGE methodology from acute myeloid leukaemia models, normal polymorphonuclear and monocytic cells (Piquemal *et al*., 2002; Bertrand *et al*., 2004; Quere *et al*., 2007; Rivals *et al*., 2007). Differential gene expression analyses were performed as previously described (Piquemal *et al*., 2002). SAGE libraries data are available at GEO (http://www.ncbi.nlm.nih.gov/geo/) under accession number GSM32698: untreated U937 cell line; GSM32699: differentiated U937 cell line; GSM151619: untreated NB4 cell line; GSM151622: differentiated NB4 cell line. The SAGE libraries were described in Rivals *et al* and Bertrand *et al* for normal monocytes and granulocytes, respectively (Rivals *et al*., 2007; Bertrand *et al*., 2004). By mining the SAGE data, 92 transcripts showing significant variation following myeloid cell differentiation and 1 calibration marker (RPS19) were selected for high-throughput real-time polymerase chain reaction (PCR) analysis. The listing of the 93 genes is provided in supplementary data. They correspond to transcripts over-expressed in leukaemia differentiated cells, cell cycle genes and transcripts already known as cancer-related genes (Piquemal *et al*., 2002; Quéré *et al*., 2007). We used also Affymetrix data of 21 CMML samples from the Microarray Innovations in Leukaemia (MILE) study (Haferlach *et al*., 2010). All samples were obtained from untreated patients at the time of diagnosis. These data are publicly available via GEO under accession number GSE13204. Information on survival and clinical parameters were provided by Pr Mills's group.

*RNA extraction, reverse transcription, and high-throughput real-time PCR*

RNA was extracted with RNeasy Qiagen kit. RNA quality was monitored and quantified using the 2100-Bioanalyzer (Agilent Technologies, Waldronn, Germany). Reverse transcription was performed with random primers (High-capacity cDNA Archive kit; Applied Biosystems, Courtaboeuf, France) using 1 μg total RNA. PCR analyses were performed on microfluidic cards with 100ng of cDNA, using the TaqMan® Gene Expression Assays and the ABI7900HT system (Université de Limoges Q-PCR facility). Analysis of the relative quantity gene expression (RQ) data was performed using the $2^{-\Delta\Delta Ct}$ method (Livak & Schmittgen, 2001). Transcriptional modulation ($log_{10}$ RQ) was calculated using data from normal PBMCs as reference. Data were collected and analysed with Sequence Detector Software (SDS2.2; Applied Biosystems). Similar results were obtained from relative quantity gene expression comparisons using the 3 calibrator genes. For the final normalization, RPS19 was selected. The accuracy of the technology was validated by testing the reliability of SAGE and the high-throughput real-time PCR. Among the differentially expressed markers selected from the SAGE data ($P \leq .01$), 95% displayed significant modulation when tested on microfluidic cards. Standard error (SE) was measured using U937 samples already tested in a separate study. Paired samples from 26 patients were tested to evaluate the reproducibility of our method. In the unsupervised hierarchical cluster, each sample and its duplicate came out together in the same subtype.

*Statistical analysis*

Genes with no measured expression in all samples were discarded. A total of 93 genes were selected for unsupervised analysis. Hierarchical clusters were performed with the Cluster and Treeview softwares from Eisen *et al* (Eisen *et al*., 1998). Gene expression data was analysed with SAM (Significance Analysis of Microarrays) software with a 1000-permutations adjustment (Cui & Churchill, 2003). For each selected gene, the patients' samples were ordered by low to high expression values. For each increasing signal position in this scale, the overall survival difference between patients having a lower or equal versus a higher signal was assessed using a log-rank test with the Maxstat package used in R software (http://cran.r-project.org/). Overall survival of subgroups of patients was compared with the log-rank test and survival curves computed with the Kaplan-Meïer method (R software; survival package). Benjamini and Hochberg Multiple Testing correction was used to select the strongest genes associated with the overall survival (Camargo *et al*., 2008). At rank one, this within-probe adjustment is realized by multiplying the maximum P-value by the number of calculated positions. Genes with P value >.05 were discarded (Carlin & Chib, 1995). For the index computation, first patients were dichotomised (+1 or -1 for gene value under or below the significant cut-off) for each significant gene and pondered by the beta-coefficient (issued from Kaplan-Meïer analysis). Then, for each patient, the index was calculated by the sum of the dichotomised value pondered by the beta-coefficient of each gene (Kassambara *et al*., 2011). Statistical comparisons were done with Mann-Whitney, Chi-square, or unpaired or paired Student's *t* tests.

The networks were generated through the use of Ingenuity Pathways Analysis (Ingenuity® Systems, www.ingenuity.com). A data set containing gene identifiers and corresponding expression values was uploaded into the application. Each gene identifier was mapped to its corresponding gene object in the Ingenuity Pathways Knowledge Base. These genes, called focus genes, were overlaid onto a global molecular network developed from information contained in the Ingenuity Pathways Knowledge Base. Networks of these focus genes were then algorithmically generated based on their connectivity. Gene expression data were extracted from the Oncomine Cancer Microarray database (http://www.oncomine.org) (Rhodes *et al*., 2004) and the Amazonia database (http://amazonia.montp.inserm.fr) (Le Carrour *et al*., 2010).

**Results**

*Patients*

A total of 32 CMML patients including 21 males (66%) and 11 females (34%) were studied. Their main clinical and haematological characteristics are shown in Table I. We had same proportions of different clinical parameters as previously described (Such *et al*., 2011). Median age was 76 years (range 45-86). According to FAB criteria, 15 patients (47%) had MD-CMML and 17 patients (53%) had MP-CMML. According to WHO classification, 27 patients (90%) were diagnosed as having CMML-1 and 3 patients (10%) as having CMML-2. Karyotype was normal in 20 patients (63%) and abnormal in 4 patients (13%); data were not available for 8 patients (25%). Among cytogenetic aberrations, we find one patient with trisomy 8, one patient with monosomy 7, one patient with loss of the Y chromosome and one patient with other anomalies. Five patients developed acute myeloid leukaemia of which three showed an abnormal karyotype. There were no significant differences in blast proportion in patients' bone marrow.

*Gene expression-based analyses defines two subsets of CMML patients*

We undertook a comparison study of gene expression variation between different clinical samples. Gene expression data were generated from PBMC cDNA obtained for 32 CMML patients and their paired samples, 4 *de novo* AML patients and 2 samples of proliferative or differentiated U937 cells using microfluidic low density arrays. Using an unsupervised hierarchical clustering approach, two main groups of samples were defined: G1 and G2 . *De novo* and secondary AML and U937 samples came out together in the G1 group, while all CMML samples clustered in the G2 group, which was subdivided into two subgroups: G2A and G2B. In order to select genes which could highly discriminate between the identified subgroups, we employed a supervised approach using Significance Analysis of Microarrays (SAM) tool. Twenty-eight genes passed SAM analysis with a false discovery rate (FDR) <5%. These genes were selected as a 'predictor set' for survival. They enabled the characterization of two categories of patients with different gene signatures (Fig 1). We next investigated which of them are known to interact biologically by carrying out pathway analysis using the Ingenuity Pathway Analysis (IPA) tool. Twenty-two genes mapped to genetic networks and two networks were found to be highly significant (51 and 18 as respective scores). They were mainly associated with cell cycle, DNA replication, and cellular growth and proliferation.

*'Survival Index' scoring and biological significance*

In order to stringently identify a gene signature predictive of survival, we aimed to construct a 'prognosis index' which can separate categories of patients with different survival. To do so, overall survival (OS) curves were plotted for each gene in the 'predictor set' and P-values were corrected by Benjamini and Hochberg multiple testing correction. Five genes showed a significant bad prognostic value: *G6PD;* Glucose-6-phosphate dehydrogenase, *PGD;* 6-phosphogluconate dehydrogenase; *TKT;* Transketolase, *ELANE*; Neutrophil elastase and *CEACAM4;* Carcinoembryonic antigen-related cell adhesion molecule 4. We computed the 'prognosis index' by combining the prognostic information of the five selected genes as described in *materials and methods.* OS curve was plotted (Fig 2). Patients were distributed between two groups: good (dotted grey) and poor survival (black) with 50% of patients in each group. As shown in figure 2, OS was significantly increased in patients with low survival index score. The 10-year OS was 94% in the good prognosis group versus 19% in the poor prognosis group.

We compared the expression of our 5 genes in a panel of 16 cancer types to their normal counterparts using the Oncomine Cancer Microarray database, a publicly available gene expression data (Table II). Interestingly, the 5 genes are expressed at least in 1/3 haematological cancers and 4/13 solid tumours. *TKT* was found to be over-expressed in leukaemia, lymphoma, myeloma and expressed in 10/13 solid tumours. When comparing their expression profiles in various normal haematological tissues using the public microarray database Amazonia, *TKT, G6PD, PGD, ELANE* and *CEACAM4* displayed a myeloid phenotype and were expressed in normal bone marrow (Fig 3). *ELANE* shows a promyelocytic restricted pattern, as *TKT, G6PD, PGD* and *CEACAM4* are also expressed in immature and differentiated granulocytes and in monocyte populations.

*Index association with clinical characteristics and validation*

We investigated association of the index survival groups obtained with clinical and biological characteristics. We observed no specific pattern with age, gender and cytogenetic abnormalities. As shown in table III, there were neither association with FAB, WHO and IPSS (International Prognosis Scoring System) classification systems. Anyhow, with respect to clinical data, no significant prognostic difference was detectable for MD-CMML and MP-CMML categories (*P*=*.39*, *data not shown*). Yet, due the limited number of CMML-2 compared to CMML-1 cases, we did not separate the cohort into these two categories in subsequent analyses.

With regard to treatment and AML transformation, 76% of treated patients were found in the group of worse survival. This correlated with progression, as all AML-transformed patients were also included in this category. This observation could suggest a more aggressive disease that progresses over time.

Assuming that our 'prognosis index' is able to discriminate between different clinical samples, we sought to demonstrate its robustness and prognostic independence in a new cohort of 21 CMML patients that were included in the MILE study (Haferlach *et al*., 2010). Briefly, this cohort consists of 15 patients (71%) with normal karyotype and 6 patients (29%) with abnormal karyotype. Median age was 74,7 years. IPSS varied between favourable (11 patients, 52%) and intermediate-1 (9 patients, 48%). 4 patients (19%) evolved to AML. We performed an index based survival analysis using the new cohort. Contrary to our gene expression data obtained from TaqMan low density arrays, we used here HG-U133Plus2.0; Affymetrix data. Despite that, we successfully identified two categories of patients with significant outcomes (*P*=*.03*) (Fig. 4A). Samples were equally distributed in each group. Similarly, we observed no specific correlation between the obtained classification of samples and other clinical and biological characteristics. When adding the two cohorts together (53 patients in total) (Fig. 4B), the statistical prognostic value was yet increased (*P*=*.002*). These results shows that our five gene based 'prognosis index' could be adapted to other cohorts of CMML with distinct types of gene expression data. It could be a powerful tool to predict clinical outcome and to discover novel subclasses for this malignancy.

## Discussion

In haematological malignancies, GEP allowed for detecting new biologically and prognostically relevant subtypes despite the genetic heterogeneity of the disease (Moreaux *et al*., 2011; Wouters *et al*., 2009; Bresolin *et al*., 2010). The objective of our study was to select genetic markers which could be proposed as new tool for prognosis in CMML. Using microfluldic low density arrays, we profiled a series of 32 untreated CMML patients at diagnosis. By supervised analysis, we identified 28 out of the 93 selected genes. We then established a five-gene prognostic index potentially more easily applicable in daily clinical practice. Using this index, we classified patients, independently from classical prognostic features, in two groups with different clinical outcome: a good class with 10-year OS of 94%, and a poor class with 10-year OS of 19%. Importantly, the strength and prognostic independent value of our survival index was successfully checked on a validation cohort of 21 CMML patients with data obtained from Affymetrix microarrays. All together, we demonstrated the usefulness of GEP prognostic in CMML regardless of the quantitative gene expression method.

The significant networks we identified as related to cell cycle, DNA replication, and cellular growth and proliferation corroborated with published data. Alterations in biological processes that contribute to an adaptation of tumour cells and an increase of their aggressiveness were also observed. Among our prognostic predictors, we found *G6PD, TKT* and *PGD* which displays a significant function in glycolysis by regulating the pentose phosphate pathway. They favour the production of ribose which is essential for RNA and DNA synthesis in rapidly growing cells. Deregulation of this metabolic pathway radically alters *G6PD, TKT* and *PGD* genes promoting tumour cell proliferation and poor prognosis; hence their elevated levels of expression and activity in breast, colon and various other types of cancer (Baba *et al*., 1989; Toyokuni *et al*., 1995; Furuta *et al*., 2010). *CEACAM4*, a carcinoembryonic antigen (CEA) family member, is uniquely expressed on primary human granulocytes (Schmitter *et al*., 2007). CEACAM proteins are well-known markers associated with progression of colorectal tumours. Interestingly, the Oncomine Cancer Microarray database confirms that four out of our five outcome predictors are over expressed in haematological cancers and solid tumours. *TKT* was the more frequently involved as it was found to be over-expressed in leukaemia, lymphoma, myeloma and major solid tumours.

In the same way, the molecular markers identified in the present study could facilitate the identification of key pathways and abnormal cell subtypes involved in CMML. When comparing expression profiles of the five genes in various haematological tissues, all of them displayed a myeloid phenotype as they are mainly expressed in immature and differentiated granulocytes. *ELANE* shows the more restricted phenotype as it's exclusively expressed in promyelocytic cells. Recently, Droin *et al.* (2010) explored the cellular heterogeneity of the leukaemia clone and underlined the presence of immature dysplastic granulocytes in the peripheral blood. These cells, clearly distinct from $CD14^+$ monocytes, belong to the tumoral population and highly express *CEBPE* and *GFI1,* two transcription factors involved in the myeloid lineage that controls *ELANE* gene expression, one of the detected molecular markers. It's not clear if this granulocytic immature population is present in all CMML patients but in the present study, it's noteworthy that high expression levels of promyelocytic and immature granulocyte markers with cell cycle characteristics correlate with a poor prognostic. It would be interesting to determine if the molecular predictors correlate with the presence of distinct leukemia cell populations in the peripheral blood with specific proliferative status.

In conclusion, we have developed, and validated in two independent series of samples, a five-gene index associated with survival. The heterogeneity of the disease reflected by the current classification system doesn't sufficiently contribute to stratify high risk patients. As already described from microarray data analysis of myelodysplastic syndromes (Mills *et al*., 2009), our data demonstrated the prognostic potential of GEP in CMML and revealed the heterogeneity of this disorder that would be essential for therapeutic proposals. Indeed, the poor survival profile seems to correlate with a more aggressive disease as the group included most of patients receiving a treatment and those presenting a high risk of AML transformation. Conversely, the fact that the favourable group is mainly characterised by the absence of treatment, could reflect a more indolent form. Furthermore, a better understanding of the implication of these genes in CMML and their power in respect to prognosis could be of clinical interest for physicians.

| N° | sex/age (years) | Peripheral Blood | | Bone Marrow | | | Treatment at sampling | | | ***Progression** after sampling* | *Time to **progression** (montbs)* |
|---|---|---|---|---|---|---|---|---|---|---|---|
| | | *WBC (G/l)* | *Monocytes (G/l)* | *Blasts (%)* | *Monocytes (%)* | *Karyotype* | *Chemo.* | *Transfu.* | *CSF* | | |
| 1 | M/.83 | 17,4 | 1,65 | ND | ND | ND | no | no | no | no | |
| 2 | M/45 | 30,0 | 3,9 | | 7 14 | 45 XY, -7 | no | yes | yes | AML 4 | 13 |
| 3 | M/66 | 11,8 | 1,9 | 1 | 4 | 46 XY | no | no | no | no | |
| 4 | F/84 | 2,8 | 1,1 | 3 | 6 | ND | no | yes | no | no | |
| 5 | M/82 | 12,9 | 2,7 | 1 | 12 | ND | no | yes | no | no | |
| 6 | M/62 | 6,2 | 1,9 | 1 | 1 | 46 XY | no | yes | no | no | |
| 7 | M/68 | 18,3 | 6,6 | 3 | 25 | 46 XY | yes | yes | no | no | |
| 8 | M/85 | 13,3 | 6,7 | 11 | 28 | ND | no | no | no | no | |
| 9 | M/62 | 10,3 | 3,4 | 4 | 15 | 46 XY | yes | yes | no | AML1 | 48 |
| 10 | M/73 | 7,0 | 2,2 | 2 | 6 | 46 XY | no | yes | no | no | |
| 11 | M/73 | 4,6 | 1,4 | 4 | 18 | ND | no | yes | no | no | |
| 12 | M/73 | 4,2 | 0,9 | 3 | 14 | 45 X, -Y | no | no | no | no | |
| 13 | M/68 | 23,3 | 4,1 | 2 | 8 | 46 XY | no | no | no | no | |
| 14 | F/83 | 12,1 | 2,1 | 0 | 10 | 46 XX | no | no | no | no | |
| 15 | M/80 | 9,4 | 2,5 | ND | ND | ND | no | no | no | no | |
| 16 | M/81 | 4,0 | 1,0 | 2 | 6 | 46 XY | no | no | no | no | |
| 17 | F/85 | 6,6 | 1,8 | 1 | 7 | ND | no | yes | yes | no | |
| 18 | M/69 | 37,8 | 6,4 | 1 | 5 | 46 XY | no | yes | no | no | |
| 19 | M/80 | 10,5 | 3,0 | 2 | 7 | 46 XY | no | yes | no | AML 4 | 8 |
| 20 | M/55 | 19,0 | 1,7 | 1 | 4 | 46 XY | no | no | no | no | |
| 21 | M/57 | 5,9 | 2,9 | 2 | 9 | 46 XY | no | no | no | no | |
| 22 | F/79 | 30,6 | 2,8 | 1 | 1 | 46 XX | no | no | no | no | |
| 23 | F/86 | 4,4 | 1,2 | 2 | 29 | 46 XX | yes | no | yes | no | |
| 24 | M/76 | 15,8 | 3,8 | 11 | 10 | 46 XY | no | yes | no | no | |
| 25 | F/65 | 11,1 | 2,2 | 1 | 10 | 46 XX | no | no | no | no | |
| 26 | F/82 | 6,6 | 1,4 | 1 | 9 | 46 XX | no | no | no | no | |
| 27 | M/80 | 73,1 | 13,2 | 2 | 11 | 46 XY, t(13;22), del(13) | yes | yes | no | AML 4 | 32 |
| 28 | M/81 | 5,4 | 1,6 | 1 | 11 | 46 XY | no | yes | no | no | |
| 29 | F/73 | 9,9 | 1,9 | | 5 3 | ND | yes | yes | no | no | |
| 30 | F/79 | 6,9 | 1,7 | 4 | 14 | 47 XX, +8 | yes | yes | no | AML 4 | 17 |

(continued)

| | | Peripheral Blood | | Bone Marrow | | | Treatment at sampling | | | **Progression** after sampling | Time to **progression** (montbs) |
|---|---|---|---|---|---|---|---|---|---|---|---|
| N° | sex/age (years) | WBC (G/l) | Monocytes (G/l) | Blasts (%) | Monocytes (%) | Karyotype | Chemo. | Transfu. | CSF | | |
| 31 | F/76 | 13,7 | 4,5 | 1 | 6 | 46 XX | no | no | no | no | |
| 32 | F/75 | 6,9 | 2,1 | 8 | 12 | 46 XX | no | no | no | no | |

**Table II: Expression of genes encoding *TKT*, *G6PD*, *PGD*, *ELANE* and *CEACAM4* in human cancer samples in comparison to their normal counterparts.**

Expression data were obtained from the Oncomine Cancer Microarray database. Genes which were over- and under-expressed in cancer cell samples in comparison with their normal counterpart are indicated in this table.

| | Cancer sample type | Genes over- and under-expressed in cancer samples in comparison to their normal tissue counterpart | | | | |
|---|---|---|---|---|---|---|
| | | *TKT* | *G6PD* | *PGD* | *ELANE* | *CEACAM4* |
| Haematological cancer | Leukaemia | **Up** | **Up** | Down | **Up** | Down |
| | Lymphoma | **Up** | - | **Up** | - | - |
| | Myeloma | **Up** | - | - | - | - |
| Solid Tumours | Bladder cancer | **Up** | - | **Up** | - | - |
| | Brain cancer | Down | - | - | **Up** | - |
| | Breast cancer | Down | - | Down | - | - |
| | Colorectal cancer | **Up** | **Up** | **Up** | Down | Down |
| | Gastric cancer | **Up** | - | - | Down | - |
| | Liver cancer | **Up** | **Up** | - | - | - |
| | Lung cancer | **Up** | - | **Up** | - | - |
| | Melanoma | **Up** | **Up** | - | - | - |
| | Ovarian cancer | **Up** | **Up** | - | **Up** | - |
| | Pancreatic cancer | Down | **Up** | - | - | Down |
| | Prostate cancer | **Up** | - | - | - | - |
| | Renal cancer | **Up** | **Up** | - | **Up** | Down |
| | Testicular cancer | **Up** | - | **Up** | - | - |

**Table III: Correlations between results of the 'Survival Index' classification and patients' clinical and biological characteristics**

| | Good survival group (n=16) | | Poor survival group (n=16) | |
|---|---|---|---|---|
| **Characteristic** | **No.** | **%** | **No.** | **%** |
| Median Age at Diagnosis | 73,3 | | 74,6 | |
| Gender | | | | |
| *Female/Male* | 5/11 | 31/69 | 6/10 | 37/63 |
| Cytogenetic abnormalities (data available for 24 patients) | | | | |
| *Normal Karyotype* | 10 | 91 | 10 | 77 |
| *Abnormal karyotype* | 1 | 9 | 3 | 23 |
| FAB classification (data available for 32 patients) | | | | |
| *MD-CMML* | 7 | 44 | 8 | 50 |
| *MP-CMML* | 9 | 56 | 8 | 50 |
| WHO classification (data available for 30 patients) | | | | |
| *CMML-1* | 12 | 86 | 15 | 94 |
| *CMML-2* | 2 | 14 | 1 | 6 |
| IPSS classification (data available for 24 patients) | | | | |
| *Favourable* | 9 | 50 | 9 | 50 |
| *Int-1* | 1 | 25 | 3 | 75 |
| *Int-2* | 1 | 50 | 1 | 50 |
| Treatment during follow-up (data available for 32 patients) | | | | |
| *Chemotherapy* | 1 | 17 | 5 | 83 |
| *Supportive treatment (Transf & HGF)* | 3 | 27 | 8 | 73 |
| *All treatments* | 4 | 24 | 13 | 76 |
| AML-transformation (data available for 32 patients) | 0 | 0 | 5 | 100 |

Abbreviations: FAB, French-American-British classification; MD, Myelodysplastic; MP, Myeloproliferative; WHO, World Health Organisation; IPSS, International Prognosis Scoring System; Int-1, Intermediate 1; Int-2, Intermediate 2; HGF, Hematopoietic Growth Factors.

**BIBLIOGRAPHY**

Baba M et al., Int. J. Cancer 1989; 43(5):892-5
Benjamini Y, 1995, Journal of the royal statistical society B57, 289-300
Bennett, J.M., (1994) British Journal of Haematology, 87, 746-754.
Bertrand G et al. J Immunol Methods. 2004 ; 292(1-2):43-58.
Bonafoux B and Commes T, Methods Mol Biol. 2009; 496:299-311
Bresolin, S., (2010) Journal of Clinical Oncology, 28, 1919 -1927.
Camargo A et al., Source Code Biol Med, 2008; 3:15
Carlin & Chib (1995) Journal of the Royal Statistical Society, Series B57, 473-184.
Cui X, et Churchill GA, Genome Biol. 2003;4(4):210
Droin, N., (2010). Blood, 115, 78-88.
Eckel-Passow JE et al. Cancer Res. 2005 Apr 15;65(8):2985-9
Eisen MB, et al. Proc Natl Acad Sci USA. 1998;95:14863-14868
Furuta E et al.,Biochimica et Biophysica Acta, 2010 ; 1805(2):141-52
Grossmann, V., (2011). Leukemia, 25, 877-879.
Haab BB. Mol Cell Proteomics. 2005 Apr;4(4):377-83
Haferlach, T., (2010). Journal of Clinical Oncology, 28, 2529 -2537.
Hall DA et al. Mech Ageing Dev. 2007 Jan;128(1):161-7
Jaffe, E.S., (2001) Lyon: IARC Press.
Janssen WE et al Cytotherapy, 2010; 12(3):418-24
Kassambara, A. (2011) Haematologica, DOI: 10.3324/haematol.2011.046821.
Kingsmore SF. Nat Rev Drug Discov. 2006 Apr;5(4):310-20
Kohlmann, A. et al., Jornal of Clinical Oncology, 2010; 28(24):3858-65
Kosmider O et al., Haematologica, 2009 ; 94(12):1676-81
Le Carrour, T., (2010) The Open Bioinformatics Journal, 4, 5-10.
Lee HS et al., Life Sciences, 2007; 80(7):690-8
Lee, HJ et al., Gastroenterology, 2010 ;139(1):213-25.
Livak KJ & Schmittgen TD. Methods. 2001; 25(4):402-8
Mills, K.I., (2009) Blood, 114, 1063 -1072.
Moreaux, J., (2011). Haematologica, 96, 574-582.
Orazi, A. & Germing, U. Leukemia, 2008; 22(7):1308-19
Piquemal et al, Genomics 2002 ; 80(3):361-71
Quere R et al. Blood 2007; 109(10):4450-60
Reiter, A., (2009) Haematologica, 94, 1634-1638.
Rhodes, D.R., (2004) Neoplasia (New York, N.Y.), 6, 1-6.
Ricci, C. et al., Clinical Cancer Research, 2010; 16(8):2246-56
Rivals, E., (2007). Nucleic Acids Research, 35, e108-e108.
Schmitter, T., (2007) Infection and Immunity, 75, 4116-4126.
Such, E., et al. (2011). Haematologica, 96, 375-383.
Surasilp T et al., Mol Cell Probes. 2011
Tefferi A et al., Leukemia, 2009; 23(5):900-4
Theilgaard-Monch, K., (2011). Leukemia, 25, 909-920.
Toyokuni S et al., FEBS Letters 1995; 358(1):1-3
Vardiman, J.W., (2002). Blood, 100, 2292-2302.
Wouters, B.J., (2009). Blood, 113, 291 -298.

## Claims

**1.** A method for *in vitro* determining the prognosis of chronic myelomonocytic leukaemia in a human patient suffering thereof, comprising the following steps:

a) measuring the expression level of the five genes G6PD (SEQ ID NO:2 or 3), 6PGD (SEQ ID NO:4), TKT (SEQ ID NO:9 or 10), CEACA*M4* (SEQ ID NO:11) and ELANE (SEQ ID NO:23), in a test sample which is a sample of peripheral blood mononuclear cells (PBMC) of said human patient,
b) comparing said expression levels to the expression level of said at least five genes in a control sample of a known healthy human subject,
c) predicting the outcome of the chronic myelomonocytic leukaemia in said patient.

**2.** The method according to claim 1, wherein higher expression level of at least the *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) genes in said test sample, as compared to said control sample, indicates a long-term survival of said human patient.

**3.** The method according to claim 1, wherein lower expression level of at least the *G6PD* (SEQ ID NO:2 or 3), *6PGD* (SEQ ID NO:4), *TKT* (SEQ ID NO:9 or 10), *CEACAM4* (SEQ ID NO:11) and *ELANE* (SEQ ID NO:23) genes, indicates a short-term survival of said human patient.

**4.** The method according to any one of claims 1 to 3, wherein step a) comprises measuring the levels of the RNA transcripts or the cDNA of the said genes by employing nucleic acid based detection methods such as microarrays, quantitative PCR, DNA chips, hybridization with labelled probes, or flow lateral dipstick.

**5.** The method according to any one of claims 1 to 3, wherein step a) comprises measuring the levels of the respective proteins of the said genes by employing antibody-based detection methods such as immunohistochemistry or western blot analysis.

**6.** A kit for determining the prognosis of chronic myelomonocytic leukaemia in a human patient suffering thereof, comprising specific amplification primers and/or probes for the specific quantitative amplification of transcripts to specifically detect the level of expression of at least the five genes: *G6PD* (SEQ ID NO:2 or 3), *6PGD* (SEQ ID NO:4), *TKT* (SEQ ID NO:9 or 10) *CEACAM4* (SEQ ID NO:11), and *ELANE* (SEQ ID NO:23).

**7.** A method for determining if patients suffering from chronic myelomonocytic leukaemia suffering will have a short-term survival or a long-term survival, comprising the steps of:

a) obtaining a test sample from said human patient,
b) determining the expression level of the five genes G6PD (SEQ ID NO:2 or 3), 6GPD (SEQ ID NO:4), TKT (SEQ ID NO:9 or 10), CEACAM4 (SEQ ID NO:11) and ELANE (SEQ ID NO:23) in said test sample, which is a sample of peripheral blood mononuclear cells (PBMC) of said patient,
c) applying a predictive model for determining if said patient will have short-term survival or long-term survival.

**8.** Method according to claim 7, wherein said predictive model comprises:

i) calculating the ratio between the expression level of the said genes in said test sample and the expression level of the same genes in a control sample of a known healthy human subject,
ii) comparing said ratio with cut-offs values for each gene and determining the dichotomisation factors for each gene,
iii) pondering said dichotomisation factors by predetermined beta-coefficient for each genes, and
iv) calculating an index I which is the sum of said dichotomised factors pondered by said beta-coefficients of said genes for said patient.

**9.** Method according to claim 8, wherein said cut-offs values are as follows:

| **Gene name** | **Cut-offs** |
|---|---|
| ELANE | 3.40 |
| G6PD | 1.15 |
| TKT | 1.2 |
| PGD | 1.22 |
| CEACAM4 | 1.34 |

and dichotomisation factors are calculated as follows:

| **Gene name** | **Fold change** | **Dichotomisation factor : D =** |
|---|---|---|
| ELANE | b | +1 if b >3.40; -1 if b ≤ 3.40 |
| G6PD | c | +1 if c > 1.15 ; -1 if c ≤ 1.15 |
| TKT | d | +1 if d > 1.2 ; -1 if d ≤ 1.2 |
| PGD | e | +1 if e > 1.22 ; -1 if e ≤ 1.22 |
| CEACAM4 | f | +1 if f > 1.34 ; -1 if f ≤ 1.34 |

**10.** Method according to claims 8 or 9, wherein said beta-coefficients are as follows:

| Gene name | Beta-coefficient (β) |
|---|---|
| ELANE | 2.01784191521554 |
| G6PD | 1.28224877578792 |
| TKT | 1.35358578043486 |
| PGD | 1.71153730912409 |
| CEACAM4 | 2.0942792881 |

## Patentansprüche

1. Verfahren zur *in vitro*-Bestimmung der Prognose von chronischer Monozytenleukämie bei einem daran leidenden menschlichen Patienten, umfassend die folgenden Schritte:

   a) Messen des Expressionslevels der fünf Gene G6PD (SEQ ID Nr. 2 oder 3), 6PDG (SEQ ID NR. 4), TKT (SEQ ID Nr. 9 oder 10), CEACAM4 (SEQ ID NR. 11 und ELANE (SEQ ID NR. 23) in einer Testprobe, die eine Probe aus peripheren mononuklearen Blutzellen (PBMC) des genannten menschlichen Patienten ist,
   b) Vergleich der genannten Expressionslevel mit dem Expressionslevel der genannten wenigstens fünf Gene in einer Kontrollprobe eines bekanntlich gesunden menschlichen Probanden,
   c) Vorhersagen des Ausgangs der chronischen Monozytenleukämie bei dem genannten Patienten.

2. Verfahren gemäß Anspruch 1, bei dem der höhere Expressionslevel wenigstens der Gene *G6PD* (SEQ ID NR. 2 oder 3), *6PDG* (SEQ ID NR. 4), *TKT* (SEQ ID NR. 9 oder 10), *CEACAM4* (SEQ ID NR. 11) und *ELANE* (SEQ ID NR. 23) in den genannten Testproben im Vergleich zu der genannten Kontrollprobe ein langfristiges Überleben des genannten menschlichen Patienten voraussagt.

3. Verfahren gemäß Anspruch 1, bei dem der niedrigere Expressionslevel wenigstens der Gene *G6PD* (SEQ ID NR. 2 oder 3), *6PDG* (SEQ ID NR. 4), *TKT* (SEQ ID NR. 9 oder 10), CEACA*M4* (SEQ ID NR. 11) und *ELANE* (SEQ ID NR. 23) eine kurzfristiges Überleben des genannten menschlichen Patienten anzeigt.

4. Verfahren gemäß Anspruch 1 bis 3, bei dem Schritt a) die Messung der Levels der RNS-Transkripte oder der cDNA der genannten Gene durch Verwendung von auf Nukleinsäure basierenden Detektionsverfahren umfasst, wie z. B. Microarrays, quantitative PCR, DNA-Chips, Hybridisierung mit gekennzeichneten Sonden oder lateralem Strömungsmessstab.

5. Verfahren gemäß irgendeinem der Ansprüche 1 bis 3, bei dem Schritt a) die Messung der Levels der jeweiligen Proteine der genannten Gene durch Verwendung von auf Antikörpern basierenden Verfahren umfasst, wie z. B. Immunohistochemie oder Western Blot-Analyse.

6. Kit zur Bestimmung der Prognose einer chronischen Monozytenleukämie bei einem daran leidenden menschlichen Patienten, umfassend spezifische Amplifikationsprimer und / oder Sonden für die spezifische quantitative Amplifikation von Transkripten, um spezifisch den Expressionslevel von wenigstens der fünf Gene zu detektieren: *G6PD* (SEQ ID NR. 2 oder 3), *6PGD* (SEQ ID NR. 4), *TKT* (SEQ ID NR. 9 oder 10) *CEACAM4* (SEQ ID NR. 11) und *ELANE* (SEQ ID NR. 23).

7. Verfahren zur Bestimmung, ob an chronischer Monozytenleukämie leidende Patienten kurzfristig überleben oder langfristig überleben werden, umfassend die Schritte:

   a) Einholen einer Testprobe von dem genannten menschlichen Patienten,
   b) Bestimmen des Expressionslevels der fünf Gene G6PD (SEQ ID NR. 2 oder 3), 6PDG (SEQ ID NR. 4), TKT (SEQ ID NR. 9 oder 10), CEACAM4 (SEQ ID NR. 11) und ELANE (SEQ ID NR. 23) in der genannten Testprobe, die eine Probe aus peripheren mononuklearen Blutzellen (PBMC) des genannten Patienten sind,
   c) Anwenden eines Vorhersagemodells zur Bestimmung, ob der genannte Patient kurzfristig überleben oder langfristig überleben wird.

8. Verfahren gemäß Anspruch 7, bei dem das genannte Vorhersagemodell umfasst:

   i) Berechnen des Verhältnisses zwischen dem Expressionslevel der genannten Gene in der genannten Testprobe und dem Expressionslevel der genannten Gene in einer Kontrollprobe eines bekannten gesunden menschlichen Patienten,

ii) Vergleichen des genannten Verhältnisses mit Kürzungswerten für jedes Gen und Bestimmung der Dichotomisierungsfakoren für jedes Gen,

iii) Gewichten der genannten Dichotomisierungsfaktoren durch vorbestimmte Beta-Koeffizienten für jedes Gen und

iv) Berechnen eines Indexes I, der die Summe der genannten dichotomisierten Faktoren ist, die durch die genannten Beta-Koeffizienten der genannten Gene für den genannten Patienten gewichtet sind.

**9.** Verfahren gemäß Anspruch 8, bei dem die genannten Kürzungswerte wie folgt lauten:

| **Name des Gens** | **Kürzungen** |
|---|---|
| ELANE | 3,40 |
| G6PD | 1,15 |
| TKT | 1,2 |
| PGD | 1,22 |
| CEACAM4 | 1,34 |

und die Dichotomisierungsfaktoren wie folgt berechnet werden:

| **Name des Gens** | **Falt-Änderung** | **Dichotomisierungsfaktor:** D = |
|---|---|---|
| ELANE | b | +1 wenn b > 3,40; - 1 wenn b ≤ 3,40 |
| G6PD | c | +1 wenn c > 1,15; -1 wenn c ≤ 1,15 |
| TKT | d | +1 wenn d > 1,2; -1 wenn d ≤ 1,2 |
| PDG | e | +1 wenn e > 1,22; -1 wenn e ≤ 1,22 |
| CEACAM4 | f | +1 wenn f > 1,34; -1 wenn f ≤ 1,34 |

**10.** Verfahren gemäß Anspruch 8 oder 9, bei dem die genannten Beta-Koeffizienten wie folgt lauten:

| **Name des Gens** | **Beta-Koeffizient (β)** |
|---|---|
| ELANE | 2,01784191521554 |
| G6PD | 1,28224877578792 |
| TKT | 1,35358578043486 |
| PGD | 1,71153730912409 |
| CEACAM4 | 2,0942792881 |

## Revendications

**1.** Procédé pour déterminer *in vitro* le pronostic de la leucémie myélomonocytaire chronique chez un patient humain qui en est atteint, qui comprend les étapes suivantes consistant à :

a) mesurer le niveau d'expression des cinq gènes *G6PD* (SEQ ID NO: 2 ou 3), *6PGD* (SEQ ID NO: 4), *TKT* (SEQ ID NO: 9 ou 10), *CEACAM4* (SEQ ID NO: 11) et *ELANE* (SEQ ID NO: 23) dans un échantillon test qui est un échantillon de cellules mononucléées du sang périphérique (PBMC) dudit patient humain,

b) comparer lesdits niveaux d'expression au niveau d'expression desdits au moins cinq gènes dans l'échantillon témoin d'un sujet humain connu en bonne santé,

c) prédire l'évolution de la leucémie myélomonocytaire chronique chez ledit patient.

**2.** Procédé selon la revendication 1, dans lequel le taux d'expression le plus haut au moins des gènes *G6PD* (SEQ ID NO: 2 ou 3), *6PGD* (SEQ ID NO: 4), *TKT* (SEQ ID NO: 9 ou 10), *CEACAM4* (SEQ ID NO:11) et *ELANE* (SEQ ID NO: 23) dans ledit échantillon test, par rapport audit échantillon témoin, indique la survie à long terme dudit patient humain.

3. Procédé selon la revendication 1, dans lequel le taux d'expression le plus bas au moins des gènes *G6PD* (SEQ ID NO: 2 ou 3), *6PGD* (SEQ ID NO: 4), *TKT* (SEQ ID NO: 9 ou 10), *CEACAM4* (SEQ ID NO: 11) et *ELANE* (SEQ ID NO: 23), indique la survie à court terme dudit patient humain.

4. Procédé selon l'une quelconque des revendications 1 à 3, dans lequel l'étape a) comprend la mesure des niveaux des produits de transcription de l'ARN ou de l'ADNc desdits gènes en employant des méthodes de détection basées sur l'acide nucléique telles que les micro-réseaux, la PCR quantitative, les puces à ADN, l'hybridation à l'aide de sondes marquées ou le dosage immuno-chromatographique à flux latéral sur bâtonnet.

5. Procédé selon l'une quelconque des revendications 1 à 3, dans lequel l'étape a) comprend la mesure des niveaux des protéines respectives desdits gènes en employant des méthodes de détection basées sur les anticorps telles que l'immunohistochimie ou l'analyse par Western Blot.

6. Kit pour déterminer le pronostic de la leucémie myélomonocytaire chronique chez un patient humain qui en est atteint, comprenant des amorces et/ou des sondes d'amplification spécifiques pour l'amplification quantitative spécifique de produits de transcription afin de détecter spécifiquement le niveau d'expression au moins des cinq gènes *G6PD* (SEQ ID NO: 2 ou 3), *6PGD* (SEQ ID NO: 4), *TKT* (SEQ ID NO: 9 ou 10), *CEACAM4* (SEQ ID NO: 11) et *ELANE* (SEQ ID NO: 23).

7. Procédé pour déterminer si les patients atteints de leucémie myélomonocytaire chronique auront une survie à court terme ou une survie à long terme, comprenant les étapes consistant à :

a) prélever un échantillon test sur ledit patient humain,
b) déterminer le niveau d'expression des cinq gènes *G6PD* (SEQ ID NO: 2 ou 3), *6PGD* (SEQ ID NO: 4), *TKT* (SEQ ID NO: 9 ou 10), *CEACAM4* (SEQ ID NO: 11) et *ELANE* (SEQ ID NO: 23) dans ledit échantillon test qui est un échantillon de cellules mononucléées du sang périphérique (PBMC) dudit patient,
c) appliquer un modèle prédictif pour déterminer si ledit patient aura une survie à court terme ou une survie à long terme.

8. Procédé selon la revendication 7, dans lequel ledit modèle prédictif comprend :

i) le calcul du rapport entre le niveau d'expression desdits gènes dans ledit échantillon test et le niveau d'expression des mêmes gènes dans l'échantillon témoin d'un sujet humain connu en bonne santé,
ii) la comparaison dudit rapport avec des valeurs seuils pour chaque gène et la détermination des facteurs de dichotomisation pour chaque gène,
iii) la pondération desdits facteurs de dichotomisation par un coefficient bêta prédéterminé pour chaque gène, et
iv) le calcul d'un indice I qui est la somme desdits facteurs dichotomisés pondérés par lesdits coefficients bêta desdits gènes pour ledit patient.

9. Procédé selon la revendication 8, dans lequel lesdites valeurs seuils sont les suivantes :

| Nom du gène | Valeurs seuils |
|---|---|
| *ELANE* | 3,40 |
| *G6PD* | 1,15 |
| *TKT* | 1,2 |
| *PGD* | 1,22 |
| *CEACAM4* | 1,34 |

et les facteurs de dichotomisation sont calculés de la manière suivante :

| Nom du gène | Rapport du niveau moyen d'expression (fold change) | Facteur de dichotomisation : D = |
|---|---|---|
| *ELANE* | b | +1 si b > 3,40 ; -1 si b ≤ 3,40 |
| *G6PD* | c | +1 si c > 1,15 ; -1 si c ≤ 1,15 |
| *TKT* | d | +1 si d > 1, 2 ; -1 si d ≤ 1,2 |
| *PGD* | e | +1 si e > 1,22 ; -1 si e ≤ 1,22 |

(suite)

| Nom du gène | Rapport du niveau moyen d'expression (fold change) | Facteur de dichotomisation : D = |
|---|---|---|
| *CEACAM4* | f | +1 si f > 1,34 ; -1 si f ≤ 1,34 |

**10.** Procédé selon les revendications 8 ou 9, dans lequel lesdits coefficients bêta sont les suivants :

| Nom du gène | Coefficient bêta (β) |
|---|---|
| *ELANE* | 2,01784191521554 |
| *G6PD* | 1,28224877578792 |
| *TKT* | 1,35358578043486 |
| *PGD* | 1,71153730912409 |
| *CEACAM4* | 2,0942792881 |

Figure 1

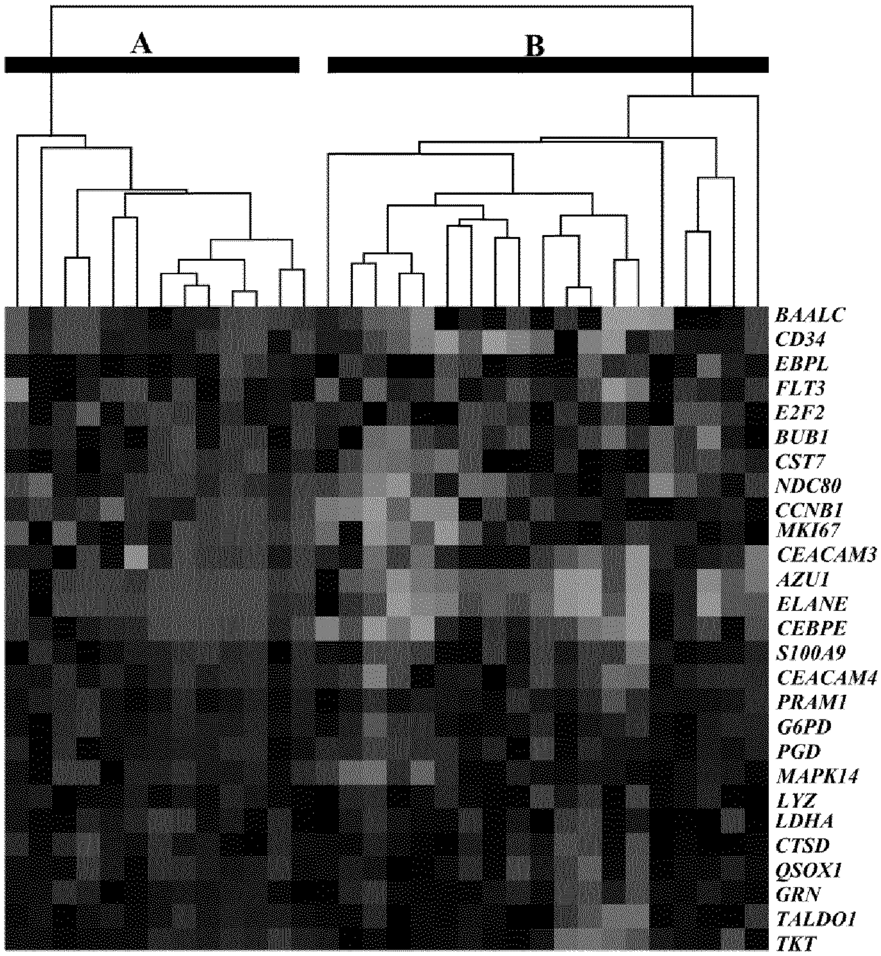
A
B
BAALC
CD34
EBPL
FLT3
E2F2
BUB1
CST7
NDC80
CCNB1
MKI67
CEACAM3
AZU1
ELANE
CEBPE
S100A9
CEACAM4
PRAM1
G6PD
PGD
MAPK14
LYZ
LDHA
CTSD
QSOX1
GRN
TALDO1
TKT

Figure 2

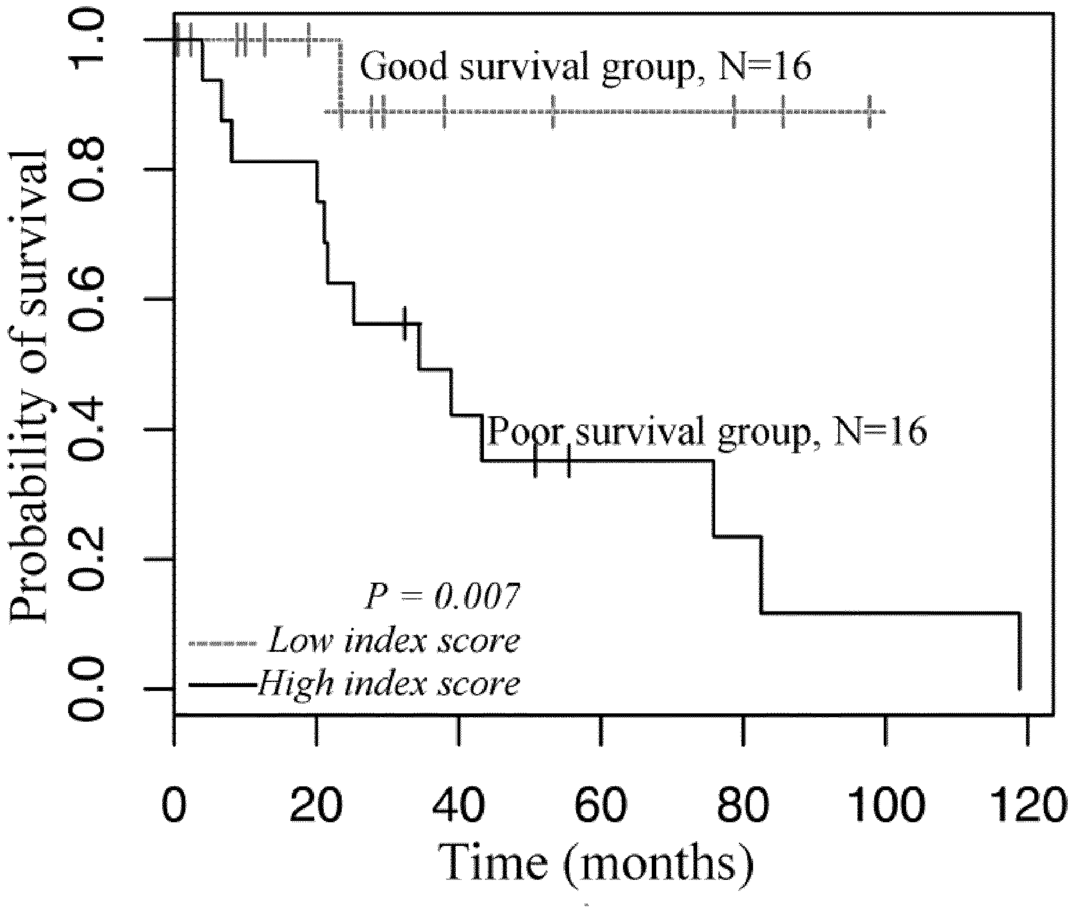
Probability of survival
1.0
0.8
0.6
0.4
0.2
0.0
Good survival group, N=16
Poor survival group, N=16
P = 0.007
Low index score
High index score
0
20
40
60
80
100
120
Time (months)

Figure 3

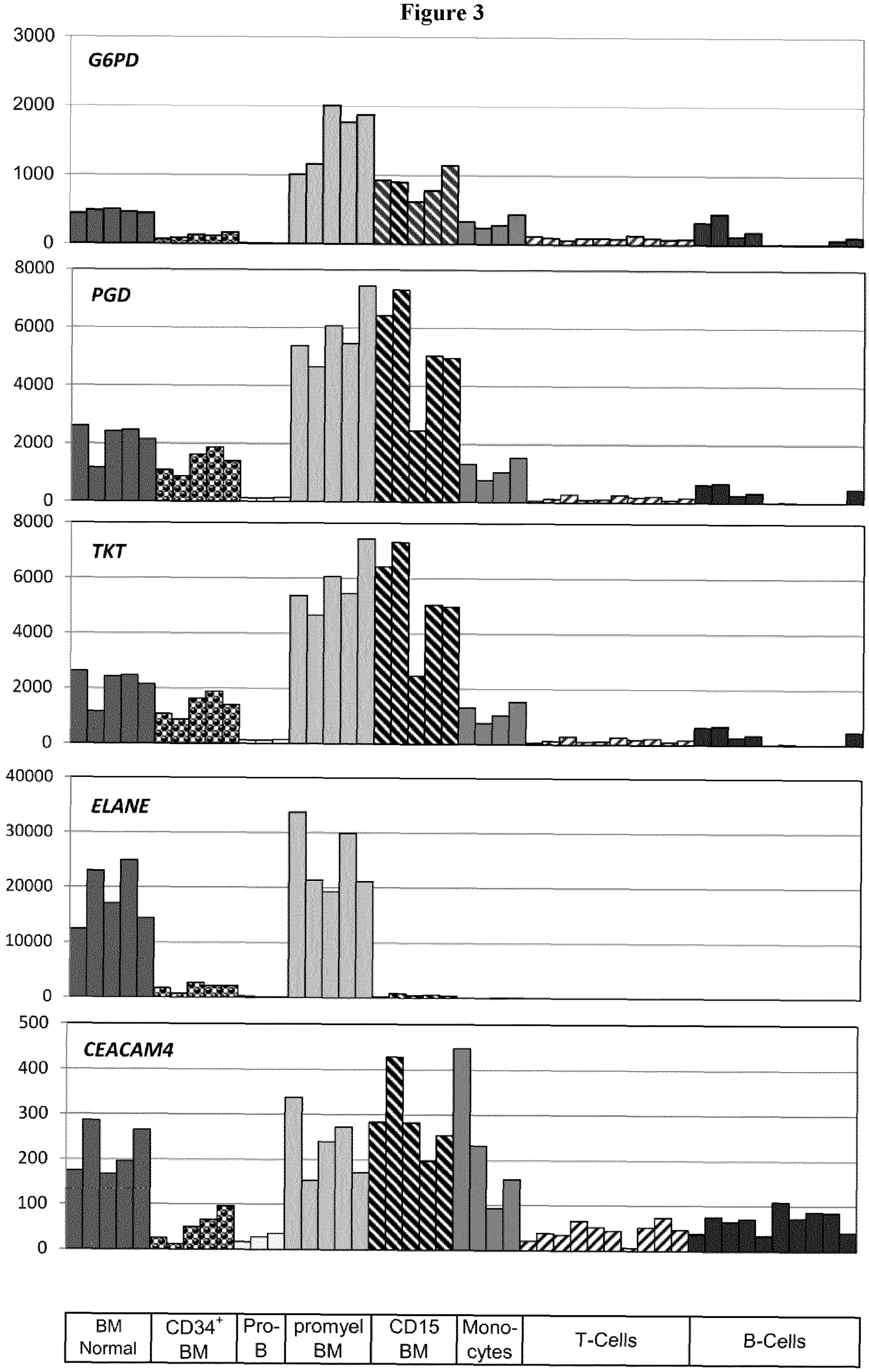
3000
2000
1000
0
G6PD
8000
6000
4000
2000
0
PGD
8000
6000
4000
2000
0
TKT
40000
30000
20000
10000
0
ELANE
500
400
300
200
100
0
CEACAM4
BM Normal
CD34+ BM
Pro-B
promyel BM
CD15 BM
Mono-cytes
T-Cells
B-Cells

**Figure 4**

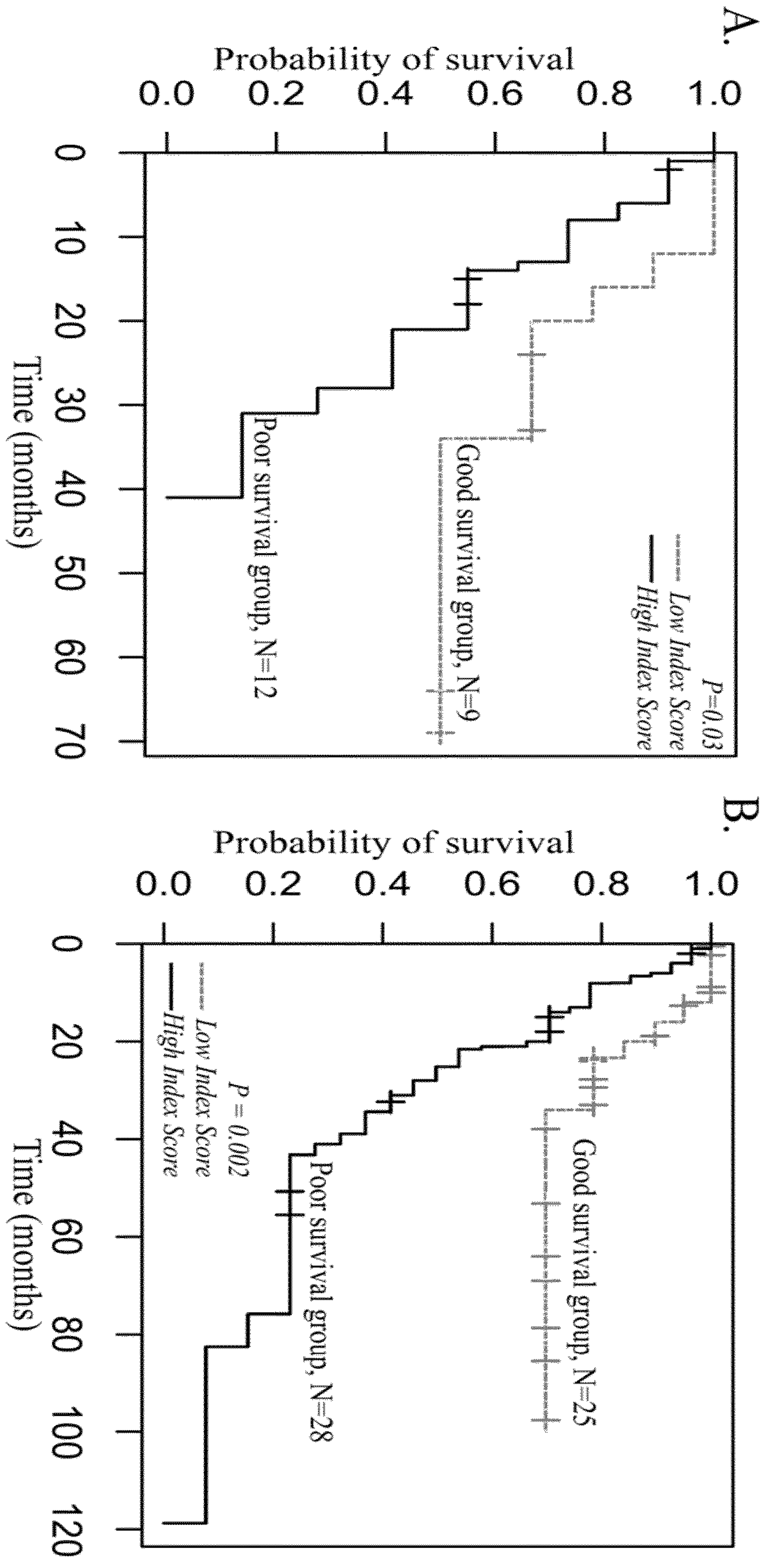
A.
Probability of survival
0.0
0.2
0.4
0.6
0.8
1.0
Time (months)
0
10
20
30
40
50
60
70
P=0.03
Low Index Score
High Index Score
Good survival group, N=9
Poor survival group, N=12
B.
Probability of survival
0.0
0.2
0.4
0.6
0.8
1.0
Time (months)
0
20
40
60
80
100
120
P = 0.002
Low Index Score
High Index Score
Good survival group, N=25
Poor survival group, N=28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 12305187 A


### Non-patent literature cited in the description


- **RICCI, C. et al.** *Clinical Cancer Research,* 2010
- **KOHLMANN, A. et al.** *Journal of Clinical Oncology,* 2010
- **TEFFERI A et al.** *Leukemia,* 2009
- **KOSMIDER O et al.** *Haematologica,* 2009
- **SURASILP T et al.** *Mol Cell Probes.,* 2011
- **BABA M et al.** *Int. J. Cancer,* 1989, vol. 43 (5), 892-5
- **BENJAMINI Y.** *Journal of the royal statistical society,* 1995, vol. 57, 289-300
- **BENNETT, J.M.** *British Journal of Haematology,* 1994, vol. 87, 746-754
- **BERTRAND G et al.** *J Immunol Methods.,* 2004, vol. 292 (1-2), 43-58
- **BONAFOUX B ; COMMES T.** *Methods Mol Biol.,* 2009, vol. 496, 299-311
- **BRESOLIN, S.** *Journal of Clinical Oncology,* 2010, vol. 28, 1919-1927
- **CAMARGO A et al.** *Source Code Biol Med,* 2008, vol. 3, 15
- **CARLIN ; CHIB.** *Journal of the Royal Statistical Society,* 1995, vol. 57, 473-184
- **CUI X ; CHURCHILL GA.** *Genome Biol.,* 2003, vol. 4 (4), 210
- **DROIN, N.** *Blood,* 2010, vol. 115, 78-88
- **ECKEL-PASSOW JE et al.** *Cancer Res.,* 15 April 2005, vol. 65 (8), 2985-9
- **EISEN MB et al.** *Proc Natl Acad Sci USA,* 1998, vol. 95, 14863-14868
- **FURUTA E et al.** *Biochimica et Biophysica Acta,* 2010, vol. 1805 (2), 141-52
- **GROSSMANN, V.** *Leukemia,* 2011, vol. 25, 877-879
- **HAAB BB.** *Mol Cell Proteomics,* April 2005, vol. 4 (4), 377-83
- **HAFERLACH, T.** *Journal of Clinical Oncology,* 2010, vol. 28, 2529-2537
- **HALL DA et al.** *Mech Ageing Dev.,* January 2007, vol. 128 (1), 161-7
- **JAFFE, E.S.** *Lyon: IARC Press,* 2001
- **JANSSEN WE et al.** *Cytotherapy,* 2010, vol. 12 (3), 418-24
- **KASSAMBARA, A.** *Haematologica,* 2011
- **KINGSMORE SF.** *Nat Rev Drug Discov.,* April 2006, vol. 5 (4), 310-20
- **KOHLMANN, A. et al.** *Jornal of Clinical Oncology,* 2010, vol. 28 (24), 3858-65
- **KOSMIDER O et al.** *Haematologica,* 2009, vol. 94 (12), 1676-81
- **LE CARROUR, T.** *The Open Bioinformatics Journal,* 2010, vol. 4, 5-10
- **LEE HS et al.** *Life Sciences,* 2007, vol. 80 (7), 690-8
- **LEE, HJ et al.** *Gastroenterology,* 2010, vol. 139 (1), 213-25
- **LIVAK KJ ; SCHMITTGEN TD.** *Methods,* 2001, vol. 25 (4), 402-8
- **MILLS, K.I.** *Blood,* 2009, vol. 114, 1063-1072
- **MOREAUX, J.** *Haematologica,* 2011, vol. 96, 574-582
- **ORAZI, A. ; GERMING, U.** *Leukemia,* 2008, vol. 22 (7), 1308-19
- **PIQUEMAL et al.** *Genomics,* 2002, vol. 80 (3), 361-71
- **QUERE R et al.** *Blood,* 2007, vol. 109 (10), 4450-60
- **REITER, A.** *Haematologica,* 2009, vol. 94, 1634-1638
- **RHODES, D.R.** *Neoplasia,* 2004, vol. 6, 1-6
- **RICCI, C. et al.** *Clinical Cancer Research,* 2010, vol. 16 (8), 2246-56
- **RIVALS, E.** *Nucleic Acids Research,* 2007, vol. 35, e108-e108
- **SCHMITTER, T.** *Infection and Immunity,* 2007, vol. 75, 4116-4126
- **SUCH, E. et al.** *Haematologica,* 2011, vol. 96, 375-383
- **SURASILP T et al.** *Mol Cell Probes,* 2011
- **TEFFERI A et al.** *Leukemia,* 2009, vol. 23 (5), 900-4
- **THEILGAARD-MONCH, K.** *Leukemia,* 2011, vol. 25, 909-920
- **TOYOKUNI S et al.** *FEBS Letters,* 1995, vol. 358 (1), 1-3
- **VARDIMAN, J.W.** *Blood,* 2002, vol. 100, 2292-2302

- **WOUTERS, B.J.** *Blood,* 2009, vol. 113, 291-298

SEQUENCE LISTING

<110> CENTRE HOSPITALIER UNIVERSITAIRE NIMES
SKULDTECH

<120> NEW METHOD FOR PROGNOSING THE SURVIVAL OF PATIENTS SUFFERING FROM CHRONIC MYELOMONOCYTIC LEUKEMIA

<130> 362440D29460

<150> EP 12305187.2

<151> 2012-02-17

<160> 24

<170> PatentIn version 3.5

<210> 1
<211> 1516
<212> DNA
<213> homo sapiens

<220>
<221> misc_feature
<223> mRNA LYZ human

<400> 1

```
aaatactggg gccagctcac cctggtcagc ctagcactct gacctagcag tcaacatgaa      60
ggctctcatt gttctggggc ttgtcctcct ttctgttacg gtccagggca aggtctttga     120
aaggtgtgag ttggccagaa ctctgaaaag attgggaatg gatggctaca ggggaatcag     180
cctagcaaac tggatgtgtt tggccaaatg ggagagtggt tacaacacac gagctacaaa     240
ctacaatgct ggagacagaa gcactgatta tgggatattt cagatcaata gccgctactg     300
gtgtaatgat ggcaaaaccc caggagcagt taatgcctgt catttatcct gcagtgcttt     360
gctgcaagat aacatcgctg atgctgtagc ttgtgcaaag agggttgtcc gtgatccaca     420
aggcattaga gcatgggtgg catggagaaa tcgttgtcaa aacagagatg tccgtcagta     480
tgttcaaggt tgtggagtgt aactccagaa ttttccttct tcagctcatt ttgtctctct     540
cacattaagg gagtaggaat taagtgaaag gtcacactac cattatttcc ccttcaaaca     600
aataatattt ttacagaagc aggagcaaaa tatggccttt cttctaagag atataatgtt     660
cactaatgtg gttattttac attaagccta caacattttt cagtttgcaa atagaactaa     720
tactggtgaa aatttaccta aaaccttggt tatcaaatac atctccagta cattccgttc     780
tttttttttt tgagacagtc tcgctctgtc gcccaggctg gagtgcagtg gcgcaatctc     840
ggctcactgc aacctccacc tcccgggttc acgccattct cctgcctcag cctcccgagt     900
agctgggatt acgggcgccc gccaccacgc ccggctaatt ttttgtattt ttagtagaga     960
cagggtttca ccgtgttagc caggatggtc tcgatctcct gaccttgtga tccacccacc    1020
tcggcctccc aaagtgctgg gattacaggc gtgagccact gcgcccggcc acattcagtt    1080
```

```
cttatcaaag aaataaccca gacttaatct tgaatgatac gattatgccc aatattaagt      1140
aaaaaatata agaaaaggtt atcttaaata gatcttaggc aaaataccag ctgatgaagg      1200
catctgatgc cttcatctgt tcagtcatct ccaaaaacag taaaaataac cactttttgt      1260
tgggcaatat gaaattttta aaggagtaga ataccaaatg atagaaacag actgcctgaa      1320
ttgagaattt tgatttctta aagtgtgttt ctttctaaat tgctgttcct taatttgatt      1380
aatttaattc atgtattatg attaaatctg aggcagatga gcttacaagt attgaaataa      1440
ttactaatta atcacaaatg tgaagttatg catgatgtaa aaaatacaaa cattctaatt      1500
aaaggctttg caacac                                                      1516
```

<210> 2
<211> 2395
<212> DNA
<213> homo sapiens

<220>
<221> misc_feature
<223> G6PD mRNA human isoform A

<400> 2

```
agaggcaggg gctggcctgg gatgcgcgcg cacctgccct cgccccgccc cgcccgcacg      60
aggggtggtg gccgaggccc cgccccgcac gcctcgcctg aggcgggtcc gctcagccca     120
ggcgcccgcc cccgcccccg ccgattaaat gggccggcgg ggctcagccc ccggaaacgg     180
tcgtacactt cggggctgcg agcgcggagg gcgacgacga cgaagcgcag acagcgtcat     240
ggcagagcag gtggccctga gccggaccca ggtgtgcggg atcctgcggg aagagctttt     300
ccagggcgat gccttccatc agtcggatac acacatattc atcatcatgg gtgcatcggg     360
tgacctggcc aagaagaaga tctaccccac catctggtgg ctgttccggg atggccttct     420
gcccgaaaac accttcatcg tgggctatgc ccgttcccgc ctcacagtgg ctgacatccg     480
caaacagagt gagcccttct tcaaggccac cccagaggag aagctcaagc tggaggactt     540
ctttgcccgc aactcctatg tggctggcca gtacgatgat gcagcctcct accagcgcct     600
caacagccac atgaatgccc tccacctggg gtcacaggcc aaccgcctct tctacctggc     660
cttgcccccg accgtctacg aggccgtcac caagaacatt cacgagtcct gcatgagcca     720
gataggctgg aaccgcatca tcgtggagaa gcccttcggg agggacctgc agagctctga     780
ccggctgtcc aaccacatct cctccctgtt ccgtgaggac cagatctacc gcatcgacca     840
ctacctgggc aaggagatgg tgcagaacct catggtgctg agatttgcca acaggatctt     900
cggccccatc tggaaccggg acaacatcgc ctgcgttatc ctcaccttca aggagccctt     960
tggcactgag ggtcgcgggg gctatttcga tgaatttggg atcatccggg acgtgatgca    1020
```

gaaccaccta ctgcagatgc tgtgtctggt ggccatggag aagcccgcct ccaccaactc 1080

agatgacgtc cgtgatgaga aggtcaaggt gttgaaatgc atctcagagg tgcaggccaa 1140

caatgtggtc ctgggccagt acgtggggaa ccccgatgga gagggcgagg ccaccaaagg 1200

gtacctggac gaccccacgg tgccccgcgg gtccaccacc gccacttttg cagccgtcgt 1260

cctctatgtg gagaatgaga ggtgggatgg ggtgcccttc atcctgcgct gcggcaaggc 1320

cctgaacgag cgcaaggccg aggtgaggct gcagttccat gatgtggccg gcgacatctt 1380

ccaccagcag tgcaagcgca acgagctggt gatccgcgtg cagcccaacg aggccgtgta 1440

caccaagatg atgaccaaga agccgggcat gttcttcaac cccgaggagt cggagctgga 1500

cctgacctac ggcaacagat acaagaacgt gaagctccct gacgcctacg agcgcctcat 1560

cctggacgtc ttctgcggga gccagatgca cttcgtgcgc agcgacgagc tccgtgaggc 1620

ctggcgtatt ttcaccccac tgctgcacca gattgagctg gagaagccca agcccatccc 1680

ctatatttat ggcagccgag gccccacgga ggcagacgag ctgatgaaga gagtgggttt 1740

ccagtatgag ggcacctaca agtgggtgaa cccccacaag ctctgagccc tgggcaccca 1800

cctccacccc cgccacggcc accctccttc ccgccgcccg accccgagtc gggaggactc 1860

cgggaccatt gacctcagct gcacattcct ggccccgggc tctggccacc ctggcccgcc 1920

cctcgctgct gctactaccc gagcccagct acattcctca gctgccaagc actcgagacc 1980

atcctggccc ctccagaccc tgcctgagcc caggagctga gtcacctcct ccactcactc 2040

cagcccaaca gaaggaagga ggagggcgcc cattcgtctg tcccagagct tattggccac 2100

tgggtctcac tcctgagtgg ggccagggtg ggagggaggg acaaggggga ggaaaggggc 2160

gagcacccac gtgagagaat ctgcctgtgg ccttgcccgc cagcctcagt gccacttgac 2220

attccttgtc accagcaaca tctcgagccc cctggatgtc ccctgtccca ccaactctgc 2280

actccatggc caccccgtgc cacccgtagg cagcctctct gctataagaa aagcagacgc 2340

agcagctggg accccteccca acctcaatgc cctgccatta aatccgcaaa cagcc 2395

<210> 3
<211> 2270
<212> DNA
<213> homo sapiens

<220>
<221> misc_feature
<223> G6PD mRNA human isoform B

<400> 3

cgagaactcg ggaagccggc gagaagtgtg aggccgcggt agggccgcat cccgctccgg 60

agagaagtct gagtccgcca ggctctgcag gcccgcggaa gctcgacagc gtcatggcag 120

agcaggtggc cctgagccgg acccaggtgt gcgggatcct gcgggaagag cttttccagg 180

```
gcgatgcctt ccatcagtcg gatacacaca tattcatcat catgggtgca tcgggtgacc      240
tggccaagaa gaagatctac cccaccatct ggtggctgtt ccgggatggc cttctgcccg      300
aaaacacctt catcgtgggc tatgcccgtt cccgcctcac agtggctgac atccgcaaac      360
agagtgagcc cttcttcaag gccaccccag aggagaagct caagctggag gacttctttg      420
cccgcaactc ctatgtggct ggccagtacg atgatgcagc ctcctaccag cgcctcaaca      480
gccacatgaa tgccctccac ctggggtcac aggccaaccg cctcttctac ctggccttgc      540
ccccgaccgt ctacgaggcc gtcaccaaga acattcacga gtcctgcatg agccagatag      600
gctggaaccg catcatcgtg gagaagccct tcgggaggga cctgcagagc tctgaccggc      660
tgtccaacca catctcctcc ctgttccgtg aggaccagat ctaccgcatc gaccactacc      720
tgggcaagga gatggtgcag aacctcatgg tgctgagatt tgccaacagg atcttcggcc      780
ccatctggaa ccgggacaac atcgcctgcg ttatcctcac cttcaaggag ccctttggca      840
ctgagggtcg cggggctat ttcgatgaat ttgggatcat ccgggacgtg atgcagaacc       900
acctactgca gatgctgtgt ctggtggcca tggagaagcc cgcctccacc aactcagatg      960
acgtccgtga tgagaaggtc aaggtgttga aatgcatctc agaggtgcag gccaacaatg     1020
tggtcctggg ccagtacgtg gggaaccccg atggagaggg cgaggccacc aaagggtacc     1080
tggacgaccc cacggtgccc cgcgggtcca ccaccgccac ttttgcagcc gtcgtcctct     1140
atgtggagaa tgagaggtgg gatggggtgc ccttcatcct gcgctgcggc aaggccctga     1200
acgagcgcaa ggccgaggtg aggctgcagt tccatgatgt ggccggcgac atcttccacc     1260
agcagtgcaa gcgcaacgag ctggtgatcc gcgtgcagcc caacgaggcc gtgtacacca     1320
agatgatgac caagaagccg ggcatgttct tcaaccccga ggagtcggag ctggacctga     1380
cctacggcaa cagatacaag aacgtgaagc tccctgacgc ctacgagcgc ctcatcctgg     1440
acgtcttctg cgggagccag atgcacttcg tgcgcagcga cgagctccgt gaggcctggc     1500
gtattttcac ccccactgctg caccagattg agctggagaa gcccaagccc atcccctata    1560
tttatggcag ccgaggcccc acggaggcag acgagctgat gaagagagtg ggtttccagt     1620
atgagggcac ctacaagtgg gtgaaccccc acaagctctg agccctgggc acccacctcc     1680
acccccgcca cggccaccct ccttcccgcc gcccgacccc gagtcgggag gactccggga     1740
ccattgacct cagctgcaca ttcctggccc cgggctctgg ccaccctggc ccgcccctcg     1800
ctgctgctac tacccgagcc cagctacatt cctcagctgc caagcactcg agaccatcct     1860
ggcccctcca gaccctgcct gagcccagga gctgagtcac ctcctccact cactccagcc     1920
caacagaagg aaggaggagg gcgcccattc gtctgtccca gagcttattg gccactgggt     1980
ctcactcctg agtggggcca gggtgggagg gagggacaag gggaggaaa ggggcgagca      2040
```

cccacgtgag agaatctgcc tgtggccttg cccgccagcc tcagtgccac ttgacattcc 2100

ttgtcaccag caacatctcg agccccctgg atgtcccctg tcccaccaac tctgcactcc 2160

atggccaccc cgtgccaccc gtaggcagcc tctctgctat aagaaaagca gacgcagcag 2220

ctgggacccc tcccaacctc aatgccctgc cattaaatcc gcaaacagcc 2270

<210> 4
<211> 1937
<212> DNA
<213> homo sapiens

<220>
<221> misc_feature
<223> mRNA 6PGD

<400> 4

```
ggccgcagtt tctggaggga gccgctgcgg gtctttccct cactcgtcct ccgcgcgtcg      60
ccgctcttcg gttctgctct gtccgccgcc atggcccaag ctgacatcgc gctgatcgga     120
ttggccgtca tgggccagaa cttaattctg aacatgaatg accacggctt tgtggtctgt     180
gcttttaata ggactgtctc caaagttgat gatttcttgg ccaatgaggc aaagggaacc     240
aaagtggtgg gtgcccagtc cctgaaagag atggtctcca agctgaagaa gccccggcgg     300
atcatcctcc tggtgaaggc tgggcaagct gtggatgatt tcatcgagaa attggtacca     360
ttgttggata ctggtgacat catcattgac ggaggaaatt ctgaatatag ggacaccaca     420
agacggtgcc gagacctcaa ggccaaggga attttatttg tggggagcgg agtcagtggt     480
ggagaggaag gggcccggta tggcccatcg ctcatgccag gagggaacaa agaagcgtgg     540
ccccacatca agaccatctt ccaaggcatt gctgcaaaag tgggaactgg agaaccctgc     600
tgtgactggg tgggagatga gggagcaggc cacttcgtga agatggtgca caacgggata     660
gagtatgggg acatgcagct gatctgtgag gcataccacc tgatgaaaga cgtgctgggc     720
atggcgcagg acgagatggc ccaggccttt gaggattgga ataagacaga gctagactca     780
ttcctgattg aaatcacagc caatattctc aagttccaag acaccgatgg caaacacctg     840
ctgccaaaga tcagggacag cgcggggcag aagggcacag ggaagtggac cgccatctcc     900
gccctggaat acggcgtacc cgtcaccctc attggagaag ctgtctttgc tcggtgctta     960
tcatctctga aggatgagag aattcaagct agcaaaaagc tgaagggtcc ccagaagttc    1020
cagtttgatg gtgataagaa atcattcctg gaggacattc ggaaggcact ctacgcttcc    1080
aagatcatct cttacgctca aggctttatg ctgctaaggc aggcagccac cgagtttggc    1140
tggactctca attatggtgg catcgccctg atgtggagag gggctgcat cattagaagt     1200
gtattcctag gaaagataaa ggatgcattt gatcgaaacc cggaacttca gaacctccta    1260
ctggacgact tctttaagtc agctgttgaa aactgccagg actcctggcg gcgggcagtc    1320
```

agcactgggg tccaggctgg cattcccatg ccctgtttta ccactgccct ctccttctat 1380

gacgggtaca gacatgagat gcttccagcc agcctcatcc aggctcagcg ggattacttc 1440

ggggctcaca cctatgaact cttggccaaa ccagggcagt ttatccacac caactggaca 1500

ggccatggtg gcaccgtgtc atcctcgtca tacaatgcct gatcatgctg ctcctgtcac 1560

cctccacgat tccacagacc aggacattcc atgtgcctca tggcactgcc acctggccct 1620

ttgccctatt ttctgttcag ttttttaaaa gtgttgtaag agactcctga ggaagacaca 1680

cagtttattt gtaaagtagc tctgtgagag ccaccatgcc ctctgccctt gcctcttggg 1740

actgaccagg agctgctcat gtgcgtgaga gtgggaacca tctccttgcg gcagtggctt 1800

ccgcgtgccc cgtgtgctgg tgcggttccc atcacgcaga caggaagggt gtttgcgcac 1860

tctgatcaac tggaacctct gtatcatgcg gctgaattcc ctttttcctt tactcaataa 1920

aagctacatc acactga 1937

<210> 5
<211> 2971
<212> DNA
<213> homo sapiens

<220>
<221> misc_feature
<223> mRNA LILRB1 human, isoform 1

<400> 5

gaggaggaac agaaaagaaa agaaaagaaa aagtgggaaa caaataatct aagaatgagg 60

agaaagcaag aagagtgacc cccttgtggg cactccattg gttttatggc gcctctactt 120

tctggagttt gtgtaaaaca aaaatattat ggtctttgtg cacatttaca tcaagctcag 180

cctgggcggc acagccagat gcgagatgcg tctctgctga tctgagtctg cctgcagcat 240

ggacctgggt cttccctgaa gcatctccag ggctggaggg acgactgcca tgcaccgagg 300

gctcatccat ccacagagca gggcagtggg aggagacgcc atgaccccca tcctcacggt 360

cctgatctgt ctcgggctga gtctgggccc caggacccac gtgcaggcag ggcacctccc 420

caagcccacc ctctgggctg aaccaggctc tgtgatcacc caggggagtc ctgtgaccct 480

caggtgtcag gggggccagg agacccagga gtaccgtcta tatagagaaa agaaaacagc 540

actctggatt acacggatcc cacaggagct tgtgaagaag ggccagttcc ccatcccatc 600

catcacctgg gaacatgcag ggcggtatcg ctgttactat ggtagcgaca ctgcaggccg 660

ctcagagagc agtgaccccc tggagctggt ggtgacagga gcctacatca aacccaccct 720

ctcagcccag cccagccccg tggtgaactc aggagggaat gtaatcctcc agtgtgactc 780

acaggtggca tttgatggct tcagtctgtg taaggaagga gaagatgaac acccacaatg 840

cctgaactcc cagccccatg cccgtgggtc gtcccgcgcc atcttctccg tgggccccgt 900
gagcccgagt cgcaggtggt ggtacaggtg ctatgcttat gactcgaact ctccctatga 960
gtggtctcta cccagtgatc tcctggagct cctggtccta ggtgtttcta agaagccatc 1020
actctcagtg cagccaggtc ctatcgtggc ccctgaggag accctgactc tgcagtgtgg 1080
ctctgatgct ggctacaaca gatttgttct gtataaggac ggggaacgtg acttccttca 1140
gctcgctggc gcacagcccc aggctgggct ctcccaggcc aacttcaccc tgggccctgt 1200
gagccgctcc tacgggggcc agtacagatg ctacggtgca cacaacctct cctccgagtg 1260
gtcggccccc agcgaccccc tggacatcct gatcgcagga cagttctatg acagagtctc 1320
cctctcggtg cagccgggcc ccacggtggc ctcaggagag aacgtgaccc tgctgtgtca 1380
gtcacaggga tggatgcaaa ctttccttct gaccaaggag gggcagctg atgacccatg 1440
gcgtctaaga tcaacgtacc aatctcaaaa ataccaggct gaattcccca tgggtcctgt 1500
gacctcagcc catgcgggga cctacaggtg ctacggctca cagagctcca aaccctacct 1560
gctgactcac cccagtgacc ccctggagct cgtggtctca ggaccgtctg gggccccag 1620
ctccccgaca acaggcccca cctccacatc tggccctgag gaccagcccc tcacccccac 1680
cgggtcggat ccccagagtg gtctgggaag gcacctgggg gttgtgatcg gcatcttggt 1740
ggccgtcatc ctactgctcc tcctcctcct cctcctcttc ctcatcctcc gacatcgacg 1800
tcagggcaaa cactggacat cgacccagag aaaggctgat ttccaacatc ctgcaggggc 1860
tgtggggcca gagcccacag acagaggcct gcagtggagg tccagcccag ctgccgatgc 1920
ccaggaagaa aacctctatg ctgccgtgaa gcacacacag cctgaggatg gggtggagat 1980
ggacactcgg agcccacacg atgaagaccc ccaggcagtg acgtatgccg aggtgaaaca 2040
ctccagacct aggagagaaa tggcctctcc tccttcccca ctgtctgggg aattcctgga 2100
cacaaaggac agacaggcgg aagaggacag gcagatggac actgaggctg ctgcatctga 2160
agccccccag gatgtgacct acgcccagct gcacagcttg accctcagac gggaggcaac 2220
tgagcctcct ccatcccagg aagggccctc tccagctgtg cccagcatct acgccactct 2280
ggccatccac tagcccaggg ggggacgcag accccacact ccatggagtc tggaatgcat 2340
gggagctgcc cccccagtgg acaccattgg accccaccca gcctggatct accccaggag 2400
actctgggaa cttttagggg tcactcaatt ctgcagtata aataactaat gtctctacaa 2460
ttttgaaata aagcaacaga cttctcaata atcaatgaag tagctgagaa aactaagtca 2520
gaaagtgcat taaactgaat cacaatgtaa atattacaca tcaagcgatg aaactggaaa 2580
actacaagcc acgaatgaat gaattaggaa agaaaaaaag taggaaatga atgatcttgg 2640
ctttcctata agaaatttag ggcagggcac ggtggctcac gcctgtaatt ccagcacttt 2700
gggaggccga ggcgggcaga tcacgagttc aggagatcga gaccatcttg gccaacatgg 2760

tgaaaccctg tctctcctaa aaatacaaaa attagctgga tgtggtggca gtgcctgtaa 2820

tcccagctat ttgggaggct gaggcaggag aatcgcttga accagggagt cagaggtttc 2880

agtgagccaa gatcgcacca ctgctctcca gcctggcgac agagggagac tccatctcaa 2940

attaaaaaaa aaaaaaaaaa agaaagaaaa a 2971

<210> 6
<211> 2802
<212> DNA
<213> homo sapiens

<220>
<221> misc_feature
<223> mRNA LILRB1 human, isoform 2

<400> 6

ctcagcctgg gcggcacagc cagatgcgag atgcgtctct gctgatctga gtctgcctgc 60

agcatggacc tgggtcttcc ctgaagcatc tccagggctg gagggacgac tgccatgcac 120

cgagggctca tccatccaca gagcagggca gtgggaggag acgccatgac ccccatcctc 180

acggtcctga tctgtctcgg gctgagtctg ggccccagga cccacgtgca ggcagggcac 240

ctccccaagc ccaccctctg ggctgaacca ggctctgtga tcacccaggg gagtcctgtg 300

accctcaggt gtcagggggg ccaggagacc caggagtacc gtctatatag agaaaagaaa 360

acagcactct ggattacacg gatcccacag gagcttgtga agaagggcca gttccccatc 420

ccatccatca cctgggaaca tgcagggcgg tatcgctgtt actatggtag cgacactgca 480

ggccgctcag agagcagtga ccccctggag ctggtggtga caggagccta catcaaaccc 540

accctctcag cccagcccag ccccgtggtg aactcaggag ggaatgtaat cctccagtgt 600

gactcacagg tggcatttga tggcttcagt ctgtgtaagg aaggagaaga tgaacaccca 660

caatgcctga actcccagcc ccatgcccgt gggtcgtccc gcgccatctt ctccgtgggc 720

cccgtgagcc cgagtcgcag gtggtggtac aggtgctatg cttatgactc gaactctccc 780

tatgagtggt ctctacccag tgatctcctg gagctcctgg tcctaggtgt ttctaagaag 840

ccatcactct cagtgcagcc aggtcctatc gtggcccctg aggagaccct gactctgcag 900

tgtggctctg atgctggcta caacagattt gttctgtata aggacgggga acgtgacttc 960

cttcagctcg ctggcgcaca gccccaggct gggctctccc aggccaactt caccctgggc 1020

cctgtgagcc gctcctacgg gggccagtac agatgctacg gtgcacacaa cctctcctcc 1080

gagtggtcgg cccccagcga cccccctggac atcctgatcg caggacagtt ctatgacaga 1140

gtctccctct cggtgcagcc gggccccacg gtggcctcag gagagaacgt gaccctgctg 1200

tgtcagtcac agggatggat gcaaactttc cttctgacca aggagggggc agctgatgac 1260

ccatggcgtc taagatcaac gtaccaatct caaaaatacc aggctgaatt ccccatgggt 1320

cctgtgacct cagcccatgc ggggacctac aggtgctacg gctcacagag ctccaaaccc 1380

tacctgctga ctcaccccag tgacccctg gagctcgtgg tctcaggacc gtctgggggc 1440

cccagctccc cgacaacagg ccccacctcc acatctgcag gccctgagga ccagcccctc 1500

acccccaccg ggtcggatcc ccagagtggt ctgggaaggc acctgggggt tgtgatcggc 1560

atcttggtgg ccgtcatcct actgctcctc ctcctcctcc tcctcttcct catcctccga 1620

catcgacgtc agggcaaaca ctggacatcg acccagagaa aggctgattt ccaacatcct 1680

gcaggggctg tggggccaga gcccacagac agaggcctgc agtggaggtc cagcccagct 1740

gccgatgccc aggaagaaaa cctctatgct gccgtgaagc acacacagcc tgaggatggg 1800

gtggagatgg acactcggca gagcccacac gatgaagacc cccaggcagt gacgtatgcc 1860

gaggtgaaac actccagacc taggagagaa atggcctctc ctccttcccc actgtctggg 1920

gaattcctgg acacaaagga cagacaggcg gaagaggaca ggcagatgga cactgaggct 1980

gctgcatctg aagcccccca ggatgtgacc tacgcccagc tgcacagctt gaccctcaga 2040

cgggaggcaa ctgagcctcc tccatcccag gaagggccct ctccagctgt gcccagcatc 2100

tacgccactc tggccatcca ctagcccagg gggggacgca gaccccacac tccatggagt 2160

ctggaatgca tgggagctgc ccccccagtg gacaccattg gaccccaccc agcctggatc 2220

taccccagga gactctggga acttttaggg gtcactcaat tctgcagtat aaataactaa 2280

tgtctctaca attttgaaat aaagcaacag acttctcaat aatcaatgaa gtagctgaga 2340

aaactaagtc agaaagtgca ttaaactgaa tcacaatgta aatattacac atcaagcgat 2400

gaaactggaa aactacaagc cacgaatgaa tgaattagga aagaaaaaaa gtaggaaatg 2460

aatgatcttg gctttcctat aagaaattta gggcagggca cggtggctca cgcctgtaat 2520

tccagcactt tgggaggccg aggcgggcag atcacgagtt caggagatcg agaccatctt 2580

ggccaacatg gtgaaaccct gtctctccta aaaatacaaa aattagctgg atgtggtggc 2640

agtgcctgta atcccagcta tttgggaggc tgaggcagga gaatcgcttg aaccagggag 2700

tcagaggttt cagtgagcca agatcgcacc actgctctcc agcctggcga cagagggaga 2760

ctccatctca aattaaaaaa aaaaaaaaaa aagaaagaaa aa 2802

<210> 7
<211> 2859
<212> DNA
<213> homo sapiens

<220>
<221> misc_feature
<223> mRNA LILRB1 human, isoform 3

<400> 7

aagtcaactt ttcttcccta tttccctgca tttctcttct gtgctcgctg ccacacgcag 60
ctcagcctgg gcggcacagc cagatgcgag atgcgtctct gctgatctga gtctgcctgc 120
agcatggacc tgggtcttcc ctgaagcatc tccagggctg gagggacgac tgccatgcac 180
cgagggctca tccatccaca gagcagggca gtgggaggag acgccatgac ccccatcctc 240
acggtcctga tctgtctcgg gctgagtctg ggccccagga cccacgtgca ggcagggcac 300
ctccccaagc ccaccctctg ggctgaacca ggctctgtga tcacccaggg gagtcctgtg 360
accctcaggt gtcagggggg ccaggagacc caggagtacc gtctatatag agaaaagaaa 420
acagcactct ggattacacg gatcccacag gagcttgtga agaagggcca gttccccatc 480
ccatccatca cctgggaaca tgcagggcgg tatcgctgtt actatggtag cgacactgca 540
ggccgctcag agagcagtga cccccctggag ctggtggtga caggagccta catcaaaccc 600
accctctcag cccagcccag ccccgtggtg aactcaggag ggaatgtaat cctccagtgt 660
gactcacagg tggcatttga tggcttcagt ctgtgtaagg aaggagaaga tgaacaccca 720
caatgcctga actcccagcc ccatgcccgt gggtcgtccc gcgccatctt ctccgtgggc 780
cccgtgagcc cgagtcgcag gtggtggtac aggtgctatg cttatgactc gaactctccc 840
tatgagtggt ctctacccag tgatctcctg gagctcctgg tcctaggtgt ttctaagaag 900
ccatcactct cagtgcagcc aggtcctatc gtggcccctg aggagaccct gactctgcag 960
tgtggctctg atgctggcta caacagattt gttctgtata aggacgggga acgtgacttc 1020
cttcagctcg ctggcgcaca gccccaggct gggctctccc aggccaactt caccctgggc 1080
cctgtgagcc gctcctacgg gggccagtac agatgctacg gtgcacacaa cctctcctcc 1140
gagtggtcgg cccccagcga cccccctggac atcctgatcg caggacagtt ctatgacaga 1200
gtctccctct cggtgcagcc gggccccacg gtggcctcag gagagaacgt gaccctgctg 1260
tgtcagtcac agggatggat gcaaactttc cttctgacca aggagggggc agctgatgac 1320
ccatggcgtc taagatcaac gtaccaatct caaaaatacc aggctgaatt ccccatgggt 1380
cctgtgacct cagcccatgc ggggacctac aggtgctacg gctcacagag ctccaaaccc 1440
tacctgctga ctcaccccag tgacccccctg gagctcgtgg tctcaggacc gtctgggggc 1500
cccagctccc cgacaacagg ccccacctcc acatctgcag gccctgagga ccagcccctc 1560
acccccaccg ggtcggatcc ccagagtggt ctgggaaggc acctgggggt tgtgatcggc 1620
atcttggtgg ccgtcatcct actgctcctc ctcctcctcc tcctcttcct catcctccga 1680
catcgacgtc agggcaaaca ctggacatcg acccagagaa aggctgattt ccaacatcct 1740
gcaggggctg tggggccaga gcccacagac agaggcctgc agtggaggtc cagcccagct 1800
gccgatgccc aggaagaaaa cctctatgct gccgtgaagc acacacagcc tgaggatggg 1860

```
gtggagatgg acactcggag cccacacgat gaagaccccc aggcagtgac gtatgccgag      1920
gtgaaacact ccagacctag gagagaaatg gcctctcctc cttccccact gtctggggaa      1980
ttcctggaca caaaggacag acaggcggaa gaggacaggc agatggacac tgaggctgct      2040
gcatctgaag ccccccagga tgtgacctac gcccagctgc acagcttgac cctcagacgg      2100
gaggcaactg agcctcctcc atcccaggaa gggccctctc cagctgtgcc cagcatctac      2160
gccactctgg ccatccacta gcccaggggg ggacgcagac cccacactcc atggagtctg      2220
gaatgcatgg gagctgcccc cccagtggac accattggac cccacccagc ctggatctac      2280
cccaggagac tctgggaact tttaggggtc actcaattct gcagtataaa taactaatgt      2340
ctctacaatt ttgaaataaa gcaacagact tctcaataat caatgaagta gctgagaaaa      2400
ctaagtcaga aagtgcatta aactgaatca caatgtaaat attacacatc aagcgatgaa      2460
actggaaaac tacaagccac gaatgaatga attaggaaag aaaaaaagta ggaaatgaat      2520
gatcttggct ttcctataag aaatttaggg cagggcacgg tggctcacgc ctgtaattcc      2580
agcactttgg gaggccgagg cgggcagatc acgagttcag gagatcgaga ccatcttggc      2640
caacatggtg aaaccctgtc tctcctaaaa atacaaaaat tagctggatg tggtggcagt      2700
gcctgtaatc ccagctattt gggaggctga ggcaggagaa tcgcttgaac cagggagtca      2760
gaggtttcag tgagccaaga tcgcaccact gctctccagc ctggcgacag agggagactc      2820
catctcaaat taaaaaaaaa aaaaaaaaag aaagaaaaa                            2859
```

<210> 8
<211> 2859
<212> DNA
<213> homo sapiens

<220>
<221> misc_feature
<223> mRNA LILRB1 human, isoform 4

<400> 8

```
aagtcaactt ttcttcccta tttccctgca tttctcttct gtgctcgctg ccacacgcag      60
ctcagcctgg gcggcacagc cagatgcgag atgcgtctct gctgatctga gtctgcctgc     120
agcatggacc tgggtcttcc ctgaagcatc tccagggctg gagggacgac tgccatgcac     180
cgagggctca tccatccaca gagcagggca gtgggaggag acgccatgac ccccatcctc     240
acggtcctga tctgtctcgg gctgagtctg ggccccagga cccacgtgca ggcagggcac     300
ctccccaagc ccaccctctg ggctgaacca ggctctgtga tcacccaggg gagtcctgtg     360
accctcaggt gtcagggggg ccaggagacc caggagtacc gtctatatag agaaaagaaa     420
acagcactct ggattacacg gatcccacag gagcttgtga agaagggcca gttccccatc     480
ccatccatca cctgggaaca tgcagggcgg tatcgctgtt actatggtag cgacactgca     540
```

```
ggccgctcag agagcagtga ccccctggag ctggtggtga caggagccta catcaaaccc     600
accctctcag cccagcccag ccccgtggtg aactcaggag ggaatgtaat cctccagtgt     660
gactcacagg tggcatttga tggcttcagt ctgtgtaagg aaggagaaga tgaacaccca     720
caatgcctga actcccagcc ccatgcccgt gggtcgtccc gcgccatctt ctccgtgggc     780
cccgtgagcc cgagtcgcag gtggtggtac aggtgctatg cttatgactc gaactctccc     840
tatgagtggt ctctacccag tgatctcctg gagctcctgg tcctaggtgt ttctaagaag     900
ccatcactct cagtgcagcc aggtcctatc gtggcccctg aggagaccct gactctgcag     960
tgtggctctg atgctggcta caacagattt gttctgtata aggacgggga acgtgacttc    1020
cttcagctcg ctggcgcaca gccccaggct gggctctccc aggccaactt caccctgggc    1080
cctgtgagcc gctcctacgg gggccagtac agatgctacg gtgcacacaa cctctcctcc    1140
gagtggtcgg cccccagcga cccccctggac atcctgatcg caggacagtt ctatgacaga    1200
gtctccctct cggtgcagcc gggccccacg gtggcctcag gagagaacgt gaccctgctg    1260
tgtcagtcac agggatggat gcaaactttc cttctgacca aggagggggc agctgatgac    1320
ccatggcgtc taagatcaac gtaccaatct caaaaatacc aggctgaatt ccccatgggt    1380
cctgtgacct cagcccatgc ggggacctac aggtgctacg gctcacagag ctccaaaccc    1440
tacctgctga ctcaccccag tgacccccctg gagctcgtgg tctcaggacc gtctgggggc    1500
cccagctccc cgacaacagg ccccacctcc acatctggcc ctgaggacca gcccctcacc    1560
cccaccgggt cggatcccca gagtggtctg ggaaggcacc tgggggttgt gatcggcatc    1620
ttggtggccg tcatcctact gctcctcctc ctcctcctcc tcttcctcat cctccgacat    1680
cgacgtcagg gcaaacactg gacatcgacc cagagaaagg ctgatttcca acatcctgca    1740
ggggctgtgg ggccagagcc cacagacaga ggcctgcagt ggaggtccag cccagctgcc    1800
gatgcccagg aagaaaacct ctatgctgcc gtgaagcaca cacagcctga ggatggggtg    1860
gagatggaca ctcggcagag cccacacgat gaagaccccc aggcagtgac gtatgccgag    1920
gtgaaacact ccagacctag gagagaaatg gcctctcctc cttccccact gtctggggaa    1980
ttcctggaca caaaggacag acaggcggaa gaggacaggc agatggacac tgaggctgct    2040
gcatctgaag ccccccagga tgtgacctac gcccagctgc acagcttgac cctcagacgg    2100
gaggcaactg agcctcctcc atcccaggaa gggccctctc cagctgtgcc cagcatctac    2160
gccactctgg ccatccacta gcccaggggg ggacgcagac cccacactcc atggagtctg    2220
gaatgcatgg gagctgcccc cccagtggac accattggac cccacccagc ctggatctac    2280
cccaggagac tctgggaact tttaggggtc actcaattct gcagtataaa taactaatgt    2340
ctctacaatt ttgaaataaa gcaacagact tctcaataat caatgaagta gctgagaaaa    2400
```

```
ctaagtcaga aagtgcatta aactgaatca caatgtaaat attacacatc aagcgatgaa    2460
actggaaaac tacaagccac gaatgaatga attaggaaag aaaaaaagta ggaaatgaat    2520
gatcttggct ttcctataag aaatttaggg cagggcacgg tggctcacgc ctgtaattcc    2580
agcactttgg gaggccgagg cgggcagatc acgagttcag gagatcgaga ccatcttggc    2640
caacatggtg aaaccctgtc tctcctaaaa atacaaaaat tagctggatg tggtggcagt    2700
gcctgtaatc ccagctattt gggaggctga ggcaggagaa tcgcttgaac cagggagtca    2760
gaggtttcag tgagccaaga tcgcaccact gctctccagc ctggcgacag agggagactc    2820
catctcaaat taaaaaaaaa aaaaaaaaag aaagaaaaa                            2859
```

<210> 9
<211> 2179
<212> DNA
<213> homo sapiens

<220>
<221> misc_feature
<223> mRNA of TKT, variant 1

<400> 9

```
gatccgagcc ccgcctcctc cccctgcccc gcctctccca tccccgcccc gccccgcccg      60
gcgacttaac gcgcccccgc cccgcgcccg gcctcggcag ccgcctgtcg ccgcgggagc     120
agccgctatc tctgtgtgtc cgcgtgtgcg cccggtcccc gcctgccgca ccatggagag     180
ctaccacaag cctgaccagc agaagctgca ggccttgaag gacacggcca accgcctacg     240
tatcagctcc atccaggcca ccactgcggc gggctctggc caccccacgt catgctgcag     300
cgccgcagag atcatggctg tcctcttttt ccacaccatg cgctacaagt cccaggaccc     360
ccggaatccg cacaatgacc gctttgtgct ctccaagggc catgcagctc ccatcctcta     420
cgcggtctgg gctgaagctg gtttcctggc cgaggcggag ctgctgaacc tgaggaagat     480
cagctccgac ttggacgggc acccggtccc gaaacaagct ttcaccgacg tggccactgg     540
ctccctgggc cagggcctcg ggccgcttg tgggatggcc tacaccggca aatacttcga     600
caaggccagc taccgagtct attgcttgct gggagacggg gagctgtcag agggctctgt     660
atgggaggcc atggccttcg ccagcatcta taagctggac aaccttgtgg ccattctaga     720
catcaatcgc ctgggccaga gtgacccggc cccactgcag caccagatgg acatctacca     780
gaagcggtgc gaggccttcg gttggcatgc catcatcgtg gatggacaca gcgtggagga     840
gctgtgcaag gcctttggcc aggccaagca ccagccaaca gccatcattg ccaagacctt     900
caagggccga gggatcacgg gggtagaaga taaggagtct tggcatggga agcccctccc     960
caaaaacatg gctgagcaga tcatccagga gatctacagc cagatccaga gcaaaaagaa    1020
gatcctggca acccctccac aggaggacgc accctcagtg gacattgcca acatccgcat    1080
```

gcccagcctg cccagctaca aagttgggga caagatagcc acccgcaagg cctacgggca 1140

ggcactggcc aagctgggcc atgccagtga ccgcatcatc gccctggatg gggacaccaa 1200

aaattccacc ttctcggaga tcttcaaaaa ggagcacccg gaccgcttca tcgagtgcta 1260

cattgctgag cagaacatgg tgagcatcgc ggtgggctgt gccacccgca acaggacggt 1320

gcccttctgc agcacttttg cagccttctt cacgcgggcc tttgaccaga ttcgcatggc 1380

cgccatctcc gagagcaaca tcaacctctg cggctcccac tgcggcgttt ccatcgggga 1440

agacgggccc tcccagatgg ccctagaaga tctggctatg tttcggtcag tccccacatc 1500

aactgtcttt tacccaagtg atggcgttgc tacagagaag gcagtggaac tagccgccaa 1560

tacaaagggt atctgcttca tccggaccag ccgcccagaa aatgccatca tctataacaa 1620

caatgaggac ttccaggtcg gacaagccaa ggtggtcctg aagagcaagg atgaccaggt 1680

gaccgttatc ggggctgggg tgaccctgca cgaggccttg gccgctgccg aactgctgaa 1740

gaaagaaaag atcaacatcc gcgtgctgga ccccttcacc atcaagcccc tggacagaaa 1800

actcattctc gacagcgctc gtgccaccaa gggcaggatc ctcaccgtgg aggaccatta 1860

ttatgaaggt ggcattggtg aggctgtgtc cagtgcagta gtgggcgagc ctggcatcac 1920

tgtcacccac ctggcagtta accgggtacc aagaagtggg aagccggctg agctgctgaa 1980

gatgtttggt atcgacaggg atgccattgc acaagctgtg aggggcctca tcaccaaggc 2040

ctagggcggg tatgaagtgt ggggcggggg tctatacatt cctgagattc tgggaaaggt 2100

gctcaaagat gtactgagag gaggggtaaa tatatgtttt gagaaaaatg aattggccct 2160

gaaaaaaaaa aaaaaaaaa 2179

<210> 10
<211> 2957
<212> DNA
<213> homo sapiens

<220>
<221> misc_feature
<223> Variant 2 de TKT

<400> 10

gatccgagcc ccgcctcctc cccctgcccc gcctctccca tccccgcccc gccccgcccg 60

gcgacttaac gcgcccccgc cccgcgcccg gcctcggcag ccgcctgtcg ccgcgggagc 120

agccgctatc tctgtgtgtc cgcgtgtgcg cccggtcccc gcctgccgca ccatggagag 180

ctaccacaag cctgaccagc agaagctgca ggccttgaag gacacggcca accgcctacg 240

tatcagctcc atccaggcca ccactgcggc gggctctggc caccccacgt catgctgcag 300

cgccgcagag atcatggctg tcctcttttt ccacaccatg cgctacaagt cccaggaccc 360

ccggaatccg cacaatgacc gctttgtgct ctccaagggc catgcagctc ccatcctcta 420
cgcggtctgg gctgaagctg gtttcctggc cgaggcggag ctgctgaacc tgaggaagat 480
cagctccgac ttggacgggc acccggtccc gaaacaagct ttcaccgacg tggccactgg 540
ctccctgggc cagggcctcg ggccgcttg tgggatggcc tacaccggca aatacttcga 600
caaggccagc taccgagtct attgcttgct gggagacggg gagctgtcag agggctctgt 660
atgggaggcc atggccttcg ccagcatcta taagctggac aaccttgtgg ccattctaga 720
catcaatcgc ctgggccaga gtgacccggc cccactgcag caccagatgg acatctacca 780
gaagcggtgc gaggccttcg gttggcatgc catcatcgtg gatggacaca gcgtggagga 840
gctgtgcaag gcctttggcc aggccaagca ccagccaaca gccatcattg ccaagacctt 900
caagggccga gggatcacgg gggtagaaga taaggagtct tggcatggga agcccctccc 960
caaaaacatg gctgagcaga tcatccagga gatctacagc cagatccaga gcaaaaagaa 1020
gatcctggca acccctccac aggaggacgc accctcagtg gacattgcca acatccgcat 1080
gcccagcctg cccagctaca aagttgggga caagatagcc acccgcaagg cctacgggca 1140
ggcactggcc aagctgggcc atgccagtga ccgcatcatc gccctggatg gggacaccaa 1200
aaattccacc ttctcggaga tcttcaaaaa ggagcacccg gaccgcttca tcgagtgcta 1260
cattgctgag cagaacatgg tgagcatcgc ggtgggctgt gccacccgca acaggacggt 1320
gcccttctgc agcactttg cagccttctt cacgcgggcc tttgaccaga ttcgcatggc 1380
cgccatctcc gagagcaaca tcaacctctg cggctcccac tgcggcgttt ccatcgggga 1440
agacgggccc tcccagatgg ccctagaaga tctggctatg tttcggtcag tccccacatc 1500
aactgtcttt tacccaagtg atggcgttgc tacagagaag gcagtggaac tagccgccaa 1560
tacaaagggt atctgcttca tccggaccag ccgcccagaa aatgccatca tctataacaa 1620
caatgaggac ttccaggtcg gacaagccaa ggtggtcctg aagagcaagg atgaccaggt 1680
gaccgttatc ggggctgggg tgaccctgca cgaggccttg gccgctgccg aactgctgaa 1740
gaaagaaaag atcaacatcc gcgtgctgga ccccttcacc atcaagcccc tggacagaaa 1800
actcattctc gacagcgctc gtgccaccaa gggcaggatc ctcaccgtgg aggaccatta 1860
ttatgaaggt ggcattggtg aggctgtgtc cagtgcagta gtgggcgagc ctggcatcac 1920
tgtcacccac ctggcagtta accgggtacc aagaagtggg aagccggctg agctgctgaa 1980
gatgtttggt atcgacaggg atgccattgc acaagctgtg aggggcctca tcaccaaggc 2040
ctagggcggc cagtgcaggt tctggggaag aagcgtggag ggtgcgctag ggaggctggt 2100
gtccttggtc ctgggccagg acctgggtgt cccaagtccc cttggaatca cctgcaactg 2160
ccctcaacct ccagaactat cgctgcctcc cattcatcac caggtgacat ttgactgcaa 2220
cctgccttct agctgagagg ctgagaccta catccctcat tgtgacctca gtccacctgg 2280

```
ccctgagcgg gctggggaac tgcctcagtc tggaagctga ccaggcactc tcagggccgc    2340
cccacctccc ccaagtcccc acagccttgc agtcaggtct cctgggatag ggaggttcac    2400
ttgcttgttg tccctcgtcc ttgtcatatc cttttaacta ggcatctcag agaagcagag    2460
acagggcagc cttcgtcctg ggggaaaagg gaccctcagg atggcatgag aggtcctcaa    2520
tcccaagtgt ggaactgtcc ccctcaactt gttaaaatgc agatttctgg gtcttgccaa    2580
tggggcctgg gactccatgt gacaactggc ccaggagctt ctgatgtcac acagaattct    2640
gcagtcccaa gctccagccc cgacctgctc tgctgttcct aggtgactgc cctcacactg    2700
ctgaccacag tggatttctc cccctgctgc tcgggctcag ctggggtcag ccctgcttat    2760
aaggtcaact gtgcaaaacc ttatactggc caagaacaaa ctagtgctgg gggaggaggg    2820
ctgggtgccc cggccactgg tggagtcccc aggaaatcct cagagctgtt gcgaggatga    2880
gacacatttg tggacacgtc cacctgtcct cctgaccgtc tggagagaat aaacctgtca    2940
aagccaaggt caaaaaa                                                   2957
```

<210> 11
<211> 1205
<212> DNA
<213> homo sapiens

<220>
<221> misc_feature
<223> mRNA of CEACAM4

<400> 11

```
acagaaggag gaaggtcagc agccccgaca gccgacagtc acagcagctc tgacaagagc      60
gttcctggag cccagctcct ctccacagag gacaagcagg cagcagagac catgggcccc     120
ccctcagccg ctccccgtgg agggcacagg ccctggcagg ggctcctgat cacagcctca     180
cttttaacct tctggcaccc gcccaccact gtccagttca ctattgaagc cctgccgtcc     240
agtgctgcag agggaaagga tgttcttcta ctggcctgca atatttcaga aactattcaa     300
gcctattatt ggcacaaggg gaaaacggca gaagggagcc ctctcattgc tggttatata     360
acagacattc aagcaaatat cccaggggcc gcatacagtg gtcgagagac agtataccccc    420
aatggatccc tgctgttcca aaacatcacc ctggaggacg caggatccta caccctacga     480
accataaatg ccagttacga ctctgaccaa gcaactggcc agctccacgt acaccaaaac     540
aacgtcccag gccttcctgt gggggccgtc gctggcatcg tgactggggt cctggttggg     600
gtggctctgg tggccgccct ggtgtgtttt ctgcttctct ccaggactgg aagggccagc     660
atccagcgtg acctcaggga gcagccgccc ccagcctcca cccctggcca tggtccctct     720
cacagatcca ccttctcggc ccctctaccc agccccagaa cagccactcc catctatgag     780
```

gaattgctat actctgatgc aaacatttac tgccagatcg accacaaagc agatgtggtc 840

tcttaggttc ctctgggagc tgctcttgtg ggttgatgga gcgtccccaa agctcccagc 900

cctggggacg gggaaggaca tggagcctga gccagagaac cagctctgag tcctgaggag 960

acacaggcct ggggacaggg agggatggga gtccctgctg aatatctgga gaccctgaca 1020

ggttgccctg ggctctgggt gggccgggac aaaggcctct catcaccaca ggaagcgggg 1080

gcttgcaagg aaagtgaatg ggcctgtggc ccacccgggg tcaccaggaa aggatctgaa 1140

taaagaggac ccttcctctc attggctctt tttctgctca cgggaactta gcagaaactc 1200

acctg 1205

<210> 12
<211> 148
<212> PRT
<213> homo sapiens

<220>
<221> misc_feature
<223> protein Lysozyme

<400> 12

```
Met Lys Ala Leu Ile Val Leu Gly Leu Val Leu Leu Ser Val Thr Val
1               5                   10                  15

Gln Gly Lys Val Phe Glu Arg Cys Glu Leu Ala Arg Thr Leu Lys Arg
            20                  25                  30

Leu Gly Met Asp Gly Tyr Arg Gly Ile Ser Leu Ala Asn Trp Met Cys
        35                  40                  45

Leu Ala Lys Trp Glu Ser Gly Tyr Asn Thr Arg Ala Thr Asn Tyr Asn
    50                  55                  60

Ala Gly Asp Arg Ser Thr Asp Tyr Gly Ile Phe Gln Ile Asn Ser Arg
65                  70                  75                  80

Tyr Trp Cys Asn Asp Gly Lys Thr Pro Gly Ala Val Asn Ala Cys His
                85                  90                  95

Leu Ser Cys Ser Ala Leu Leu Gln Asp Asn Ile Ala Asp Ala Val Ala
            100                 105                 110

Cys Ala Lys Arg Val Val Arg Asp Pro Gln Gly Ile Arg Ala Trp Val
        115                 120                 125

Ala Trp Arg Asn Arg Cys Gln Asn Arg Asp Val Arg Gln Tyr Val Gln
    130                 135                 140

Gly Cys Gly Val
145
```

<210> 13
<211> 545
<212> PRT
<213> homo sapiens

<220>
<221> misc_feature
<223> isoform A, protein G6PD

<400> 13

```
Met Gly Arg Arg Gly Ser Ala Pro Gly Asn Gly Arg Thr Leu Arg Gly
1               5                   10                  15

Cys Glu Arg Gly Gly Arg Arg Arg Arg Ser Ala Asp Ser Val Met Ala
            20                  25                  30

Glu Gln Val Ala Leu Ser Arg Thr Gln Val Cys Gly Ile Leu Arg Glu
        35                  40                  45

Glu Leu Phe Gln Gly Asp Ala Phe His Gln Ser Asp Thr His Ile Phe
    50                  55                  60

Ile Ile Met Gly Ala Ser Gly Asp Leu Ala Lys Lys Lys Ile Tyr Pro
65                  70                  75                  80

Thr Ile Trp Trp Leu Phe Arg Asp Gly Leu Leu Pro Glu Asn Thr Phe
                85                  90                  95

Ile Val Gly Tyr Ala Arg Ser Arg Leu Thr Val Ala Asp Ile Arg Lys
            100                 105                 110

Gln Ser Glu Pro Phe Phe Lys Ala Thr Pro Glu Glu Lys Leu Lys Leu
        115                 120                 125

Glu Asp Phe Phe Ala Arg Asn Ser Tyr Val Ala Gly Gln Tyr Asp Asp
    130                 135                 140

Ala Ala Ser Tyr Gln Arg Leu Asn Ser His Met Asn Ala Leu His Leu
145                 150                 155                 160

Gly Ser Gln Ala Asn Arg Leu Phe Tyr Leu Ala Leu Pro Pro Thr Val
                165                 170                 175

Tyr Glu Ala Val Thr Lys Asn Ile His Glu Ser Cys Met Ser Gln Ile
```

180 185 190

Gly Trp Asn Arg Ile Ile Val Glu Lys Pro Phe Gly Arg Asp Leu Gln
195 200 205

Ser Ser Asp Arg Leu Ser Asn His Ile Ser Ser Leu Phe Arg Glu Asp
210 215 220

Gln Ile Tyr Arg Ile Asp His Tyr Leu Gly Lys Glu Met Val Gln Asn
225 230 235 240

Leu Met Val Leu Arg Phe Ala Asn Arg Ile Phe Gly Pro Ile Trp Asn
245 250 255

Arg Asp Asn Ile Ala Cys Val Ile Leu Thr Phe Lys Glu Pro Phe Gly
260 265 270

Thr Glu Gly Arg Gly Gly Tyr Phe Asp Glu Phe Gly Ile Ile Arg Asp
275 280 285

Val Met Gln Asn His Leu Leu Gln Met Leu Cys Leu Val Ala Met Glu
290 295 300

Lys Pro Ala Ser Thr Asn Ser Asp Asp Val Arg Asp Glu Lys Val Lys
305 310 315 320

Val Leu Lys Cys Ile Ser Glu Val Gln Ala Asn Asn Val Val Leu Gly
325 330 335

Gln Tyr Val Gly Asn Pro Asp Gly Glu Gly Glu Ala Thr Lys Gly Tyr
340 345 350

Leu Asp Asp Pro Thr Val Pro Arg Gly Ser Thr Thr Ala Thr Phe Ala
355 360 365

Ala Val Val Leu Tyr Val Glu Asn Glu Arg Trp Asp Gly Val Pro Phe
370 375 380

Ile Leu Arg Cys Gly Lys Ala Leu Asn Glu Arg Lys Ala Glu Val Arg
385 390 395 400

Leu Gln Phe His Asp Val Ala Gly Asp Ile Phe His Gln Gln Cys Lys
405 410 415

Arg Asn Glu Leu Val Ile Arg Val Gln Pro Asn Glu Ala Val Tyr Thr
420 425 430

Lys Met Met Thr Lys Lys Pro Gly Met Phe Phe Asn Pro Glu Glu Ser
435 440 445

Glu Leu Asp Leu Thr Tyr Gly Asn Arg Tyr Lys Asn Val Lys Leu Pro
450 455 460

Asp Ala Tyr Glu Arg Leu Ile Leu Asp Val Phe Cys Gly Ser Gln Met
465 470 475 480

His Phe Val Arg Ser Asp Glu Leu Arg Glu Ala Trp Arg Ile Phe Thr
485 490 495

Pro Leu Leu His Gln Ile Glu Leu Glu Lys Pro Lys Pro Ile Pro Tyr
500 505 510

Ile Tyr Gly Ser Arg Gly Pro Thr Glu Ala Asp Glu Leu Met Lys Arg
515 520 525

Val Gly Phe Gln Tyr Glu Gly Thr Tyr Lys Trp Val Asn Pro His Lys
530 535 540

Leu
545

<210> 14
<211> 515
<212> PRT
<213> homo sapiens

<220>
<221> misc_feature
<223> isoform B, protein G6PD human

<400> 14

Met Ala Glu Gln Val Ala Leu Ser Arg Thr Gln Val Cys Gly Ile Leu
1 5 10 15

Arg Glu Glu Leu Phe Gln Gly Asp Ala Phe His Gln Ser Asp Thr His
20 25 30

Ile Phe Ile Ile Met Gly Ala Ser Gly Asp Leu Ala Lys Lys Lys Ile
35 40 45

Tyr Pro Thr Ile Trp Trp Leu Phe Arg Asp Gly Leu Leu Pro Glu Asn
50 55 60

Thr Phe Ile Val Gly Tyr Ala Arg Ser Arg Leu Thr Val Ala Asp Ile
65 70 75 80

```
Arg Lys Gln Ser Glu Pro Phe Phe Lys Ala Thr Pro Glu Glu Lys Leu
                85                  90                  95

Lys Leu Glu Asp Phe Phe Ala Arg Asn Ser Tyr Val Ala Gly Gln Tyr
            100                 105                 110

Asp Asp Ala Ala Ser Tyr Gln Arg Leu Asn Ser His Met Asn Ala Leu
        115                 120                 125

His Leu Gly Ser Gln Ala Asn Arg Leu Phe Tyr Leu Ala Leu Pro Pro
    130                 135                 140

Thr Val Tyr Glu Ala Val Thr Lys Asn Ile His Glu Ser Cys Met Ser
145                 150                 155                 160

Gln Ile Gly Trp Asn Arg Ile Ile Val Glu Lys Pro Phe Gly Arg Asp
                165                 170                 175

Leu Gln Ser Ser Asp Arg Leu Ser Asn His Ile Ser Ser Leu Phe Arg
            180                 185                 190

Glu Asp Gln Ile Tyr Arg Ile Asp His Tyr Leu Gly Lys Glu Met Val
        195                 200                 205

Gln Asn Leu Met Val Leu Arg Phe Ala Asn Arg Ile Phe Gly Pro Ile
    210                 215                 220

Trp Asn Arg Asp Asn Ile Ala Cys Val Ile Leu Thr Phe Lys Glu Pro
225                 230                 235                 240

Phe Gly Thr Glu Gly Arg Gly Gly Tyr Phe Asp Glu Phe Gly Ile Ile
                245                 250                 255

Arg Asp Val Met Gln Asn His Leu Leu Gln Met Leu Cys Leu Val Ala
            260                 265                 270

Met Glu Lys Pro Ala Ser Thr Asn Ser Asp Asp Val Arg Asp Glu Lys
        275                 280                 285

Val Lys Val Leu Lys Cys Ile Ser Glu Val Gln Ala Asn Asn Val Val
    290                 295                 300

Leu Gly Gln Tyr Val Gly Asn Pro Asp Gly Glu Gly Glu Ala Thr Lys
305                 310                 315                 320

Gly Tyr Leu Asp Asp Pro Thr Val Pro Arg Gly Ser Thr Thr Ala Thr
                325                 330                 335
```

```
Phe Ala Ala Val Val Leu Tyr Val Glu Asn Glu Arg Trp Asp Gly Val
            340                 345                 350

Pro Phe Ile Leu Arg Cys Gly Lys Ala Leu Asn Glu Arg Lys Ala Glu
        355                 360                 365

Val Arg Leu Gln Phe His Asp Val Ala Gly Asp Ile Phe His Gln Gln
    370                 375                 380

Cys Lys Arg Asn Glu Leu Val Ile Arg Val Gln Pro Asn Glu Ala Val
385                 390                 395                 400

Tyr Thr Lys Met Met Thr Lys Lys Pro Gly Met Phe Phe Asn Pro Glu
                405                 410                 415

Glu Ser Glu Leu Asp Leu Thr Tyr Gly Asn Arg Tyr Lys Asn Val Lys
            420                 425                 430

Leu Pro Asp Ala Tyr Glu Arg Leu Ile Leu Asp Val Phe Cys Gly Ser
        435                 440                 445

Gln Met His Phe Val Arg Ser Asp Glu Leu Arg Glu Ala Trp Arg Ile
    450                 455                 460

Phe Thr Pro Leu Leu His Gln Ile Glu Leu Glu Lys Pro Lys Pro Ile
465                 470                 475                 480

Pro Tyr Ile Tyr Gly Ser Arg Gly Pro Thr Glu Ala Asp Glu Leu Met
                485                 490                 495

Lys Arg Val Gly Phe Gln Tyr Glu Gly Thr Tyr Lys Trp Val Asn Pro
            500                 505                 510

His Lys Leu
        515
```

<210> 15
<211> 483
<212> PRT
<213> homo sapiens

<220>
<221> misc_feature
<223> Protein 6PGD

<400> 15

```
Met Ala Gln Ala Asp Ile Ala Leu Ile Gly Leu Ala Val Met Gly Gln
```

```
1               5                   10                  15

Asn Leu Ile Leu Asn Met Asn Asp His Gly Phe Val Val Cys Ala Phe
            20                  25                  30

Asn Arg Thr Val Ser Lys Val Asp Asp Phe Leu Ala Asn Glu Ala Lys
        35                  40                  45

Gly Thr Lys Val Val Gly Ala Gln Ser Leu Lys Glu Met Val Ser Lys
    50                  55                  60

Leu Lys Lys Pro Arg Arg Ile Ile Leu Leu Val Lys Ala Gly Gln Ala
65                  70                  75                  80

Val Asp Asp Phe Ile Glu Lys Leu Val Pro Leu Leu Asp Thr Gly Asp
                85                  90                  95

Ile Ile Ile Asp Gly Gly Asn Ser Glu Tyr Arg Asp Thr Thr Arg Arg
            100                 105                 110

Cys Arg Asp Leu Lys Ala Lys Gly Ile Leu Phe Val Gly Ser Gly Val
        115                 120                 125

Ser Gly Gly Glu Glu Gly Ala Arg Tyr Gly Pro Ser Leu Met Pro Gly
    130                 135                 140

Gly Asn Lys Glu Ala Trp Pro His Ile Lys Thr Ile Phe Gln Gly Ile
145                 150                 155                 160

Ala Ala Lys Val Gly Thr Gly Glu Pro Cys Cys Asp Trp Val Gly Asp
                165                 170                 175

Glu Gly Ala Gly His Phe Val Lys Met Val His Asn Gly Ile Glu Tyr
            180                 185                 190

Gly Asp Met Gln Leu Ile Cys Glu Ala Tyr His Leu Met Lys Asp Val
        195                 200                 205

Leu Gly Met Ala Gln Asp Glu Met Ala Gln Ala Phe Glu Asp Trp Asn
    210                 215                 220

Lys Thr Glu Leu Asp Ser Phe Leu Ile Glu Ile Thr Ala Asn Ile Leu
225                 230                 235                 240

Lys Phe Gln Asp Thr Asp Gly Lys His Leu Leu Pro Lys Ile Arg Asp
                245                 250                 255
```

```
Ser Ala Gly Gln Lys Gly Thr Gly Lys Trp Thr Ala Ile Ser Ala Leu
            260                 265                 270

Glu Tyr Gly Val Pro Val Thr Leu Ile Gly Glu Ala Val Phe Ala Arg
        275                 280                 285

Cys Leu Ser Ser Leu Lys Asp Glu Arg Ile Gln Ala Ser Lys Lys Leu
    290                 295                 300

Lys Gly Pro Gln Lys Phe Gln Phe Asp Gly Asp Lys Lys Ser Phe Leu
305                 310                 315                 320

Glu Asp Ile Arg Lys Ala Leu Tyr Ala Ser Lys Ile Ile Ser Tyr Ala
                325                 330                 335

Gln Gly Phe Met Leu Leu Arg Gln Ala Ala Thr Glu Phe Gly Trp Thr
            340                 345                 350

Leu Asn Tyr Gly Gly Ile Ala Leu Met Trp Arg Gly Gly Cys Ile Ile
        355                 360                 365

Arg Ser Val Phe Leu Gly Lys Ile Lys Asp Ala Phe Asp Arg Asn Pro
    370                 375                 380

Glu Leu Gln Asn Leu Leu Leu Asp Asp Phe Phe Lys Ser Ala Val Glu
385                 390                 395                 400

Asn Cys Gln Asp Ser Trp Arg Arg Ala Val Ser Thr Gly Val Gln Ala
                405                 410                 415

Gly Ile Pro Met Pro Cys Phe Thr Thr Ala Leu Ser Phe Tyr Asp Gly
            420                 425                 430

Tyr Arg His Glu Met Leu Pro Ala Ser Leu Ile Gln Ala Gln Arg Asp
        435                 440                 445

Tyr Phe Gly Ala His Thr Tyr Glu Leu Leu Ala Lys Pro Gly Gln Phe
    450                 455                 460

Ile His Thr Asn Trp Thr Gly His Gly Gly Thr Val Ser Ser Ser Ser
465                 470                 475                 480

Tyr Asn Ala
```

<210> 16
<211> 650
<212> PRT

<213> homo sapiens

<220>
<221> misc_feature
<223> Protein LILRB1, isoform 1

<400> 16

```
Met Thr Pro Ile Leu Thr Val Leu Ile Cys Leu Gly Leu Ser Leu Gly
1               5                   10                  15

Pro Arg Thr His Val Gln Ala Gly His Leu Pro Lys Pro Thr Leu Trp
            20                  25                  30

Ala Glu Pro Gly Ser Val Ile Thr Gln Gly Ser Pro Val Thr Leu Arg
        35                  40                  45

Cys Gln Gly Gly Gln Glu Thr Gln Glu Tyr Arg Leu Tyr Arg Glu Lys
    50                  55                  60

Lys Thr Ala Leu Trp Ile Thr Arg Ile Pro Gln Glu Leu Val Lys Lys
65                  70                  75                  80

Gly Gln Phe Pro Ile Pro Ser Ile Thr Trp Glu His Ala Gly Arg Tyr
                85                  90                  95

Arg Cys Tyr Tyr Gly Ser Asp Thr Ala Gly Arg Ser Glu Ser Ser Asp
            100                 105                 110

Pro Leu Glu Leu Val Val Thr Gly Ala Tyr Ile Lys Pro Thr Leu Ser
        115                 120                 125

Ala Gln Pro Ser Pro Val Val Asn Ser Gly Gly Asn Val Ile Leu Gln
    130                 135                 140

Cys Asp Ser Gln Val Ala Phe Asp Gly Phe Ser Leu Cys Lys Glu Gly
145                 150                 155                 160

Glu Asp Glu His Pro Gln Cys Leu Asn Ser Gln Pro His Ala Arg Gly
                165                 170                 175

Ser Ser Arg Ala Ile Phe Ser Val Gly Pro Val Ser Pro Ser Arg Arg
            180                 185                 190

Trp Trp Tyr Arg Cys Tyr Ala Tyr Asp Ser Asn Ser Pro Tyr Glu Trp
        195                 200                 205

Ser Leu Pro Ser Asp Leu Leu Glu Leu Leu Val Leu Gly Val Ser Lys
    210                 215                 220
```

```
Lys Pro Ser Leu Ser Val Gln Pro Gly Pro Ile Val Ala Pro Glu Glu
225                 230                 235                 240

Thr Leu Thr Leu Gln Cys Gly Ser Asp Ala Gly Tyr Asn Arg Phe Val
                245                 250                 255

Leu Tyr Lys Asp Gly Glu Arg Asp Phe Leu Gln Leu Ala Gly Ala Gln
            260                 265                 270

Pro Gln Ala Gly Leu Ser Gln Ala Asn Phe Thr Leu Gly Pro Val Ser
        275                 280                 285

Arg Ser Tyr Gly Gly Gln Tyr Arg Cys Tyr Gly Ala His Asn Leu Ser
    290                 295                 300

Ser Glu Trp Ser Ala Pro Ser Asp Pro Leu Asp Ile Leu Ile Ala Gly
305                 310                 315                 320

Gln Phe Tyr Asp Arg Val Ser Leu Ser Val Gln Pro Gly Pro Thr Val
                325                 330                 335

Ala Ser Gly Glu Asn Val Thr Leu Leu Cys Gln Ser Gln Gly Trp Met
            340                 345                 350

Gln Thr Phe Leu Leu Thr Lys Glu Gly Ala Ala Asp Asp Pro Trp Arg
        355                 360                 365

Leu Arg Ser Thr Tyr Gln Ser Gln Lys Tyr Gln Ala Glu Phe Pro Met
    370                 375                 380

Gly Pro Val Thr Ser Ala His Ala Gly Thr Tyr Arg Cys Tyr Gly Ser
385                 390                 395                 400

Gln Ser Ser Lys Pro Tyr Leu Leu Thr His Pro Ser Asp Pro Leu Glu
                405                 410                 415

Leu Val Val Ser Gly Pro Ser Gly Gly Pro Ser Ser Pro Thr Thr Gly
            420                 425                 430

Pro Thr Ser Thr Ser Gly Pro Glu Asp Gln Pro Leu Thr Pro Thr Gly
        435                 440                 445

Ser Asp Pro Gln Ser Gly Leu Gly Arg His Leu Gly Val Val Ile Gly
    450                 455                 460

Ile Leu Val Ala Val Ile Leu Leu Leu Leu Leu Leu Leu Leu Leu Phe
```

465 470 475 480

Leu Ile Leu Arg His Arg Arg Gln Gly Lys His Trp Thr Ser Thr Gln
485 490 495

Arg Lys Ala Asp Phe Gln His Pro Ala Gly Ala Val Gly Pro Glu Pro
500 505 510

Thr Asp Arg Gly Leu Gln Trp Arg Ser Ser Pro Ala Ala Asp Ala Gln
515 520 525

Glu Glu Asn Leu Tyr Ala Ala Val Lys His Thr Gln Pro Glu Asp Gly
530 535 540

Val Glu Met Asp Thr Arg Ser Pro His Asp Glu Asp Pro Gln Ala Val
545 550 555 560

Thr Tyr Ala Glu Val Lys His Ser Arg Pro Arg Arg Glu Met Ala Ser
565 570 575

Pro Pro Ser Pro Leu Ser Gly Glu Phe Leu Asp Thr Lys Asp Arg Gln
580 585 590

Ala Glu Glu Asp Arg Gln Met Asp Thr Glu Ala Ala Ala Ser Glu Ala
595 600 605

Pro Gln Asp Val Thr Tyr Ala Gln Leu His Ser Leu Thr Leu Arg Arg
610 615 620

Glu Ala Thr Glu Pro Pro Pro Ser Gln Glu Gly Pro Ser Pro Ala Val
625 630 635 640

Pro Ser Ile Tyr Ala Thr Leu Ala Ile His
645 650

<210> 17
<211> 652
<212> PRT
<213> homo sapiens

<220>
<221> misc_feature
<223> Protein LILRB1, isoform 2

<400> 17

Met Thr Pro Ile Leu Thr Val Leu Ile Cys Leu Gly Leu Ser Leu Gly
1 5 10 15

```
Pro Arg Thr His Val Gln Ala Gly His Leu Pro Lys Pro Thr Leu Trp
            20                  25                  30

Ala Glu Pro Gly Ser Val Ile Thr Gln Gly Ser Pro Val Thr Leu Arg
        35                  40                  45

Cys Gln Gly Gly Gln Glu Thr Gln Glu Tyr Arg Leu Tyr Arg Glu Lys
    50                  55                  60

Lys Thr Ala Leu Trp Ile Thr Arg Ile Pro Gln Glu Leu Val Lys Lys
65                  70                  75                  80

Gly Gln Phe Pro Ile Pro Ser Ile Thr Trp Glu His Ala Gly Arg Tyr
                85                  90                  95

Arg Cys Tyr Tyr Gly Ser Asp Thr Ala Gly Arg Ser Glu Ser Ser Asp
            100                 105                 110

Pro Leu Glu Leu Val Val Thr Gly Ala Tyr Ile Lys Pro Thr Leu Ser
        115                 120                 125

Ala Gln Pro Ser Pro Val Val Asn Ser Gly Gly Asn Val Ile Leu Gln
    130                 135                 140

Cys Asp Ser Gln Val Ala Phe Asp Gly Phe Ser Leu Cys Lys Glu Gly
145                 150                 155                 160

Glu Asp Glu His Pro Gln Cys Leu Asn Ser Gln Pro His Ala Arg Gly
                165                 170                 175

Ser Ser Arg Ala Ile Phe Ser Val Gly Pro Val Ser Pro Ser Arg Arg
            180                 185                 190

Trp Trp Tyr Arg Cys Tyr Ala Tyr Asp Ser Asn Ser Pro Tyr Glu Trp
        195                 200                 205

Ser Leu Pro Ser Asp Leu Leu Glu Leu Leu Val Leu Gly Val Ser Lys
    210                 215                 220

Lys Pro Ser Leu Ser Val Gln Pro Gly Pro Ile Val Ala Pro Glu Glu
225                 230                 235                 240

Thr Leu Thr Leu Gln Cys Gly Ser Asp Ala Gly Tyr Asn Arg Phe Val
                245                 250                 255

Leu Tyr Lys Asp Gly Glu Arg Asp Phe Leu Gln Leu Ala Gly Ala Gln
            260                 265                 270
```

Pro Gln Ala Gly Leu Ser Gln Ala Asn Phe Thr Leu Gly Pro Val Ser
275 280 285

Arg Ser Tyr Gly Gly Gln Tyr Arg Cys Tyr Gly Ala His Asn Leu Ser
290 295 300

Ser Glu Trp Ser Ala Pro Ser Asp Pro Leu Asp Ile Leu Ile Ala Gly
305 310 315 320

Gln Phe Tyr Asp Arg Val Ser Leu Ser Val Gln Pro Gly Pro Thr Val
325 330 335

Ala Ser Gly Glu Asn Val Thr Leu Leu Cys Gln Ser Gln Gly Trp Met
340 345 350

Gln Thr Phe Leu Leu Thr Lys Glu Gly Ala Ala Asp Asp Pro Trp Arg
355 360 365

Leu Arg Ser Thr Tyr Gln Ser Gln Lys Tyr Gln Ala Glu Phe Pro Met
370 375 380

Gly Pro Val Thr Ser Ala His Ala Gly Thr Tyr Arg Cys Tyr Gly Ser
385 390 395 400

Gln Ser Ser Lys Pro Tyr Leu Leu Thr His Pro Ser Asp Pro Leu Glu
405 410 415

Leu Val Val Ser Gly Pro Ser Gly Gly Pro Ser Ser Pro Thr Thr Gly
420 425 430

Pro Thr Ser Thr Ser Ala Gly Pro Glu Asp Gln Pro Leu Thr Pro Thr
435 440 445

Gly Ser Asp Pro Gln Ser Gly Leu Gly Arg His Leu Gly Val Val Ile
450 455 460

Gly Ile Leu Val Ala Val Ile Leu Leu Leu Leu Leu Leu Leu Leu Leu
465 470 475 480

Phe Leu Ile Leu Arg His Arg Arg Gln Gly Lys His Trp Thr Ser Thr
485 490 495

Gln Arg Lys Ala Asp Phe Gln His Pro Ala Gly Ala Val Gly Pro Glu
500 505 510

Pro Thr Asp Arg Gly Leu Gln Trp Arg Ser Ser Pro Ala Ala Asp Ala
515 520 525

```
Gln Glu Glu Asn Leu Tyr Ala Ala Val Lys His Thr Gln Pro Glu Asp
    530                 535                 540

Gly Val Glu Met Asp Thr Arg Gln Ser Pro His Asp Glu Asp Pro Gln
545                 550                 555                 560

Ala Val Thr Tyr Ala Glu Val Lys His Ser Arg Pro Arg Arg Glu Met
                565                 570                 575

Ala Ser Pro Pro Ser Pro Leu Ser Gly Glu Phe Leu Asp Thr Lys Asp
            580                 585                 590

Arg Gln Ala Glu Glu Asp Arg Gln Met Asp Thr Glu Ala Ala Ala Ser
        595                 600                 605

Glu Ala Pro Gln Asp Val Thr Tyr Ala Gln Leu His Ser Leu Thr Leu
    610                 615                 620

Arg Arg Glu Ala Thr Glu Pro Pro Pro Ser Gln Glu Gly Pro Ser Pro
625                 630                 635                 640

Ala Val Pro Ser Ile Tyr Ala Thr Leu Ala Ile His
                645                 650
```

<210> 18
<211> 651
<212> PRT
<213> homo sapiens

<220>
<221> misc_feature
<223> Protein LILRB1, isoform 3

<400> 18

```
Met Thr Pro Ile Leu Thr Val Leu Ile Cys Leu Gly Leu Ser Leu Gly
1               5                   10                  15

Pro Arg Thr His Val Gln Ala Gly His Leu Pro Lys Pro Thr Leu Trp
            20                  25                  30

Ala Glu Pro Gly Ser Val Ile Thr Gln Gly Ser Pro Val Thr Leu Arg
        35                  40                  45

Cys Gln Gly Gly Gln Glu Thr Gln Glu Tyr Arg Leu Tyr Arg Glu Lys
    50                  55                  60

Lys Thr Ala Leu Trp Ile Thr Arg Ile Pro Gln Glu Leu Val Lys Lys
```

```
65                  70                  75                  80

Gly Gln Phe Pro Ile Pro Ser Ile Thr Trp Glu His Ala Gly Arg Tyr
                85                  90                  95

Arg Cys Tyr Tyr Gly Ser Asp Thr Ala Gly Arg Ser Glu Ser Ser Asp
            100                 105                 110

Pro Leu Glu Leu Val Val Thr Gly Ala Tyr Ile Lys Pro Thr Leu Ser
        115                 120                 125

Ala Gln Pro Ser Pro Val Val Asn Ser Gly Gly Asn Val Ile Leu Gln
    130                 135                 140

Cys Asp Ser Gln Val Ala Phe Asp Gly Phe Ser Leu Cys Lys Glu Gly
145                 150                 155                 160

Glu Asp Glu His Pro Gln Cys Leu Asn Ser Gln Pro His Ala Arg Gly
                165                 170                 175

Ser Ser Arg Ala Ile Phe Ser Val Gly Pro Val Ser Pro Ser Arg Arg
            180                 185                 190

Trp Trp Tyr Arg Cys Tyr Ala Tyr Asp Ser Asn Ser Pro Tyr Glu Trp
        195                 200                 205

Ser Leu Pro Ser Asp Leu Leu Glu Leu Leu Val Leu Gly Val Ser Lys
    210                 215                 220

Lys Pro Ser Leu Ser Val Gln Pro Gly Pro Ile Val Ala Pro Glu Glu
225                 230                 235                 240

Thr Leu Thr Leu Gln Cys Gly Ser Asp Ala Gly Tyr Asn Arg Phe Val
                245                 250                 255

Leu Tyr Lys Asp Gly Glu Arg Asp Phe Leu Gln Leu Ala Gly Ala Gln
            260                 265                 270

Pro Gln Ala Gly Leu Ser Gln Ala Asn Phe Thr Leu Gly Pro Val Ser
        275                 280                 285

Arg Ser Tyr Gly Gly Gln Tyr Arg Cys Tyr Gly Ala His Asn Leu Ser
    290                 295                 300

Ser Glu Trp Ser Ala Pro Ser Asp Pro Leu Asp Ile Leu Ile Ala Gly
305                 310                 315                 320
```

```
Gln Phe Tyr Asp Arg Val Ser Leu Ser Val Gln Pro Gly Pro Thr Val
                325                 330                 335

Ala Ser Gly Glu Asn Val Thr Leu Leu Cys Gln Ser Gln Gly Trp Met
            340                 345                 350

Gln Thr Phe Leu Leu Thr Lys Glu Gly Ala Ala Asp Asp Pro Trp Arg
        355                 360                 365

Leu Arg Ser Thr Tyr Gln Ser Gln Lys Tyr Gln Ala Glu Phe Pro Met
    370                 375                 380

Gly Pro Val Thr Ser Ala His Ala Gly Thr Tyr Arg Cys Tyr Gly Ser
385                 390                 395                 400

Gln Ser Ser Lys Pro Tyr Leu Leu Thr His Pro Ser Asp Pro Leu Glu
                405                 410                 415

Leu Val Val Ser Gly Pro Ser Gly Gly Pro Ser Ser Pro Thr Thr Gly
            420                 425                 430

Pro Thr Ser Thr Ser Ala Gly Pro Glu Asp Gln Pro Leu Thr Pro Thr
        435                 440                 445

Gly Ser Asp Pro Gln Ser Gly Leu Gly Arg His Leu Gly Val Val Ile
    450                 455                 460

Gly Ile Leu Val Ala Val Ile Leu Leu Leu Leu Leu Leu Leu Leu Leu
465                 470                 475                 480

Phe Leu Ile Leu Arg His Arg Arg Gln Gly Lys His Trp Thr Ser Thr
                485                 490                 495

Gln Arg Lys Ala Asp Phe Gln His Pro Ala Gly Ala Val Gly Pro Glu
            500                 505                 510

Pro Thr Asp Arg Gly Leu Gln Trp Arg Ser Ser Pro Ala Ala Asp Ala
        515                 520                 525

Gln Glu Glu Asn Leu Tyr Ala Ala Val Lys His Thr Gln Pro Glu Asp
    530                 535                 540

Gly Val Glu Met Asp Thr Arg Ser Pro His Asp Glu Asp Pro Gln Ala
545                 550                 555                 560

Val Thr Tyr Ala Glu Val Lys His Ser Arg Pro Arg Arg Glu Met Ala
                565                 570                 575
```

Ser Pro Pro Ser Pro Leu Ser Gly Glu Phe Leu Asp Thr Lys Asp Arg
580 585 590

Gln Ala Glu Glu Asp Arg Gln Met Asp Thr Glu Ala Ala Ala Ser Glu
595 600 605

Ala Pro Gln Asp Val Thr Tyr Ala Gln Leu His Ser Leu Thr Leu Arg
610 615 620

Arg Glu Ala Thr Glu Pro Pro Pro Ser Gln Glu Gly Pro Ser Pro Ala
625 630 635 640

Val Pro Ser Ile Tyr Ala Thr Leu Ala Ile His
645 650

<210> 19
<211> 651
<212> PRT
<213> homo sapiens

<220>
<221> misc_feature
<223> Protein LILRB1, isoform 4

<400> 19

Met Thr Pro Ile Leu Thr Val Leu Ile Cys Leu Gly Leu Ser Leu Gly
1 5 10 15

Pro Arg Thr His Val Gln Ala Gly His Leu Pro Lys Pro Thr Leu Trp
20 25 30

Ala Glu Pro Gly Ser Val Ile Thr Gln Gly Ser Pro Val Thr Leu Arg
35 40 45

Cys Gln Gly Gly Gln Glu Thr Gln Glu Tyr Arg Leu Tyr Arg Glu Lys
50 55 60

Lys Thr Ala Leu Trp Ile Thr Arg Ile Pro Gln Glu Leu Val Lys Lys
65 70 75 80

Gly Gln Phe Pro Ile Pro Ser Ile Thr Trp Glu His Ala Gly Arg Tyr
85 90 95

Arg Cys Tyr Tyr Gly Ser Asp Thr Ala Gly Arg Ser Glu Ser Ser Asp
100 105 110

Pro Leu Glu Leu Val Val Thr Gly Ala Tyr Ile Lys Pro Thr Leu Ser
115 120 125

```
Ala Gln Pro Ser Pro Val Val Asn Ser Gly Gly Asn Val Ile Leu Gln
    130                 135                 140

Cys Asp Ser Gln Val Ala Phe Asp Gly Phe Ser Leu Cys Lys Glu Gly
145                 150                 155                 160

Glu Asp Glu His Pro Gln Cys Leu Asn Ser Gln Pro His Ala Arg Gly
                165                 170                 175

Ser Ser Arg Ala Ile Phe Ser Val Gly Pro Val Ser Pro Ser Arg Arg
            180                 185                 190

Trp Trp Tyr Arg Cys Tyr Ala Tyr Asp Ser Asn Ser Pro Tyr Glu Trp
        195                 200                 205

Ser Leu Pro Ser Asp Leu Leu Glu Leu Leu Val Leu Gly Val Ser Lys
    210                 215                 220

Lys Pro Ser Leu Ser Val Gln Pro Gly Pro Ile Val Ala Pro Glu Glu
225                 230                 235                 240

Thr Leu Thr Leu Gln Cys Gly Ser Asp Ala Gly Tyr Asn Arg Phe Val
                245                 250                 255

Leu Tyr Lys Asp Gly Glu Arg Asp Phe Leu Gln Leu Ala Gly Ala Gln
            260                 265                 270

Pro Gln Ala Gly Leu Ser Gln Ala Asn Phe Thr Leu Gly Pro Val Ser
        275                 280                 285

Arg Ser Tyr Gly Gly Gln Tyr Arg Cys Tyr Gly Ala His Asn Leu Ser
    290                 295                 300

Ser Glu Trp Ser Ala Pro Ser Asp Pro Leu Asp Ile Leu Ile Ala Gly
305                 310                 315                 320

Gln Phe Tyr Asp Arg Val Ser Leu Ser Val Gln Pro Gly Pro Thr Val
                325                 330                 335

Ala Ser Gly Glu Asn Val Thr Leu Leu Cys Gln Ser Gln Gly Trp Met
            340                 345                 350

Gln Thr Phe Leu Leu Thr Lys Glu Gly Ala Ala Asp Asp Pro Trp Arg
        355                 360                 365

Leu Arg Ser Thr Tyr Gln Ser Gln Lys Tyr Gln Ala Glu Phe Pro Met
```

```
    370                 375                 380

Gly Pro Val Thr Ser Ala His Ala Gly Thr Tyr Arg Cys Tyr Gly Ser
385                 390                 395                 400

Gln Ser Ser Lys Pro Tyr Leu Leu Thr His Pro Ser Asp Pro Leu Glu
                405                 410                 415

Leu Val Val Ser Gly Pro Ser Gly Gly Pro Ser Ser Pro Thr Thr Gly
            420                 425                 430

Pro Thr Ser Thr Ser Gly Pro Glu Asp Gln Pro Leu Thr Pro Thr Gly
        435                 440                 445

Ser Asp Pro Gln Ser Gly Leu Gly Arg His Leu Gly Val Val Ile Gly
    450                 455                 460

Ile Leu Val Ala Val Ile Leu Leu Leu Leu Leu Leu Leu Leu Leu Phe
465                 470                 475                 480

Leu Ile Leu Arg His Arg Arg Gln Gly Lys His Trp Thr Ser Thr Gln
                485                 490                 495

Arg Lys Ala Asp Phe Gln His Pro Ala Gly Ala Val Gly Pro Glu Pro
            500                 505                 510

Thr Asp Arg Gly Leu Gln Trp Arg Ser Ser Pro Ala Ala Asp Ala Gln
        515                 520                 525

Glu Glu Asn Leu Tyr Ala Ala Val Lys His Thr Gln Pro Glu Asp Gly
    530                 535                 540

Val Glu Met Asp Thr Arg Gln Ser Pro His Asp Glu Asp Pro Gln Ala
545                 550                 555                 560

Val Thr Tyr Ala Glu Val Lys His Ser Arg Pro Arg Arg Glu Met Ala
                565                 570                 575

Ser Pro Pro Ser Pro Leu Ser Gly Glu Phe Leu Asp Thr Lys Asp Arg
            580                 585                 590

Gln Ala Glu Glu Asp Arg Gln Met Asp Thr Glu Ala Ala Ala Ser Glu
        595                 600                 605

Ala Pro Gln Asp Val Thr Tyr Ala Gln Leu His Ser Leu Thr Leu Arg
    610                 615                 620
```

```
Arg Glu Ala Thr Glu Pro Pro Pro Ser Gln Glu Gly Pro Ser Pro Ala
625                 630                 635                 640

Val Pro Ser Ile Tyr Ala Thr Leu Ala Ile His
                645                 650
```

<210> 20
<211> 623
<212> PRT
<213> homo sapiens

<220>
<221> misc_feature
<223> Variant 1 de TKT

<400> 20

```
Met Glu Ser Tyr His Lys Pro Asp Gln Gln Lys Leu Gln Ala Leu Lys
1               5                   10                  15

Asp Thr Ala Asn Arg Leu Arg Ile Ser Ser Ile Gln Ala Thr Thr Ala
            20                  25                  30

Ala Gly Ser Gly His Pro Thr Ser Cys Cys Ser Ala Ala Glu Ile Met
        35                  40                  45

Ala Val Leu Phe Phe His Thr Met Arg Tyr Lys Ser Gln Asp Pro Arg
    50                  55                  60

Asn Pro His Asn Asp Arg Phe Val Leu Ser Lys Gly His Ala Ala Pro
65                  70                  75                  80

Ile Leu Tyr Ala Val Trp Ala Glu Ala Gly Phe Leu Ala Glu Ala Glu
                85                  90                  95

Leu Leu Asn Leu Arg Lys Ile Ser Ser Asp Leu Asp Gly His Pro Val
            100                 105                 110

Pro Lys Gln Ala Phe Thr Asp Val Ala Thr Gly Ser Leu Gly Gln Gly
        115                 120                 125

Leu Gly Ala Ala Cys Gly Met Ala Tyr Thr Gly Lys Tyr Phe Asp Lys
    130                 135                 140

Ala Ser Tyr Arg Val Tyr Cys Leu Leu Gly Asp Gly Glu Leu Ser Glu
145                 150                 155                 160

Gly Ser Val Trp Glu Ala Met Ala Phe Ala Ser Ile Tyr Lys Leu Asp
                165                 170                 175
```

```
Asn Leu Val Ala Ile Leu Asp Ile Asn Arg Leu Gly Gln Ser Asp Pro
            180                 185                 190

Ala Pro Leu Gln His Gln Met Asp Ile Tyr Gln Lys Arg Cys Glu Ala
        195                 200                 205

Phe Gly Trp His Ala Ile Ile Val Asp Gly His Ser Val Glu Glu Leu
    210                 215                 220

Cys Lys Ala Phe Gly Gln Ala Lys His Gln Pro Thr Ala Ile Ile Ala
225                 230                 235                 240

Lys Thr Phe Lys Gly Arg Gly Ile Thr Gly Val Glu Asp Lys Glu Ser
                245                 250                 255

Trp His Gly Lys Pro Leu Pro Lys Asn Met Ala Glu Gln Ile Ile Gln
            260                 265                 270

Glu Ile Tyr Ser Gln Ile Gln Ser Lys Lys Lys Ile Leu Ala Thr Pro
        275                 280                 285

Pro Gln Glu Asp Ala Pro Ser Val Asp Ile Ala Asn Ile Arg Met Pro
    290                 295                 300

Ser Leu Pro Ser Tyr Lys Val Gly Asp Lys Ile Ala Thr Arg Lys Ala
305                 310                 315                 320

Tyr Gly Gln Ala Leu Ala Lys Leu Gly His Ala Ser Asp Arg Ile Ile
                325                 330                 335

Ala Leu Asp Gly Asp Thr Lys Asn Ser Thr Phe Ser Glu Ile Phe Lys
            340                 345                 350

Lys Glu His Pro Asp Arg Phe Ile Glu Cys Tyr Ile Ala Glu Gln Asn
        355                 360                 365

Met Val Ser Ile Ala Val Gly Cys Ala Thr Arg Asn Arg Thr Val Pro
    370                 375                 380

Phe Cys Ser Thr Phe Ala Ala Phe Phe Thr Arg Ala Phe Asp Gln Ile
385                 390                 395                 400

Arg Met Ala Ala Ile Ser Glu Ser Asn Ile Asn Leu Cys Gly Ser His
                405                 410                 415

Cys Gly Val Ser Ile Gly Glu Asp Gly Pro Ser Gln Met Ala Leu Glu
            420                 425                 430
```

Asp Leu Ala Met Phe Arg Ser Val Pro Thr Ser Thr Val Phe Tyr Pro
435 440 445

Ser Asp Gly Val Ala Thr Glu Lys Ala Val Glu Leu Ala Ala Asn Thr
450 455 460

Lys Gly Ile Cys Phe Ile Arg Thr Ser Arg Pro Glu Asn Ala Ile Ile
465 470 475 480

Tyr Asn Asn Asn Glu Asp Phe Gln Val Gly Gln Ala Lys Val Val Leu
485 490 495

Lys Ser Lys Asp Asp Gln Val Thr Val Ile Gly Ala Gly Val Thr Leu
500 505 510

His Glu Ala Leu Ala Ala Ala Glu Leu Leu Lys Lys Glu Lys Ile Asn
515 520 525

Ile Arg Val Leu Asp Pro Phe Thr Ile Lys Pro Leu Asp Arg Lys Leu
530 535 540

Ile Leu Asp Ser Ala Arg Ala Thr Lys Gly Arg Ile Leu Thr Val Glu
545 550 555 560

Asp His Tyr Tyr Glu Gly Gly Ile Gly Glu Ala Val Ser Ser Ala Val
565 570 575

Val Gly Glu Pro Gly Ile Thr Val Thr His Leu Ala Val Asn Arg Val
580 585 590

Pro Arg Ser Gly Lys Pro Ala Glu Leu Leu Lys Met Phe Gly Ile Asp
595 600 605

Arg Asp Ala Ile Ala Gln Ala Val Arg Gly Leu Ile Thr Lys Ala
610 615 620

<210> 21
<211> 623
<212> PRT
<213> homo sapiens

<220>
<221> misc_feature
<223> Variant 2 de TKT

<400> 21

Met Glu Ser Tyr His Lys Pro Asp Gln Gln Lys Leu Gln Ala Leu Lys

```
1               5                   10                  15

Asp Thr Ala Asn Arg Leu Arg Ile Ser Ser Ile Gln Ala Thr Thr Ala
            20                  25                  30

Ala Gly Ser Gly His Pro Thr Ser Cys Cys Ser Ala Ala Glu Ile Met
        35                  40                  45

Ala Val Leu Phe Phe His Thr Met Arg Tyr Lys Ser Gln Asp Pro Arg
    50                  55                  60

Asn Pro His Asn Asp Arg Phe Val Leu Ser Lys Gly His Ala Ala Pro
65                  70                  75                  80

Ile Leu Tyr Ala Val Trp Ala Glu Ala Gly Phe Leu Ala Glu Ala Glu
                85                  90                  95

Leu Leu Asn Leu Arg Lys Ile Ser Ser Asp Leu Asp Gly His Pro Val
            100                 105                 110

Pro Lys Gln Ala Phe Thr Asp Val Ala Thr Gly Ser Leu Gly Gln Gly
        115                 120                 125

Leu Gly Ala Ala Cys Gly Met Ala Tyr Thr Gly Lys Tyr Phe Asp Lys
    130                 135                 140

Ala Ser Tyr Arg Val Tyr Cys Leu Leu Gly Asp Gly Glu Leu Ser Glu
145                 150                 155                 160

Gly Ser Val Trp Glu Ala Met Ala Phe Ala Ser Ile Tyr Lys Leu Asp
                165                 170                 175

Asn Leu Val Ala Ile Leu Asp Ile Asn Arg Leu Gly Gln Ser Asp Pro
            180                 185                 190

Ala Pro Leu Gln His Gln Met Asp Ile Tyr Gln Lys Arg Cys Glu Ala
        195                 200                 205

Phe Gly Trp His Ala Ile Ile Val Asp Gly His Ser Val Glu Glu Leu
    210                 215                 220

Cys Lys Ala Phe Gly Gln Ala Lys His Gln Pro Thr Ala Ile Ile Ala
225                 230                 235                 240

Lys Thr Phe Lys Gly Arg Gly Ile Thr Gly Val Glu Asp Lys Glu Ser
                245                 250                 255
```

```
Trp His Gly Lys Pro Leu Pro Lys Asn Met Ala Glu Gln Ile Ile Gln
            260                 265                 270

Glu Ile Tyr Ser Gln Ile Gln Ser Lys Lys Lys Ile Leu Ala Thr Pro
        275                 280                 285

Pro Gln Glu Asp Ala Pro Ser Val Asp Ile Ala Asn Ile Arg Met Pro
    290                 295                 300

Ser Leu Pro Ser Tyr Lys Val Gly Asp Lys Ile Ala Thr Arg Lys Ala
305                 310                 315                 320

Tyr Gly Gln Ala Leu Ala Lys Leu Gly His Ala Ser Asp Arg Ile Ile
                325                 330                 335

Ala Leu Asp Gly Asp Thr Lys Asn Ser Thr Phe Ser Glu Ile Phe Lys
            340                 345                 350

Lys Glu His Pro Asp Arg Phe Ile Glu Cys Tyr Ile Ala Glu Gln Asn
        355                 360                 365

Met Val Ser Ile Ala Val Gly Cys Ala Thr Arg Asn Arg Thr Val Pro
    370                 375                 380

Phe Cys Ser Thr Phe Ala Ala Phe Phe Thr Arg Ala Phe Asp Gln Ile
385                 390                 395                 400

Arg Met Ala Ala Ile Ser Glu Ser Asn Ile Asn Leu Cys Gly Ser His
                405                 410                 415

Cys Gly Val Ser Ile Gly Glu Asp Gly Pro Ser Gln Met Ala Leu Glu
            420                 425                 430

Asp Leu Ala Met Phe Arg Ser Val Pro Thr Ser Thr Val Phe Tyr Pro
        435                 440                 445

Ser Asp Gly Val Ala Thr Glu Lys Ala Val Glu Leu Ala Ala Asn Thr
    450                 455                 460

Lys Gly Ile Cys Phe Ile Arg Thr Ser Arg Pro Glu Asn Ala Ile Ile
465                 470                 475                 480

Tyr Asn Asn Asn Glu Asp Phe Gln Val Gly Gln Ala Lys Val Val Leu
                485                 490                 495

Lys Ser Lys Asp Asp Gln Val Thr Val Ile Gly Ala Gly Val Thr Leu
            500                 505                 510
```

His Glu Ala Leu Ala Ala Ala Glu Leu Leu Lys Lys Glu Lys Ile Asn
515 520 525

Ile Arg Val Leu Asp Pro Phe Thr Ile Lys Pro Leu Asp Arg Lys Leu
530 535 540

Ile Leu Asp Ser Ala Arg Ala Thr Lys Gly Arg Ile Leu Thr Val Glu
545 550 555 560

Asp His Tyr Tyr Glu Gly Gly Ile Gly Glu Ala Val Ser Ser Ala Val
565 570 575

Val Gly Glu Pro Gly Ile Thr Val Thr His Leu Ala Val Asn Arg Val
580 585 590

Pro Arg Ser Gly Lys Pro Ala Glu Leu Leu Lys Met Phe Gly Ile Asp
595 600 605

Arg Asp Ala Ile Ala Gln Ala Val Arg Gly Leu Ile Thr Lys Ala
610 615 620

<210> 22
<211> 244
<212> PRT
<213> homo sapiens

<220>
<221> misc_feature
<223> CEACAM4

<400> 22

Met Gly Pro Pro Ser Ala Ala Pro Arg Gly Gly His Arg Pro Trp Gln
1 5 10 15

Gly Leu Leu Ile Thr Ala Ser Leu Leu Thr Phe Trp His Pro Pro Thr
20 25 30

Thr Val Gln Phe Thr Ile Glu Ala Leu Pro Ser Ser Ala Ala Glu Gly
35 40 45

Lys Asp Val Leu Leu Leu Ala Cys Asn Ile Ser Glu Thr Ile Gln Ala
50 55 60

Tyr Tyr Trp His Lys Gly Lys Thr Ala Glu Gly Ser Pro Leu Ile Ala
65 70 75 80

Gly Tyr Ile Thr Asp Ile Gln Ala Asn Ile Pro Gly Ala Ala Tyr Ser
85 90 95

```
Gly Arg Glu Thr Val Tyr Pro Asn Gly Ser Leu Leu Phe Gln Asn Ile
            100                 105                 110

Thr Leu Glu Asp Ala Gly Ser Tyr Thr Leu Arg Thr Ile Asn Ala Ser
        115                 120                 125

Tyr Asp Ser Asp Gln Ala Thr Gly Gln Leu His Val His Gln Asn Asn
    130                 135                 140

Val Pro Gly Leu Pro Val Gly Ala Val Ala Gly Ile Val Thr Gly Val
145                 150                 155                 160

Leu Val Gly Val Ala Leu Val Ala Ala Leu Val Cys Phe Leu Leu Leu
                165                 170                 175

Ser Arg Thr Gly Arg Ala Ser Ile Gln Arg Asp Leu Arg Glu Gln Pro
            180                 185                 190

Pro Pro Ala Ser Thr Pro Gly His Gly Pro Ser His Arg Ser Thr Phe
        195                 200                 205

Ser Ala Pro Leu Pro Ser Pro Arg Thr Ala Thr Pro Ile Tyr Glu Glu
    210                 215                 220

Leu Leu Tyr Ser Asp Ala Asn Ile Tyr Cys Gln Ile Asp His Lys Ala
225                 230                 235                 240

Asp Val Val Ser
```

<210> 23
<211> 938
<212> DNA
<213> homo sapiens

<220>
<221> misc_feature
<223> ELANE

<400> 23

```
gcacggaggg gcagagaccc cggagcccca gccccaccat gaccctcggc cgccgactcg      60

cgtgtctttt cctcgcctgt gtcctgccgg ccttgctgct gggggcacc gcgctggcct      120

cggagattgt gggggccgg cgagcgcggc cccacgcgtg gcccttcatg gtgtccctgc      180

agctgcgcgg aggccacttc tgcggcgcca ccctgattgc gcccaacttc gtcatgtcgg      240

ccgcgcactg cgtggcgaat gtaaacgtcc gcgcggtgcg ggtggtcctg ggagcccata      300
```

```
acctctcgcg gcgggagccc acccggcagg tgttcgccgt gcagcgcatc ttcgaaaacg      360
gctacgaccc cgtaaacttg ctcaacgaca tcgtgattct ccagctcaac gggtcggcca      420
ccatcaacgc caacgtgcag gtggcccagc tgccggctca gggacgccgc ctgggcaacg      480
gggtgcagtg cctggccatg ggctggggcc ttctgggcag gaaccgtggg atcgccagcg      540
tcctgcagga gctcaacgtg acggtggtga cgtccctctg ccgtcgcagc aacgtctgca      600
ctctcgtgag ggccggcag gccggcgtct gtttcgggga ctccggcagc cccttggtct      660
gcaacgggct aatccacgga attgcctcct tcgtccgggg aggctgcgcc tcagggctct      720
accccgatgc ctttgccccg gtggcacagt ttgtaaactg gatcgactct atcatccaac      780
gctccgagga caacccctgt ccccaccccc gggacccgga cccggccagc aggacccact      840
gagaagggct gcccgggtca cctcagctgc ccacacccac actctccagc atctggcaca      900
ataaacattc tctgttttgt agaaaaaaaa aaaaaaaa                              938
```

<210> 24
<211> 267
<212> PRT
<213> homo sapiens

<220>
<221> misc_feature
<223> ELANE

<400> 24

```
Met Thr Leu Gly Arg Arg Leu Ala Cys Leu Phe Leu Ala Cys Val Leu
1               5                   10                  15

Pro Ala Leu Leu Leu Gly Gly Thr Ala Leu Ala Ser Glu Ile Val Gly
            20                  25                  30

Gly Arg Arg Ala Arg Pro His Ala Trp Pro Phe Met Val Ser Leu Gln
        35                  40                  45

Leu Arg Gly Gly His Phe Cys Gly Ala Thr Leu Ile Ala Pro Asn Phe
    50                  55                  60

Val Met Ser Ala Ala His Cys Val Ala Asn Val Asn Val Arg Ala Val
65                  70                  75                  80

Arg Val Val Leu Gly Ala His Asn Leu Ser Arg Arg Glu Pro Thr Arg
                85                  90                  95

Gln Val Phe Ala Val Gln Arg Ile Phe Glu Asn Gly Tyr Asp Pro Val
            100                 105                 110
```

```
Asn Leu Leu Asn Asp Ile Val Ile Leu Gln Leu Asn Gly Ser Ala Thr
        115                 120                 125

Ile Asn Ala Asn Val Gln Val Ala Gln Leu Pro Ala Gln Gly Arg Arg
    130                 135                 140

Leu Gly Asn Gly Val Gln Cys Leu Ala Met Gly Trp Gly Leu Leu Gly
145                 150                 155                 160

Arg Asn Arg Gly Ile Ala Ser Val Leu Gln Glu Leu Asn Val Thr Val
                165                 170                 175

Val Thr Ser Leu Cys Arg Arg Ser Asn Val Cys Thr Leu Val Arg Gly
            180                 185                 190

Arg Gln Ala Gly Val Cys Phe Gly Asp Ser Gly Ser Pro Leu Val Cys
        195                 200                 205

Asn Gly Leu Ile His Gly Ile Ala Ser Phe Val Arg Gly Gly Cys Ala
    210                 215                 220

Ser Gly Leu Tyr Pro Asp Ala Phe Ala Pro Val Ala Gln Phe Val Asn
225                 230                 235                 240

Trp Ile Asp Ser Ile Ile Gln Arg Ser Glu Asp Asn Pro Cys Pro His
                245                 250                 255

Pro Arg Asp Pro Asp Pro Ala Ser Arg Thr His
            260                 265
```